# EUROPÄISCHE PATENTANMELDUNG

(11) **EP 1 707 202 A1**
(43) Veröffentlichungstag der Anmeldung: **04.10.2006**
(21) Anmeldenummer: 06111981.4
(22) Anmeldetag: 30.03.2006
(51) Int. Cl.: A61K 31/445, A61P 25/28, A61P 31/18, A61P 33/06

(54) **Organische Verbindungen**

(30) Priorität: 31.03.2005 EP 05102559
(71) Anmelder: Speedel Experimenta AG, 4123 Allschwil (CH)
(72) Erfinder: Herold, Peter, 4055, Basel (CH); Mah, Robert, 4132, Muttenz (CH); Stutz, Stefan, 4053, Basel (CH); Stojanovic, Aleksandar, 4055, Basel (CH); Tschinke, Vincenzo, 4102, Binningen (CH); Jotterand, Nathalie, 4053, Basel (CH); Schumacher, Christoph, 4126, Bettingen (CH)
(74) Vertreter: Maué, Paul Georg

(57) **Zusammenfassung**

Es wird die Verwendung substituierter Piperidine der allgemeinen Formeln (I) und (II) 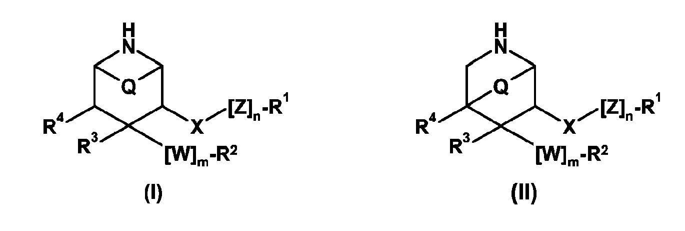 mit den Substituentenbedeutungen wie sie in der Beschreibung näher erläutert sind als Beta-Sekretase-, Cathepsin D-, Plasmepsin II- und/oder HIV-Protease-Hemmer beschrieben.

## Beschreibung

Die vorliegende Erfindung betrifft die Verwendung substituierter Piperidine als Beta-Sekretase-, Cathepsin D-, Plasmepsin II- und/oder HIV-Protease-Hemmer.

Piperidinderivate zur Verwendung als Heilmittel sind z.B. aus der EP 678503 bekannt. Hinsichtlich insbesondere der Beta-Sekretase-, Cathepsin D-, Plasmepsin II- und/oder HIV-Protease-Hemmung besteht jedoch weiterhin ein Bedarf an hoch potenten Wirkstoffen. Hierbei steht die Verbesserung der pharmakokinetischen Eigenschaften im Vordergrund. Diese auf eine bessere Bioverfügbarkeit gerichteten Eigenschaften sind beispielsweise Absorption, metabolische Stabilität, Löslichkeit oder Lipophilie.

### Alzheimer Aspartyl Protease: Beta-Secretase

Die Alzheimer Krankheit ist eine progressive degenerative Krankheit des Gehirns. Die Symptome von Alzheimer sind progressiver Gedächtnisverlust, Sprachschwierigkeiten und schlussendlich Verlust der elementaren Nervenfunktion, die zum Tod führen kann. Als Biomarker sind im zentralen Nervensystem von Alzheimer Patienten Amyloid Plaques, intrazelluläre neurofibrilläre Vernetzungen "Tangles" und aktivierte Mikroglia zu finden. Das Auftreten dieser Biomarker trägt wahrscheinlich zum neuronalen Zelltod und Gedächtnisverlust in Alzheimer Patienten bei. Das beta-Amyloid ist ein definierendes Merkmal der Alzheimer Krankheit. Heute ist man der Meinung, dass es ein Mitverursacher ist und tragend das Fortschreiten der Krankheit beeinflusst. Das Auftreten von amyloiden Plaques und

Angiopathien im Gehirn sind auch charakteristisch für Menschen mit Trisomy 21 (Down's Syndrom), Hereditary Cerebral Hemorrhage with Amloidosis of the Dutch-Type (HCHWA-D) und anderen neurodegenerativen Störungen.

Die beta-amyloiden Plaques bestehen vor allem aus dem Amyloid Beta-Peptid (A-Beta, manchmal auch als betaA4 bezeichnet). Das A-Beta Peptid entsteht proteolytisch vom Beta Amyloid Präkursor Protein (APP). Das Beta-APP wird durch drei geordnetnete enzymische Aktivitäten prozessiert. Der Grossteil des beta-APPs wird über die alpha-Sekretase in einem nicht-amyloidogenen Mechanismus verarbeitet. Ein kleiner Anteil des beta-APPs wird durch die beta-Sekretase Aktivität gespaltet um damit das membrangebundene carboxyterminale Fragment C99 zu bilden. Die gamma-Sekretase verarbeited das C99 Fragement zum amyloidogenen A-Beta Peptid, das aus 39-42 Aminosäuren besteht. Die Aspartyl-Protease Aktivität der Beta-Sekretase wurde bei Anwendung unterschiedlicher Nomenklatur auch als BACE (beta-site APP cleaving enzyme), Asp und Memapsin bezeichnet.

Die Bedeutung der proteolytischen Spaltung von Beta-APP durch die Beta-Sekretase, als entscheidender Schritt, in der Enstehung der Alzheimer Krankheit, ist durch die Beobachtung belegt, dass humane Mutationen in der Beta-Sekretase (Schwedische Mutanten) zu einer erhöhten A-Beta Peptid Erzeugung und somit zu einer frühen, vererbaren Form der Alzheimer Krankheit führen. Andererseits vermögen BACE1 defiziente Mäuse, die einen normalen Phenotypen zeigen, kein A-Beta Peptid zu bilden. Wenn diese Mäuse mit APP transgenen Mäusen, die durch einen Überschuss an APP gezeichnet sind, gekreuzt werden, kann bei den Nachkommen, im Vergleich zu BACE1 normalen Tieren, eine reduzierte Menge an A-Beta Peptid in Gehirnextrakten festgestellt werden. Diese Feststellungen unterstützen den Ansatz, dass die pharmakologische Hemmung der Beta-Sekretase und die einhergehende Verminderung der A-Beta Peptid Ablagerungen im Gehirn, eine therapeutische Strategy zur Behandlung der Alzheimer Krankheit und weiteren beta-amyloiden Syndromen darstellt.

Chemische Verbindungen, welche effektiv die Beta-Sekretase Aktivität hemmen, werden die durch die Beta-Sekretase erzeugte Spaltung von APP zum A-Beta Peptid inhibieren. Die pharmakologische Hemmung der A-Beta Peptid Erzeugung reduziert möglicherweise Beta-Amyloid Ansammlungen, respektive die Entstehung von Plaques. Beta-Sekretase hemmende chemische Verbindungen sind daher zur Behandlung oder zur Prevention von Krankheiten von Nutzen, welche durch Amyloid Beta Ansammlungen oder Ablagerungen charakterisiert sind. Die vorliegende Erfindung bezieht sich auch auf Methoden und Anwendung therapeutisch wirksamer pharmazeutischer Zusammensetzungen zur Behandlung von Patienten oder zur Prevention von Krankheiten. Die Methoden umfassen die Alzheimer Krankheit, die Prevention oder Verzögerung der Entwicklung von Alzheimer, die Behandlung von Patienten mit MCI (mild cognitive impairment), von Patienten, deren MCI zur Alzheimer Krankheit fortschreitet, die Behandlung des Down Syndromes, die Behandlung von HCHWAD, die Behandlung der zerebralen amyloiden Angiopathy, und die Behandlung degenerativer Hirnleistungsschwächen.

### Alzheimer Aspartyl Protease: Cathepsin D

Das humane Enzym Cathepsin D ist eine intrazelluläre Aspartyl Peptidase, welches hauptsächlich in Lysosomen gefunden wird. Cathepsin D reguliert verschiedene Haushaltsfunktionen des gesamten Metabolismus, unter anderem den Abbau von zellulären und phagozytierten Proteinen. Das Enzyme ist auch wahrscheinlich in einer Vielzahl von Krankheitszuständen involviert, so wie zum Beispiel in Krebs und der Alzheimer Krankheit. Klinische Studien haben gezeigt, dass Cathepsin D übermässig exprimiert in Brustkrebs-Zellen vorkommt und dies scheint, durch erhöhtes Zellwachstum, mit einem erhöhten Risiko zu Metastasen verbunden sein. Es wird auch vermutet, dass Cathepisin D in der Bildung des Beta-Amyloid Peptides in der Alzheimer Krnakheit involviert ist. Die Verfügbarkeit von selektiven und potenten Hemmern von Cathepsin D werden helfen, um weitere Funktionen des Cathepsin D Enzymes in Krankheitszuständen zu definieren und somit die Entwicklung von neuen therapeutischen Substanzen erleichtern.

### Malaria Aspartyl Protease: Plasmepsin I und II

Malaria wird als eine der ernsthaftesten Infektionskrankheiten der Welt betrachtet, welche rund 500 Millionen Personen betrifft. Die Krankheit wird durch die Anopheles-Mücke verbreitet, welche hauptsächlich in tropischen Regionen anzutreffen ist. Die Spezies Plasmodium Falziparum ist für mehr als 95% der mit Malaria verbundenen Morbiditäts- und Mortalitätsfälle verantwortlich. Das Plasmodium Falziparum ist zunehmend resistent gegen bestehende Behandlungsansätze wie Chloroquine, Mefloquine und Sulfadoxime/Pyrimethamine. Daher besteht ein dringenden Bedarf für neue Behandlungsmöglichkeiten.

Im erythrozytären Stadium des parasitären Lebenszyklus dringt der Parasit in die roten Blutzellen seines Gastgeber Orgnismus ein und verzehrt bis zu 80% des Hemoglobins als Nahrungsquelle für Wachstum und Entwicklung. Der Hemoglobinabbau findet in einer aziden Vakuole im Parasiten statt und viele der gegenwärtigen Malaria Medikamente scheinen diese wichtige Vakuolenfunktion zu beeinträchtigen. Die Vakuole enthält Aspartyl-, Cysteine- und Metallo-Proteasen, welche alle im Hemoglobinabbau involviert sein könnten. Mindestens 10 Gene zur Kodierung von Aspartyl-Proteasen wurden im Plasmodiumgenom identifiziert. Vier dieser Aspartyl-Proteasen wurden in den aziden Nahrungsmittelvakuole des Parasiten entdeckt, namentlich Plasmepsin I, II, IV und HAP, ein Histoaspartyl-Protease. Inhibitoren der Plasmepsin I und II Proteasen haben ihren Nutzen in Zell- und Tiermodellen für Malaria gezeigt, wodurch diese Enzyme als therapeutischer Ansatz zur Entdeckung von neuen Medikamenten validiert wurden. Ein nicht-selektiver Inhibitor der Aspartyl-Proteasen, Pepstatin, inhibiert das Wachstum von Plasmodium Falziparum in vitro. Ähnliche Resultate wurden mit Analogen von Pepstatin erziehlt. Diese Experimente zeigen, dass die Hemmung von Aspartyl-Proteasen den Lebenszyklus des Plasmodium Falziparum unterbrechen kann. Die dargestellten Erfindungen beziehen sich auf die Identifikation von niedermolekularen, nicht-peptidischen Inhibitoren der Plasmodium Falziparum Protease Plasmepsin II oder anderen Aspartyl-Proteasen zur Behandlung oder Verhinderung der Übertragung von Malaria.

### HIV Aspartyl Protease: HIV-1 Peptidase

Das im Jahre 1981 erstmals in einer kleinen Anzahl von Patienten festgestellte Acquired Immunodeficiency Syndrome (AIDS) ist heute eine bedeutende Epidemie mit weltweit mehr als 38 Millionen infizierten Menschen, von welchen rund 1 Million in den Vereinigten Staaten, 580'000 in West-Europa und mehr als 25 Millionen in Afrika leben (http://www.unaids.org). Seit dem Zeitpunkt als AIDS erstmals klinisch identifiziert wurde, hat zwar ein bedeutender wissenschaftlicher und therapeutischer Fortschritt stattgefunden, jedoch bleibt AIDS, vor allem in den Entwicklungsländern, eine nicht-kontrollierbare Krankheit.

Die Prognosen für AIDS Patienten, welche uneingeschränkten Zugang zu den heutigen Therapiemöglichkeiten haben, hat sich seit der Entdeckung von AIDS komplett verändert. Heute ist die durchschnittliche Lebenserwartung für behandelte HIV-positive Patienten über 8 Jahre. Bevor das Medikament AZT im Jahr 1987 eingeführt wurde, lag die Lebenserwartung von AIDS Patienten noch unter einem Jahr. Diese drastische Verbesserung der Lebenserwartung ist vor allem auf die Entwicklung von wirkungsvollen Therapien, auf die frühe Erkennung von Human Immunodeficiency Virus (HIV)-Trägern und auf das kontinuierliche Bestreben deer Verminderung der Virenresistenz durch die Einfuhrung neuer Medikamente und Kombinationstherapien, zurückzuführen.

Die von der FDA bewilligten Therapien hemmen drei Mechanismen des HIV-Lebenszyklus, nämlich die reverse Transkription, die proteolytische Reifung und die Zellfusion. Dreifachtherapien, gewöhnlich als Highly Active Antiretroviral Therapie (HAART) bekannt, stellen heute den Behandlungsstandard dar. Diese Behandlungsmethode schreibt die Anwendung eines Protease Inhibitors oder eines Non-Nucleoside Reverse Transkriptase Inhibitors in Kombination mit zwei Nucleoside Reverse Transkriptase Inhibitoren vor.

Die Translation der HIV Ribonukleinsäure führt zur Erzeugung von zwei Polyprotein Vorstufen, das Gag und das Gag-Pol. Das 55-kDa grosse Gag Polyprotein beinhaltet die viralen Strukturproteine und das 160-kDa grosse Gag-Pol Polyprotein enthält die funkionellen viralen Enzyme Reverse Transcriptase, Protease und Integrase. Die exprimierten Gag und

Gag-Pol Polyproteine werden zur Plasmamembran transportiert, wo üblicherweise eine Ansammlung von Typ-C Retroviren und Lentiviren entsteht. Während der Ansammlung von Partikeln zerteilt die virale Protease die Gag und Gag-Pol Vorstufen in die, für die Virenvermehrung essentiellen, strukturellen und funktionellen Proteine. Die Protease Aktivität im Zytoplasma von infizierten Zellen erlaubt es das Viruspartikel zu bilden und von der Zelle durch Knospung freizusetzen und zu verbreiten.

Die aktive HIV-1 Protease bildet ein Dimer aus zwei identischen, 11 kDa grossen Untereinheiten, welche je ein katalytisches Aspartyl beitragen. Im Gegensatz dazu sind nichtvirale, dh. von humanen Zellen gebildete Mitglieder der Aspartyl-Protease Familie monomere Enzyme, die zwei Asp-Thr-Gly Sequenzbereiche enthalten. Die einzigartige dimere Struktur der retroviralen Protease wird hauptsächlich durch eine antiparallele Beta-Faltblattstruktur stabilisiert, welche durch Verzahnung der Untereinheiten an deren amino- und carboxyterminalen Enden geformt wird.

Die Aktivierung der HIV-1 Protease durch die Dimerisierung oder durch die autokatalytischen Freigabe des Gag-Pol Polyproteins ist ein kritischer Schritt im viralen Lebenszyklus. So führt die Hemmung der viralen Proteaseaktivität zur Hinderung der Gag Polyproteinverarbeitung, Virusbildung und zum Verlust der viralen Infektiosität. Somit sind virale Proteasehemmer eine wichtige therapeutische Strategy gegen HIV, die zu mehreren Medikamenten geführt hat. Das Design dieser Medikamente wurde durch den Zugang zu Kristallstrukturen, die entweder das nackte Enzym oder den Enzym-Inhibitorkomplex darstellen, stark erleichtert. Zudem sind heutzutage ausführliche biochemische Daten vorhanden, welche die katalytischen Mechanismen und die molekulare Basis der enzymatischen Substratbindung ausführlich schildern.

Ein Gegenstand der Erfindung ist daher die Verwendung substituierter Piperidine der allgemeinen Formeln (I) und (II) 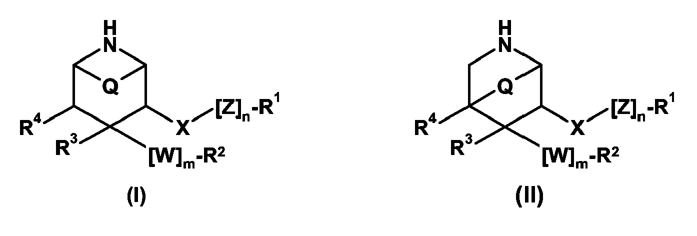 worin
(A) R¹ in Formel (I) substituiertes oder unsubstituiertes Oxazolyl, Indolyl, Pyrrolyl, Pyrazolyl, Triazinyl, 2-Oxo-dihydro-benzo-[d][1,3]oxazinyl, 4-Oxo-dihydro-imidazolyl, 5-Oxo-4H-[1,2,4]triazinyl, 3-Oxo-4H-benzo[1,4]thiazinyl, Tetrahydro-chinoxalinyl, 1,1,3-Trioxodihydro-2H-1lambda*6*-benzo[1,4]thiazinyl, 1-Oxo-pyridyl, Dihydro-2H-benzo[1,4]oxazinyl, 2-Oxo-tetrahydro-benzo[e][1,4]diazepinyl, 2-Oxo-dihydrobenzo[e][1,4]diazepinyl, 1H-Pyrrolizinyl, Phthalazinyl, 1-Oxo-3H-isobenzofuranyl, 4-Oxo-3H-thieno[2,3-d]pyrimidinyl, 3-Oxo-4H-benzo[1,4]oxazinyl, [1,5]Naphthyridyl, Dihydro-2H-benzo[1,4]thiazinyl, 1,1-Dioxo-dihydro-2H-benzo[1,4]thiazinyl, 2-Oxo-1H-pyrido[2,3-b][1,4]oxazinyl, Dihydro-1H-pyrido[2,3-b][1,4]oxazinyl, 1 H-Pyrrolo[2,3-b]pyridyl, Benzo[1,3]dioxolyl, Benzooxazolyl, 2-Oxo-benzooxazolyl, 2-Oxo-1,3-dihydroindolyl, 2,3-Dihydroindolyl, Indazolyl, Benzofuranyl, Dihydrobenzofuranyl, Tetrahydropyranyl, 2-Oxo-piperidinyl oder 2-Oxo-azepanyl ist; oder
(B) R¹ in Formel (I) Aryl oder Heterocyclyl ist, welches durch mindestens einen Substituenten ausgewählt aus C₁₋₆-Alkoxy-C₁₋₆-alkoxy-C₁₋₆-alkyl, C₃₋₈-Cycloalkyl-C₁₋₆-alkyl, C₁₋₆₋Alkoxy-carbonyl, C₀₋₆-Alkylcarbonylamino, C₀₋₆-Alkylcarbonylamino-C₁₋₆-alkyl, C₀₋₆₋Alkylcarbonylamino-C₁₋₆-alkoxy, (N-C₁₋₆-Alkyl)-C₀₋₆-alkylcarbonylamino-C₁₋₆-alkyl, (N-C₁₋₆-Alkyl)-C₀₋₆-alkylcarbonylamino-C₁₋₆-alkoxy, C₃₋₈-Cycloalkylcarbonylamino-C₁₋₆₋alkyl, C₃₋₈-Cycloalkylcarbonylamino-C₁₋₆-alkoxy, C₁₋₆-Alkoxy-C₁₋₆-alkyl, Hydroxy-C₁₋₆₋alkyl, Hydroxy-C₁₋₆-alkoxy-C₁₋₆-alkyl, Hydroxy-C₁₋₆-alkoxy-C₁₋₆-alkoxy, C₁₋₆-Alkoxycarbonylamino-C₁₋₆-alkyl, C₁₋₆-Alkoxycarbonylamino-C₁₋₆-alkoxy, C₁₋₆-Alkylaminocarbonylamino-C₁₋₆-alkyl, C₁₋₆-Alkylaminocarbonylamino-C₁₋₆-alkoxy, C₁₋₆-Alkylaminocarbonyl-C₁₋₆-alkyl, C₁₋₆-Alkylaminocarbonyl-C₁₋₆-alkoxy, C₁₋₆-Alkylaminocarbonyl-C₁-₆₋alkoxy-C₁₋₆-alkyl, Di-C₁₋₆-alkylaminocarbonyl-C₁₋₆-alkyl, Di-C₁₋₆-alkylaminocarbonyl-C₁₋₆-al koxy, C₁₋₆-Alkylcarbonyloxy-C₁₋₆-alkyl, C₁₋₆-Alkoxycarbonyloxy-C₁₋₆-alkyl,C₁₋₆₋Alkylcarbonyloxy-C₁₋₆-alkoxy, Cyano-C₁₋₆-alkyl, Cyano-C₁₋₆-alkoxy, C₁₋₆-Alkoxycarbonyl-C₁₋₆-alkyl, C₁₋₆-Alkoxycarbonyl-C₁₋₆-alkoxy, C₁₋₆-Alkylsulfonylamino-C₁₋₆-alkyl, C₁₋₆-Alkylsulfonylamino-C₁₋₆-alkoxy, (N-C₁₋₆-Alkyl)-C₁₋₆-alkylsulfonylamino-C₁₋₆-alkyl, (N-C₁₋₆-Alkyl)-C₁₋₆-alkylsulfonylamino-C₁₋₆-alkoxy, Amino-C₁₋₆-alkyl, Amino-C₁₋₆-alkoxy, C₁₋₆-Alkylamino-C₁₋₆-alkyl, C₁₋₆-Alkylamino-C₁₋₆-alkoxy, Di-C₁₋₆-alkylamino-C₁₋₆-alkyl, Di-C₁₋₆-alkylamino-C₁₋₆-alkoxy, C₁₋₆-Alkylsulfonyl-C₁₋₆-alkyl, C₁₋₆-Alkylsulfonyl-C₁₋₆₋alkoxy, Carboxy-C₁₋₆-alkyl, Carboxy-C₁₋₆-alkoxy, Carboxy-C₁₋₆-alkoxy-C₁₋₆-alkyl, C₁₋₆₋Alkoxy-C₁₋₆-alkyl-carbonyl, Acyl-C₁₋₆-alkoxy-C₁₋₆-alkyl, (N-C₁₋₆-Alkyl)-C₁₋₆-alkoxycarbonylamino, (N-Hydroxy)-C₁₋₆-alkylaminocarbony-C₁₋₆-alkyl, (N-Hydroxy)-C₁₋₆₋alkylaminocarbonyl-C₁₋₆-alkoxy, (N-Hydroxy)-aminocarbonyl-C₁₋₆-alkyl, (N-Hydroxy)-aminocarbonyl-C₁₋₆-alkoxy, C₁₋₆-Alkoxy-aminocarbonyl-C₁₋₆-alkyl, C₁₋₆-Alkoxyaminocarbonyl-C₁₋₆-alkoxy, (N-C₁₋₆-Alkoxy)-C₁₋₆-alkylaminocarbonyl-C₁₋₆-alkyl, (N-C₁₋₆₋Alkoxy)-C₁₋₆-alkylaminocarbonyl-C₁₋₆-alkoxy, (N-Acyl)-C₁₋₆-alkoxy-C₁₋₆-alkylamino, C₁₋₆-Alkoxy-C₁₋₆-alkyl-carbamoyl, (N-C₁₋₆-Alkyl)-C₁₋₆-alkoxy-C₁₋₆-alkyl-carbamoyl, C₁₋₆₋Alkoxy-C₁₋₆-alkyl-carbonyl, C₁₋₆-Alkoxy-C₁₋₆-alkyl-carbonylamino, (N-C₁₋₆-Alkyl)-C₁₋₆₋alkoxy-C₁₋₆-alkyl-carbonylamino, Carbamoyl-C₁₋₆-alkyl, Carbamoyl-C₁₋₆-alkoxy, C₁₋₆₋Alkyl-carbamoyl, Di-C₁₋₆-alkyl-carbamoyl, C₁₋₆-Alkyl-sulfonyl, C₁₋₆-Alkyl-amidinyl, Acetamidinyl-C₁₋₆-alkyl, O-Methyloximyl-C₁₋₆-alkyl und O,N-Dimethylhydroxylamino-C₁₋₆-alkyl substituiert ist; oder
(C) R¹ in Formel (I) Aryl oder Heterocyclyl ist, welches durch mindestens einen Substituenten ausgewählt aus [1,2,4]-Triazol-1-yl-alkyl, [1,2,4]-Triazol-1-yl-alkoxy, [1,2,4]-Triazol-4-yl-alkyl, [1,2,4]-Triazol-4-yl-alkoxy, [1,2,4]-Oxadiazol-5-yl-alkyl, [1,2,4]-Oxadiazol-5-yl-alkoxy, 3-Methyl-[1,2,4]-oxadiazol-5-yl-alkyl, 3-Methyl-[1,2,4]-oxadiazol-5-yl-alkoxy, 5-Methyl-[1,2,4]-oxadiazol-3-yl-alkyl, 5-Methyl-[1,2,4]-oxadiazol-3-yl-alkoxy, Tetrazol-1-yl-alkyl, Tetrazol-1-yl-alkoxy, Tetrazol-2-yl-alkyl, Tetrazol-2-yl-alkoxy, Tetrazol-5-yl-alkyl, Tetrazol-5-yl-alkoxy, 5-Methyl-tetrazol-1-yl-alkyl, 5-Methyl-tetrazol-1-yl-alkoxy, Thiazol-4-yl-alkyl, Thiazol-4-yl-alkoxy, Oxazol-4-yl-alkyl, Oxazol-4-yl-alkoxy, 2-Oxo-pyrrolidinyl-alkyl, 2-Oxo-pyrrolidinyl-alkoxy, Imidazolyl-alkyl, Imidazolyl-alkoxy, 2-Methyl-imidazolyl-alkyl, 2-Methyl-imidazolyl-alkoxy, Dioxolanyl, Dioxanyl, Dithiolanyl, Dithianyl, Pyrrolidinyl, Piperidinyl, Piperazinyl, Pyrrolyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 3-Acetamidomethylpyrrolidinyl, 3-C₁₋₆-Alkoxy-C₁₋₆-alkylpyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl, 2-Oxo-tetrahydro-pyrimidinyl, 2-Oxo-oxazolidinyl-C₁₋₆-alkyl, 2-Oxo-oxazolidinyl-C₁₋₆-alkoxy, 1-C₁₋₆-Alkoxy-C₁₋₆-alkyl-imidazol-2-yl, 1-C₁₋₆-Alkoxy-C₁₋₆-alkyl-tetrazol-5-yl, 5-C₁₋₆₋Alkoxy-C₁₋₆-alkyl-tetrazol-1-yl und 2-C₁₋₆-Alkoxy-C₁₋₆-alkyl-4-oxo-imidazol-1-yl substituiert ist; oder
(D) R¹ in Formel (I) Aryl oder Heterocyclyl ist, wenn n die Zahl 0 ist und X -O-CH-R¹¹-CO-NR⁹- ist, oder wenn n und m die Zahl 0 ist und X -O-CH-R¹¹- und R² durch C₁₋₆₋Alkoxybenzyloxy-C₁₋₆-alkoxy substituiertes Phenyl ist; oder
(E) R¹ in Formel (I) Aryl oder Heterocyclyl ist, wenn n die Zahl 1 und Z Alk-NR⁹- ist, wobei Alk C₁₋₆-Alkylen bezeichnet; oder
(F) R¹ in Formel (I) Aryl oder Heterocyclyl ist, wenn R² Tetrazolyl oder Imidazolyl, welche durch 1-3 Halogen, Hydroxy, Cyano, Trifluormethyl, C₁₋₆-Alkyl, Halo-C₁₋₆-alkyl, Hydroxy-C₁₋₆-alkyl, C₁₋₆-Alkoxy-C₁₋₆-alkyl, Cyano-C₁₋₆-alkyl, Carboxy-C₁₋₆-alkyl, C₁₋₆₋Alkanoyloxy-C₁₋₆-alkyl, C₁₋₆-Alkoxycarbonyloxy-C₁₋₆-alkyl, C₁₋₆-Alkoxycarbonyl, oder C₁₋₆-Alkoxygruppen, oder eine C₁₋₆-Alkylendioxygruppe, und/oder durch einen Rest L1-T1-L2-T2-L3-T3-L4-T4-L5-U substituiert sein können, darstellt; oder
(G) R¹ in Formel (II) Aryl oder Heterocyclyl ist; R² Phenyl, Naphthyl, Acenaphthyl, Cyclohexyl, Pyridyl, Pyrimidinyl, Pyrazinyl, Oxo-pyridinyl, Diazinyl, Triazolyl, Thienyl, Oxazolyl, Oxadiazolyl, Thiazolyl, Pyrrolyl, Furyl, Tetrazolyl oder Imidazolyl, welche Reste durch 1-3 Halogen, Hydroxy, Cyano, Trifluormethyl, C₁₋₆-Alkyl, Halo-C₁₋₆-alkyl, Hydroxy-C₁₋₆-alkyl, C₁₋₆-Alkoxy-C₁₋₆-alkyl, Cyano-C₁₋₆-alkyl, Carboxy-C₁₋₆- alkyl, C₁₋₆-Alkanoyloxy-C₁₋₆-alkyl, C₁₋₆-Alkoxycarbonyloxy-C₁₋₆-alkyl, C₁₋₆-Alkoxycarbonyl, oder C₁₋₆-Alkoxygruppen, oder eine C₁₋₆-Alkylendioxygruppe, und/oder durch einen Rest L1-T1- L2-T2-L3-T3-L4-T4-L5-U substituiert sein können, darstellen;
L1, L2, L3, L4 und L5 unabhängig voneinander eine Bindung, C₁₋₈-Alkylen, C₂₋₈-Alkenylen oder C₂₋₈-Alkinylen darstellen, oder abwesend sind;
T1, T2, T3 und T4 unabhängig voneinander
(a) eine Bindung darstellen, oder abwesend sind, oder eine der Gruppen
(b) -CH(OH)-
(c) -CH(OR⁶)-
(d) -CH(NR⁵R⁶)-
(e) -CO-
(f) -CR⁷R^{a}-
(g) -O- oder -NR⁶-
(h) -S(O)₀₋₂-
(i) -SO₂NR⁶-
(j) -NR⁶SO₂-
(k) -CONR⁶-
(l) -NR⁶CO-
(m) -O-CO-
(n) -CO-O-
(o) -O-CO-O-
(p) -O-CO-NR⁶-
(q) -N(R⁶)-CO-N(R⁶)-
(r) -N(R⁶)-CO-O-
(s) Pyrrolidinylen, Piperidinylen oder Piperazinylen
(t) -C(R¹¹)(R¹²)-
darstellen,
wobei die von (b)-(t) ausgehenden Bindungen zu einem gesättigten oder aromatischen C-Atom der benachbarten Gruppe führen, falls die Bindung von einem Heteroatom ausgeht, und wobei nicht mehr als zwei Gruppen (b)-(f), drei Gruppen (g)-(h) und eine Gruppe (i)-(t) anwesend sind;
R³ Wasserstoff, Hydroxy, C₁₋₆-Alkoxy oder C₂₋₆-Alkenyloxy;
R⁴ Wasserstoff, C₁₋₆-Alkyl, C₂₋₆-Alkenyl, C₁₋₆-Alkoxy, Hydroxy-C₁₋₆-alkyl, C₁₋₆-Alkoxy-C₁₋₆-alkyl, Benzyl, Oxo, oder eine Gruppe
R^{4a}-Z1-X1- darstellen, wobei R^{4a}
(a) H-
(b) C₁₋₆-Alkyl-
(c) C₂₋₆-Alkenyl-
(d) Hydroxy-C₁₋₆-alkyl-
(e) Polyhydroxy-C₁₋₆-alkyl-
(f) C₁₋₆-Alkyl-O-C₁₋₆-alkyl-
(g) Aryl-
(h) Heterocyclyl-
(i) Arylalkyl-
(j) Heterocyclylalkyl-
(k) Aryloxyalkyl-
(I) Heterocyclyloxyalkyl-
(m) (R⁵,R⁶)N-(CH₂)₁₋₃-
(n) (R⁵, R⁶)N-
(o) C₁₋₆-Alkyl-S(O)₀₋₂-
(p) Aryl-S(O)₀₋₂-
(q) Heterocyclyl-S(O)₀₋₂-
(r) HO-SO₃- bzw. deren Salze
(s) H₂N-C(NH)-NH-
(t) NC-
darstellt und die von (n)-(t) ausgehenden Bindungen zu einem C-Atom der benachbarten Gruppe führen und dieses C-Atom gesättigt ist, falls die Bindung von einem Heteroatom ausgeht;
Z1
(a) eine Bindung darstellt, abwesend ist, oder eine der Gruppen
(b) C₁₋₆-Alkylen-
(c) C₂₋₆-Alkenylen-
(d) -O-, -N(R¹¹)-, -S(O)₀₋₂-
(e) -CO-
(f) -O-CO-
(g) -O-CO-O-
(h) -O-CO-N(R¹¹)-
(i) -N(R¹¹)-CO-O-
(j) -CO-N(R¹¹)-
(k) -N(R¹¹)-CO-
(l) -N(R¹¹)-CO-N(R¹¹)-
(m) -CH(OR⁹)-
darstellt und die von (d) und (f)-(m) ausgehenden Bindungen zu einem C-Atom der benachbarten Gruppe führen und dieses C-Atom gesättigt ist, falls die Bindung von einem Heteroatom ausgeht;
X1
(a) eine Bindung darstellt, abwesend ist, oder eine der Gruppen
(b) -O-
(c) -N(R¹¹)-
(d) -S(O)₀₋₂-
(e) -(CH₂)₁₋₃-
darstellt;
oder R³ und R⁴ zusammen eine Bindung darstellen;
R⁵ und R⁶ Wasserstoff, C₁₋₆-Alkyl, C₂₋₆-Alkenyl, Aryl-C₁₋₆-alkyl oder Acyl bedeuten, oder gemeinsam mit dem N-Atom, an das sie gebunden sind, einen 5- oder 6-gliedrigen heterocyclischen Ring bedeuten, der ein zusätzliches N-, O- oder S-Atom oder eine -SO- oder -SO₂- Gruppe enthalten kann, wobei das zusätzliche N-Atom gegebenenfalls durch C₁₋₆₋Alkyl-Reste substituiert sein kann;
R⁷ und R⁸ gemeinsam mit dem C-Atom, an das sie gebunden sind, einen 3-7-gliedrigen Ring darstellen, der ein oder zwei -O- oder -S- Atome oder -SO- oder -SO₂- Gruppen enthalten kann;
R⁹ Wasserstoff, C₁₋₆-Alkyl, C₁₋₆-Alkoxy-C₁₋₆-alkyl, Acyl oder Arylalkyl;
R¹⁰ Carboxyalkyl, Alkoxycarbonylalkyl, Alkyl oder Wasserstoff;
R¹¹ Wasserstoff oder C₁₋₆-Alkyl ist;
R¹² Wasserstoff oder C₁₋₆-Alkyl ist;
U Wasserstoff, C₁₋₆-alkyl, C₃₋₈-Cycloalkyl, Cyano, gegebenenfalls substituiertes C₃₋₈-Cycloalkyl, Aryl, oder Heterocyclyl darstellt;
Q Ethylen darstellt oder abwesend ist (Formel I) oder Ethylen oder Methylen darstellt (Formel II);
X eine Bindung, Sauerstoff oder Schwefel, wobei die von einem Sauerstoff- oder Schwefelatom ausgehende Bindung zu einem gesättigten C-Atom der Gruppe Z oder zu R¹ führt, oder eine Gruppe >CH-R¹¹, >CHOR⁹, -O-CO-, >CO, >C=NOR¹⁰, -O-CHR¹¹- oder -O-CHR¹¹-CO-NR⁹- darstellt;
W Sauerstoff oder Schwefel darstellt;
Z C₁₋₆-Alkylen, C₂₋₆-Alkenylen, Hydroxy-C₁₋₆-alkyliden, -O-, -S-, -O-Alk-, -S-Alk-, -Alk-O-, Alk-S- oder -Alk-NR⁹- ist, wobei Alk C₁₋₆-Alkylen bezeichnet; und wobei
(a) falls Z -O- oder -S- darstellt, X -CH-R¹¹- ist und entweder R² einen Substituenten L1-T1-L2-T2-L3-T3-L4-T4-L5-U enthält oder R⁴ ein wie oben definierter, von Wasserstoff verschiedener Substituent ist;
(b) falls Z -O-Alk- oder -S-Alk- darstellt, X -CH-R¹¹- ist; und
(c) falls X eine Bindung darstellt, Z C₂₋₆-Alkenylen, -Alk-O- oder-Alk-S- darstellt,
n die Zahl 0 oder 1 ist;
m die Zahl 0 oder 1 ist;
und pharmazeutisch anwendbare Salze davon.

Beispiele für Alkyl- und Alkoxyreste sind C₁₋₆-Alkyl- und Alkoxyreste wie Methyl, Ethyl, n-Propyl, Isopropyl, n-Butyl, Isobutyl, sec.-Butyl, tert.-Butyl, Pentyl, Hexyl bzw. Methoxy, Ethoxy, Propoxy, Isopropoxy, Butoxy, Isobutoxy, sec.-Butoxy und tert.-Butoxy. C₁₋₆₋Alkylendioxyreste sind vorzugsweise Methylendioxy, Aethylendioxy und Propylendioxy. Beispiele von C₁₋₆-Alkanoylresten sind Acetyl, Propionyl und Butyryl. Cycloalkyl bedeutet einen gesättigten, cyclischen Kohlenwasserstoffreste mit 3-12 Kohlenstoffatomen, d.h. Cyclopropyl, Cyclobutyl, Cyclopentyl, Cyclohexyl, Cycloheptyl, Bicyclo[2.2.1]heptyl, Cyclooctyl, Bicyclo[2.2.2]octyl und Adamantyl-C₁₋₈-Alkylenreste sind z.B. Methylen, Ethylen, Propylen, 2-Methyl-propylen, Tetra-, Penta- und Hexamethylen; C₂₋₈-Alkenylenreste sind z.B. Vinylen und Propenylen; C₂₋₈-Alkinylenreste ist z.B. Ethinylen; Acylreste sind Alkanoylreste, vorzugsweise C₁₋₆-Alkanoylreste, oder Aroylreste, wie Benzoyl. Aryl bezeichnet ein- oder mehrkernige aromatische Reste, die ein- oder mehrfach substituiert sein können, wie beispielsweise Phenyl, substituiertes Phenyl, Naphthyl, substituiertes Naphthyl, Tetrahydronaphthyl oder substituiertes Tetrahydronaphthyl. Beispiele für Substituenten an solchen Arylresten oder an Heterocyclylresten sind C₁₋₆-Alkyl, Trifluormethyl, Trifluormethoxy, Nitro, Amino, C₂₋₆-Alkenyl, C₁₋₆-Alkoxy, C₁₋₆-Alkylsulfinyl, C₁₋₆-Alkylcarbonyloxy, Hydroxy, Halogen, Cyano, Carbamoyl, Carboxy und C₁₋₆-Alkylendioxy, sowie gegebenenfalls durch Halogen, C₁₋₆-Alkyl, C₁₋₆-Alkoxy oder Dihydroxy-C₁₋₆-alkylaminocarbonyl substituiertes Phenyl, Phenoxy, Phenylthio, Phenyl-C₁₋₆-alkyl oder Phenyl-C₁₋₆-alkoxy, Weitere Beispiele von Substituenten an Aryl- oder Heterocyclylresten sind Oxo, C₁₋₆-Alkoxycarbonylphenyl, Hydroxy-C₁₋₆-alkylphenyl, Benzyloxy, Pyridylcarbonylamino-C₁₋₆-alkyl, C₂₋₆-Alkenyloxy, C₁₋₆₋Alkoxy-C₁₋₆-alkoxy, C₁₋₆-Alkoxy-C₁₋₆-alkoxy-C₁₋₆-alkyl, Hydroxy-C₁₋₆-alkoxy, Di-C₁₋₆-alkylamino, 2,3-Dihydroxypropoxy, 2,3-Dihydroxypropoxy-C₁₋₆-alkoxy, 2,3-Dimethoxypropoxy Methoxybenzyloxy, Hydroxybenzyloxy, Phenethyloxy, Methylendioxybenzyloxy, Dioxolanyl-C₁₋₆₋alkoxy, Cyclopropyl-C₁₋₆-alkyl, Cyclopropyl-C₁₋₆-alkoxy, Hydroxy-C₁₋₆-alkoxy, Carbamoyloxy-C₁₋₆-alkoxy, Pyridyl-carbamoyloxy-C₁₋₆-alkoxy, Benzoyloxy-C₁₋₆-alkoxy, Picolyloxy, C₁₋₆₋Alkoxy-carbonyl, C₀₋₆-Alkylcarbonylamino, C₀₋₆-Alkylcarbonylamino-C₁₋₆-alkyl, C₀₋₆-Alkylcarbonylamino-C₁₋₆-alkoxy, (N-C₁₋₆-Alkyl)-C₀₋₆-alkylcarbonylamino-C₁₋₆-alkyl, (N-C₁₋₆-Alkyl)-C₀₋₆-alkylcarbonylamino-C₁₋₆-alkoxy, C₃₋₈-Cycloalkylcarbonylamino-C₁₋₆-alkyl, C₃₋₈₋Cycloalkylcarbonylamino-C₁₋₆-alkoxy, C₁₋₆-Alkoxy-C₁₋₆-alkyl, Hydroxy-C₁₋₆-alkyl, Hydroxy-C₁₋₆₋alkoxy-C₁₋₆-alkyl, Hydroxy-C₁₋₆-alkoxy-C₁₋₆-alkoxy, C₁₋₆-Alkoxycarbonylamino-C₁₋₆-alkyl, C₁₋₆₋Alkoxycarbonylamino-C₁₋₆-alkoxy, C₁₋₆-Alkylaminocarbonylamino-C₁₋₆-alkyl, C₁₋₆-Alkylaminocarbonylamino-C₁₋₆-alkoxy, C₁₋₆-Alkylaminocarbonyl-C₁₋₆-alkyl, C₁₋₆-Alkylaminocarbonyl-C₁₋₆₋alkoxy, C₁₋₆-Alkylaminocarbonyl-C₁₋₆-alkoxy-C₁₋₆-alkyl, Di-C₁₋₆-alkylaminocarbonyl-C₁₋₆-alkyl, Di-C₁₋₆-alkylaminocarbonyl-C₁₋₆-alkoxy, C₁₋₆-Alkoxycarbonyloxy-C₁₋₆-alkyl, C₁₋₆-Alkylcarbonyloxy-C₁₋₆-alkyl, C₁₋₆-Alkylcarbonyloxy-C₁₋₆-alkoxy, Cyano-C₁₋₆-alkyl, Cyano-C₁₋₆-alkoxy, 2-Oxo-oxazolidinyl-C₁₋₆-alkyl, 2-Oxo-oxazolidinyl-C₁₋₆-alkoxy, C₁₋₆-Alkoxycarbonyl-C₁₋₆-alkyl, C₁₋₆₋Alkoxycarbonyl-C₁₋₆-alkoxy, C₁₋₆-Alkylsulfonylamino-C₁₋₆-alkyl, C₁₋₆-Alkylsulfonylamino-C₁₋₆₋alkoxy, (N-C₁₋₆-Alkyl)-C₁₋₆-alkylsulfonylamino-C₁₋₆-alkyl, (N-C₁₋₆-Alkyl)-C₁₋₆-alkylsulfonylamino-C₁₋₆-alkoxy, Amino-C₁₋₆-alkyl, Amino-C₁₋₆-alkoxy, C₁₋₆-Alkylamino-C₁₋₆-alkyl, C₁₋₆-Alkylamino-C₁₋₆-alkoxy, Di-C₁₋₆-alkylamino-C₁₋₆-alkyl, Di-C₁₋₆-alkylamino-C₁₋₆-alkoxy, C₁₋₆-Alkylsulfonyl-C₁₋₆-alkyl, C₁₋₆-Alkylsulfonyl-C₁₋₆-alkoxy, Carboxy-C₁₋₆-alkyl, Carboxy-C₁₋₆-alkoxy, Carboxy-C₁₋₆-alkoxy-C₁₋₆-alkyl, C₁₋₆-Alkoxy-C₁₋₆-alkyl-carbonyl, Acyl-C₁₋₆-alkoxy-C₁₋₆-alkyl, (N-C₁₋₆₋Alkyl)-C₁₋₆-alkoxy-carbonylamino, (N-Hydroxy)-C₁₋₆-alkylaminocarbonyl-C₁₋₆-alkyl, (N-Hydroxy)-C₁₋₆-alkylaminocarbonyl-C₁₋₆-alkoxy, (N-Hydroxy)-aminocarbonyl-C₁₋₆-alkyl, (N-Hydroxy)-aminocarbonyl-C₁₋₆-alkoxy, C₁₋₆-Alkoxy-aminocarbonyl-C₁₋₆-alkyl, C₁₋₆-Alkoxyaminocarbonyl-C₁₋₆-alkoxy, (N-C₁₋₆-Alkoxy)-C₁₋₆-alkylaminocarbonyl-C₁₋₆-alky), (N-C₁₋₆₋Alkoxy)-C₁₋₆-alkylaminocarbonyl-C₁₋₆-alkoxy, (N-Acyl)-C₁₋₆-alkoxy-C₁₋₆-alkylamino, C₁₋₆₋Alkoxy-C₁₋₆-alkyl-carbamoyl, (N-C₁₋₆-Alkyl)-C₁₋₆-alkoxy-C₁₋₆-alkyl-carbamoyl, C₁₋₆-Alkoxy-C₁₋₆₋alkyl-carbonyl, C₁₋₆-Alkoxy-C₁₋₆-alkyl-carbonylamino, (N-C₁₋₆-Alkyl)-C₁₋₆-alkoxy-C₁₋₆-alkylcarbonylamino, 1-C₁₋₆-Alkoxy-C₁₋₆-alkyl-imidazol-2-y), 1-C₁₋₆-Alkoxy-C₁₋₆-alkyl-tetrazol-5-yl, 5-C₁₋₆-Alkoxy-C₁₋₆-alkyl-tetrazol-1-yl, 2-C₁₋₆-Alkoxy-C₁₋₆-alkyl-4-oxo-imidazol-1-yl, Carbamoyl-C₁₋₆-alkyl, Carbamoyl-C₁₋₆-alkoxy, C₁₋₆-Alkyl-carbamoyl, Di-C₁₋₆-alkyl-carbamoyl, C₁₋₆-Alkylsulfonyl, C₁₋₆-Alkyl-amidinyl, Acetamidinyl-C₁₋₆-alkyl, O-Methyloximyl-C₁₋₆-alkyl, O,N-Dimethylhydroxylamino-C₁₋₆-alkyl, C₃₋₈-Cycloalkyl-C₁₋₆-alkanoyl, Aryl-C₁₋₆-alkanoyl, Heterocyclyl-C₁₋₆₋Alkanoyl; sowie gegebenenfalls durch Halogen, C₁₋₆-Alkyl, C₁₋₆-Alkoxy oder Dihydroxy-C₁₋₆₋alkylaminocarbonyl substituiertes Pyridyl, Pyridyloxy, Pyridylthio, Pyridylamino, Pyridyl-C₁₋₆₋alkyl, Pyridyl-C₁₋₆-alkoxy, Pyrimidinyl, Pyrimidinyloxy, Pyrimidinylthio, Pyrimidinylamino, Pyrimidinyl-C₁₋₆-alkyl, Pyrimidinyl-C₁₋₆-alkoxy, Thienyl, Thienyl-C₁₋₆-alkyl, Thienyl-C₁₋₆-alkoxy, Furyl, Furyl-C₁₋₆-alkyl, Furyl-C₁₋₆-alkoxy.

Der Ausdruck Heterocyclyl bezeichnet mono- oder bicyclische, gesättigte und ungesättigte heterocyclische Reste mit 1 bis 4 Stickstoff- und/oder 1 oder 2 Schwefel- oder Sauerstoffatomen, die ein- oder mehrfach, insbesondere durch (im Falle ungesättigter Heterocyclylreste) Alkyl, Hydroxy, Alkoxy, Nitro oder Halogen oder durch Substituenten, wie oben für Arylreste definiert, substituiert sein können oder (im Falle gesättigter Heterocyclylreste) durch Alkyl oder Alkoxy substituiert sein können. Beispiele für Heterocyclyl-Reste sind Pyridyl, Thienyl, Pyrazinyl, Triazolyl, Imidazolyl, Benzthiazolyl, Furyl, Pyranyl, Tetrahydropyranyl, Azetidinyl, Pyrimidinyl, Morpholinyl, Chinazolinyl, Chinolyl, Chinoxalinyl, Isochinolyl, Tetrahydrochinolyl, Tetrahydroisochinolyl" Benzo[b]thienyl, Isobenzofuranyl, Benzoimidazolyl, 2-Oxo-benzoimidazolyl, Oxazolyl, Thiazolyl, Indolyl, Pyrrolyl, 2-Oxo-dihydro-benzo-[d][1,3]oxazinyl, 4-Oxo-dihydro-imidazolyl, 5-Oxo-4H-[1,2,4]triazinyl, 3-Oxo-4H-benzo[1,4]thiazinyl, Tetrahydro-chinoxalinyl, 1,1,3-Trioxo-dihydro-2H-1lambda*6*-benzo[1,4]thiazinyl, 1-Oxo-pyridyl, Dihydro-2H-benzo[1,4]oxazinyl, 2-Oxo-tetrahydrobenzo[e][1,4]diazepinyl, 2-Oxo-dihydro-benzo[e][1,4]diazepinyl, 1H-Pyrrolizinyl, Phthalazinyl, 1-Oxo-3H-isobenzofuranyl, 4-Oxo-3H-thieno[2,3-d]pyrimidinyl, 3-Oxo-4H-benzo[1,4]oxazinyl, [1,5]Naphthyridyl, Dihydro-2H-benzo[1,4]thiazinyl, 1,1-Dioxo-dihydro-2H-benzo[1,4]thiazinyl, 2-0xo-1 H-pyrido[2,3-b][1,4]oxazinyl, Dihydro-1 H-pyrido[2,3-b][1,4]oxazinyl, 1 H-Pyrrolo[2,3-b]pyridyl, Benzo[1,3]dioxolyl, Benzooxazolyl, 2-Oxo-benzooxazolyl, 2-Oxo-1,3-dihydroindolyl, 2,3-Dihydroindolyl, 2-Oxo-dihydro-1 H-chinazolinyl, Indazolyl oder Benzofuranyl. Beispiele substituierter Heterocyclreste sind Nitrobenzthiazolyl, Phenyl-tetrazolyl, Phenyl-oxadiazolyl, Phenyl-piperidinyl, Phenyl-piperazinyl, Phenyl-pyrrolidinyl, Thienyl-oxadiazolyl, Furanyloxadiazolyl, Benzyl-oxadiazolyl oder Phenyl-oxazolyl. Beispiele gesättigter Heterocyclyl-Reste sind Dioxolanyl, Dioxanyl, Dithiolanyl, Dithianyl, Pyrrolidinyl, Piperidinyl, Piperazinyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, Tetrahydropyranyl, 2-Oxo-imidazolidinyl, 2-Oxo-oxazolidinyl, 2-Oxo-piperidinyl, 2-Oxo-pyrrolidinyl, 2-Oxo-[1,3]oxazinyl, 2-Oxo-azepanyl, 2-Oxo-tetrahydro-pyrimidinyl und dergleichen.

Im Falle von R¹, R^{4a} und R⁹ können die Aryl, Aroyl- und Heterocyclylreste zusätzlich noch durch Heterocyclylalkyl, Heterocyclylalkoxy, Heterocyclylalkoxyalkyl oder Heterocyclyl wie beispielsweise Piperidinoalkyl, Piperidinoalkoxy, Piperidinoalkoxyalkyl, Morpholinoalkyl, Morpholinoalkoxy, Morpholinoalkoxyalkyl, Piperazinoalkyl, Piperazinoalkoxy, Piperazinoalkoxyalkyl, [1,2,4]-Triazol-1-yl-alkyl, [1,2,4]-Triazol-1-yl-alkoxy, [1,2,4]-Triazol-4-yl-alkyl, [1,2,4]-Triazol-4-yl-alkoxy, [1,2,4]-Oxadiazol-5-yl-alkyl, [1,2,4]-Oxadiazol-5-yl-alkoxy, 3-Methyl-[1,2,4]-oxadiazol-5-yl-alkyl, 3-Methyl-[1,2,4]-oxadiazol-5-yl-alkoxy, 5-Methyl-[1,2,4]-oxadiazol-3-yl-alkyl, 5-Methyl-[1,2,4]-oxadiazol-3-yl-alkoxy, Tetrazol-1-yl-alkyl, Tetrazol-1-yl-alkoxy, Tetrazol-2-yl-alkyl, Tetrazol-2-yl-alkoxy, Tetrazol-5-yl-alkyl, Tetrazol-5-yl-alkoxy, 5-Methyl-tetrazol-1-yl-alkyl, 5-Methyl-tetrazol-1-yl-alkoxy, Thiazol-4-yl-alkyl, Thiazol-4-yl-alkoxy, Oxazol-4-yl-alkyl, Oxazol-4-yl-alkoxy, 2-Oxo-pyrrolidinyl-alkyl, 2-Oxo-pyrrolidinyl-alkoxy, Imidazolyl-alkyl, Imidazolyl-alkoxy, 2-Methyl-imidazolyl-alkyl, 2-Methyl-imidazolyl-alkoxy, N-Methylpiperazinoalkyl, N-Methylpiperazinoalkoxy, N-Methylpiperazinoalkoxyalkyl, sowie Alkylaminoalkyl, Alkylaminoalkoxy, Alkylaminoalkoxyalkyl, Mono- und Polyhydroxy-alkyl, -alkoxy, -alkoxyalkyl und -alkoxyalkoxy, Carbamoylalkyloxy, C₁₋₆-Alkoxy, Amino-C₁₋₆₋alkoxy, Hydroxy-C₁₋₆-alkoxy, Dioxolanyl, Dioxanyl, Dithiolanyl, Dithianyl, Pyrrolidinyl, Piperidinyl, Piperazinyl, Pyrrolyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 3-Acetamidomethylpyrrolidinyl, 3-C₁₋₆-Alkoxy-C₁₋₆-alkyl-pyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl, 2-Oxo-tetrahydro-pyrimidinyl und dergleichen oder durch den Rest -O-CH₂CH(OH)CH₂NRx substituiert sein, wobei NRx einen mono- oder di-C₁₋₆-Alkyl-amino-, Piperidino-, Morpholino-, Piperazino- oder N-Methylpiperazinorest darstellt.

Beispiele für durch NR⁵R⁶ dargestellte 5- und 6-gliedrige heterocyclische Ringe sind Pyrrolidinyl, Piperidinyl, Piperazinyl, Morpholinyl, Thiomorpholinyl, 2-Hydroxymethylpyrrolidinyl, 3-Hydroxypyrrolidinyl, 3,4-Dihydroxypyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl, 2-Oxo-tetrahydro-pyrimidinyl und dergleichen. Beispiele für durch CR⁷R⁸ dargestellte 3-7-gliedrige Ringe sind Cyclopentyl, Cyclohexyl, Cycloheptyl, 1,3-Dioxolanyl, 1,3-Dioxanyl, 1,3-Dithiolanyl und 1,3-Dithianyl.

Der Ausdruck Polyhydroxy-alkyl bezeichnet C₁-C₇-Alkylreste, die mit 2-6 Hydroxy-Gruppen substituiert sein können, wie z.B. Glyceryl, Arabityl, Sorbityl usw.

Halogen oder Halo bezeichnet beispielsweise Fluor, Chlor oder Brom beziehungsweise einen durch Fluor, Chlor oder Brom ein-, mehrfach oder vollständig substituierten Rest.

Die Verbindungen der Formel (I) oder Formel (II) besitzen mindestens zwei asymmetrische Kohlenstoffatome und können deshalb in Form von optisch reinen Diastereomeren, Diastereomerengemischen, diastereomeren Racematen, Gemischen von diastereomeren Racematen oder als Mesoverbindungen vorliegen. Die Erfindung umfasst alle diese Formen. Diastereomerengemische, diastereomere Racemate oder Gemische von diastereomeren Racematen können nach üblichen Methoden aufgetrennt werden, z.B. durch Säulenchromatographie, Dünnschichtchromatographie, HPLC und dergleichen.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen.

Die Verbindungen der Formeln (I) und (II) schliessen auch solche Verbindungen ein, bei welchen ein oder mehrere Atome durch ihre stabilen, nicht radioaktiven Isotope ersetzt sind; beispielsweise ein Wasserstoffatom durch Deuterium.

Die im folgenden genannten Verbindungsgruppen sind nicht als geschlossen zu betrachten, sondern es können in sinnvoller Weise, z.B. zur Ersetzung allgemeiner durch speziellere Definitionen, Teile dieser Verbindungsgruppen untereinander oder durch die oben gegebenen Definitionen ausgetauscht oder weggelassen werden.

Bevorzugte erfindungsgemässe Verbindungen sind solche der allgemeinen Formeln (IA) oder (IIA) 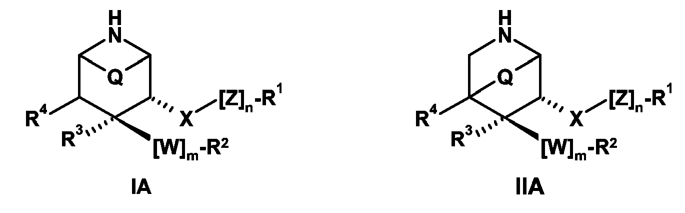 worin R¹, R², R³, R⁴, Q, W, X und Z, n und m die für die Verbindungen der Formeln (I) bzw. (II) oben angegebene Bedeutung haben.

Eine weitere, bevorzugte Gruppe von Verbindungen der Formel (I) oder (II), bzw. besonders bevorzugt der Formel (IA) oder (IIA), sind Verbindungen worin
R¹ die Bedeutung wie für (A), (B), (C), (D), (E), (F) oder (G), besonders bevorzugt wie für (A), (B), (C) oder (D) und ganz besonders bevorzugt wie für (A), angegeben hat;
R² Phenyl, Cyclohexyl, Tetrazolyl, durch Halogen, Hydroxy, Cyano, Trifluormethyl, C₁₋₆-Alkyl, Halo-C₁₋₆-alkyl, Hydroxy-C₁₋₆-alkyl, C₁₋₆-Alkoxy-C₁₋₆-alkyl, Cyano-C₁₋₆-alkyl, Carboxy-C₁₋₆- alkyl, C₁₋₆-Alkanoyloxy-C₁₋₆-alkyl, C₁₋₆-Alkoxycarbonyloxy-C₁₋₆-alkyl, C₁₋₆-Alkoxycarbonyl, C₁₋₆- Alkoxy, C₁₋₆-Alkylendioxy, oder durch einen Rest L1-T1-L2-T2-L3-T3-L4-T4-L5-U substituiertes Phenyl, Cyclohexyl oder Tetrazolyl; oder Naphthyl oder Acenaphthyl;
L1, L2, L3, L4 und L5 unabhängig voneinander eine Bindung, C₁₋₈-Alkylen, C₂₋₈-Alkenylen oder C₂₋₈-Alkinylen darstellen, oder abwesend sind;
T1, T2, T3 und T4 unabhängig voneinander
(a) eine Bindung darstellen, oder abwesend sind, oder eine der Gruppen
(b) -CH(OH)-
(c) -CH(OR⁶)-
(d) -CH(NR⁵R⁶)-
(e) -CO-
(f) -CR⁷R⁸-
(g) -O- oder -NR⁶-
(h) -S(O)₀₋₂-
(l) -SO₂NR⁶-
(j) -NR⁶SO₂-
(k) -CONR⁶-
(l) -NR⁶CO-
(m) -O-CO-
(n) -CO-O-
(o) -O-CO-O-
(p) -O-CO-NR⁶-
(q) -N(R⁶)-CO-N(R⁶)-
(r) -N(R⁶)-CO-O-
(s) Pyrrolidinylen, Piperidinylen oder Piperazinylen
(t) -C(R¹¹)(R¹²)-
darstellen,
wobei die von (b)-(t) ausgehenden Bindungen zu einem gesättigten oder aromatischen C-Atom der benachbarten Gruppe führen, falls die Bindung von einem Heteroatom ausgeht, und wobei nicht mehr als zwei Gruppen (b)-(f), drei Gruppen (g)-(h) und eine Gruppe (i)-(t) anwesend sind;
R³ Wasserstoff, Hydroxy, C₁₋₆-Alkoxy oder C₂₋₆-Alkenyloxy;
R⁴ Wasserstoff, C₁₋₆-Alkyl, C₂₋₆-Alkenyl, C₁₋₆-Alkoxy, Hydroxy-C₁₋₆-alkyl, C₁₋₆-Alkoxy-C₁₋₆-alkyl oder Benzyl ist;
R⁵ und R⁶ Wasserstoff, C₁₋₆-Alkyl oder Acyl, oder gemeinsam mit dem N-Atom, an das sie gebunden sind, einen 5- oder 6-gliedrigen heterocyclischen Ring, der ein zusätzliches N-, O-oder S-Atom enthalten kann;
R⁷ und R⁸ gemeinsam mit dem C-Atom, an das sie gebunden sind, einen 3-7-gliedrigen Ring darstellen, der ein oder zwei -O- oder -S- Atome enthalten kann;
R⁹ Wasserstoff, C₁₋₆-Alkyl, Acyl oder Arylalkyl ist;
U Wasserstoff, C₁₋₆-alkyl, C₃₋₈-Cycloalkyl, Cyano, Aryl oder Heterocyclyl;
Q Ethylen oder abwesend ist (Formel (I)) und Ethylen oder Methylen ist (Formel (II));
X Sauerstoff, Schwefel oder eine Gruppe -CH₂-, -CHOR⁹-, -O-CO-, >CO oder -O-CH-R¹¹⁻CO-NR⁹- ist;
W Sauerstoff oder Schwefel darstellen kann, wenn R³ Wasserstoff ist;
Z C₁₋₆-Alkylen oder -Alk-O- ;
n 1 die Zahl 0 oder 1 ist;
m die Zahl 0 oder 1 ist;
und pharmazeutisch anwendbare Salze davon.

Weiterhin sind Verbindungen der Formeln (I), (IA), (II) und (IIA) bevorzugt, in denen W abwesend ist (m die Zahl 0 ist), und solche, in denen Q abwesend ist.
Bevorzugte Reste R¹, insbesondere in Verbindungen der Gruppen (D), (E), (F) oder (G), sind Phenyl und durch 1-3 Reste ausgewählt aus C₁₋₆-Alkyl, C₁₋₆-Alkoxy, Halogen, Hydroxy, Hydroxy-C₁₋₆-alkoxy, C₁₋₆-Alkoxy-C₁₋₆-alkoxy, C₁₋₆-Alkylsulfinyl, Carbamoyl, Cyano, Trifluormethyl, Trifluormethoxy, Carboxy, C₁₋₆-Alkoxy-carbonyl, C₁₋₆-Alkoxy-C₁₋₆-alkoxy-C₁₋₆-alkyl, C₀₋₆-Alkylcarbonylamino, C₀₋₆-Alkylcarbonylamino-C₁₋₆-alkyl, C₀₋₆-Alkylcarbonylamino-C₁₋₆₋alkoxy, (N-C₁₋₆-Alkyl)-C₀₋₆-alkylcarbonylamino-C₁₋₆-alkyl, (N-C₁₋₆-Alkyl)-C₀₋₆-alkylcarbonylamino-C₁₋₆-alkoxy, C₃₋₈-Cycloalkylcarbonylamino-C₁₋₆-alkyl, C₃₋₈-Cycloalkylcarbonylamino-C₁₋₆-alkoxy, C₁₋₆-Alkoxy-C₁₋₆-alkyl, Hydroxy-C₁₋₆-alkyl, Hydroxy-C₁₋₆-alkoxy-C₁₋₆-alkyl, Hydroxy-C₁₋₆-alkoxy-C₁₋₆-alkoxy, C₁₋₆-Alkoxycarbonylamino-C₁₋₆-alkyl, C₁₋₆-Alkoxycarbonylamino-C₁₋₆-alkoxy, C₁₋₆-Alkylaminocarbonylamino-C₁₋₆-alkyl, C₁₋₆-AlkylaminocarbonylaminoC₁₋₆-alkoxy, C₁₋₆-Alkylaminocarbonyl-C₁₋₆-alkyl, C₁₋₆-Alkylaminocarbonyl-C₁₋₆-alkoxy, C₁₋₆₋Alkylaminocarbonyl-C₁₋₆-alkoxy-C₁₋₆-alkyl, Di-C₁₋₆-alkylaminocarbonyl-C₁₋₆-alkyl, Di-C₁₋₆₋alkylaminocarbonyl-C₁₋₆-alkoxy, C₁₋₆-Alkylcarbonyloxy-C₁₋₆-alkyl, C₁₋₆-Alkylcarbonyloxy-C₁₋₆₋alkoxy, Cyano-C₁₋₆-alkyl, Cyano-C₁₋₆-alkoxy, 2-Oxo-oxazolidinyl-C₁₋₆-alkyl, 2-Oxo-oxazolidinyl-C₁₋₆-alkoxy, C₁₋₆-Alkoxycarbonyl-C₁₋₆-alkyl, C₁₋₆-Alkoxycarbonyl-C₁₋₆-alkoxy, C₁₋₆₋Alkylsulfonylamino-C₁₋₆-alkyl, C₁₋₆-Alkylsulfonylamino-C₁₋₆-alkoxy, (N-C₁₋₆-Alkyl)-C₁₋₆-alkyl-sulfonylamino-C₁₋₆-alkyl, (N-C₁₋₆-Alkyl)-C₁₋₆-alkylsulfonylamino-C₁₋₆-alkoxy, Amino-C₁₋₆-alkyl, Amino-C₁₋₆-alkoxy, C₁₋₆-Alkylamino-C₁₋₆-alkyl, C₁₋₆-Alkylamino-C₁₋₆-alkoxy, Di-C₁₋₆-alkylamino-C₁₋₆-alkyl, Di-C₁₋₆-alkylamino-C₁₋₆-alkoxy, C₁₋₆-Alkylsulfonyl-C₁₋₆-alkyl, C₁₋₆-Alkylsulfonyl-C₁₋₆₋alkoxy, Carboxy-C₁₋₆-alkyl, Carboxy-C₁₋₆-alkoxy, Carboxy-C₁₋₆-alkoxy-C₁₋₆-alkyl, C₁₋₆-Alkoxy-C₁₋₆-alkyl-carbonyl, Acyl-C₁₋₆-alkoxy-C₁₋₆-alkyl, (N-C₁₋₆-Alkyl)-C₁₋₆-alkoxy-carbonylamino, (N-Hydroxy)-C₁₋₆-alkylaminocarbonyl-C₁₋₆-alkyl, (N-Hydroxy)-C₁₋₆-alkylaminocarbonyl-C₁₋₆₋alkoxy, (N-Hydroxy)-aminocarbonyl-C₁₋₆-alkyl, (N-Hydroxy)-aminocarbonyl-C₁₋₆-alkoxy, C₁₋₆₋Alkoxy-aminocarbonyl-C₁₋₆-alkyl, C₁₋₆-Alkoxy-aminocarbonyl-C₁₋₆-alkoxy, (N-C₁₋₆-Alkoxy)-C₁₋₆₋alkylaminocarbonyl-C₁₋₆-alkyl, (N-C₁₋₆-Alkoxy)-C₁₋₆-alkylaminocarbonyl-C₁₋₆-alkoxy, (N-Acyl)-C₁₋₆-alkoxy-C₁₋₆-alkylamino, C₁₋₆-Alkoxy-C₁₋₆-alkyl-carbamoyl, (N-C₁₋₆-Alkyl)-C₁₋₆-alkoxy-C₁₋₆₋alkyl-carbamoyl, C₁₋₆-Alkoxy-C₁₋₆-alkyl-carbonyl, C₁₋₆-Alkoxy-C₁₋₆-alkyl-carbonylamino, (N-C₁₋₆-Alkyl)-C₁₋₆-alkoxy-C₁₋₆-alkyl-carbonylamino, 1-C₁₋₆-Alkoxy-C₁₋₆-alkyl-imidazol-2-yl, 1-C₁₋₆-Alkoxy-C₁₋₆-alkyl-tetrazol-5-yl, 5-C₁₋₆-Alkoxy-C₁₋₆-alkyl-tetrazol-1-yl, 2-C₁₋₆-Alkoxy-C₁₋₆₋alkyl-4-oxo-imidazol-1-yl, Carbamoyl-C₁₋₆-alkyl, Carbamoyl-C₁₋₆-alkoxy, C₁₋₆-Alkyl-carbamoyl, Di-C₁₋₆-alkyl-carbamoyl, C₁₋₆-Alkyl-sulfonyl, Piperidinoalkyl, Piperidinoalkoxy, Piperidinoalkoxyalkyl, Morpholinoalkyl, Morpholinoalkoxy, Morpholinoalkoxyalkyl, Piperazinoalkyl, Piperazinoalkoxy, Piperazinoalkoxyalkyl, [1,2,4]-Triazol-1-yl-alkyl, [1,2,4]-Triazol-1-yl-alkoxy, [1,2,4]-Triazol-4-yl-alkyl, [1,2,4]-Triazol-4-yl-alkoxy, [1,2,4]-Oxadiazol-5-yl-alkyl, [1,2,4]-Oxadiazol-5-yl-alkoxy, 3-Methyl-[1,2,4]-oxadiazol-5-yl-alkyl, 3-Methyl-[1,2,4]-oxadiazol-5-yl-alkoxy, 5-Methyl-[1,2,4]-oxadiazol-3-yl-alkyl, 5-Methyl-[1,2,4]-oxadiazol-3-yl-alkoxy, Tetrazol-1-yl-alkyl, Tetrazol-1-yl-alkoxy, Tetrazol-2-yl-alkyl, Tetrazol-2-yl-alkoxy, Tetrazol-5-yl-alkyl, Tetrazol-5-yl-alkoxy, 5-Methyl-tetrazol-1-yl-alkyl, 5-Methyl-tetrazol-1-yl-alkoxy, Thiazol-4-yl-alkyl, Thiazol-4-yl-alkoxy, Oxazol-4-yl-alkyl, Oxazol-4-yl-alkoxy, 2-Oxo-pyrrolidinyl-alkyl, 2-Oxo-pyrrolidinyl-alkoxy, Imidazolyl-alkyl, Imidazolyl-alkoxy, 2-Methyl-imidazolyl-alkyl, 2-Methyl-imidazolyl-alkoxy, N-Methylpiperazinoalkyl, N-Methylpiperazinoalkoxy, N-Methylpiperazinoalkoxyalkyl, Pyrrolidinyl, Piperidinyl, Piperazinyl, Pyrrolyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 3-Acetamidomethylpyrrolidinyl, 3-C₁₋₆-Alkoxy-C₁₋₆-alkyl-pyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl und 2-Oxo-tetrahydro-pyrimidinyl substituiertes Phenyl.

Weitere bevorzugte Reste R¹, insbesondere in Verbindungen der Gruppen (D), (E), (F) oder (G), sind Naphthyl und durch 1-3 Reste ausgewählt aus Hydroxy, Oxo, Halogen, Carbamoyl, Carboxy, C₁₋₆-Alkoxy, Hydroxy-C₁₋₆-alkoxy, C₁₋₆-Alkoy-C₁₋₆-alkoxy, Di-C₁₋₆-Alkylamino, 2,3-Dihydroxypropoxy, 2,3-Dihydroxypropoxy-C₁₋₆-alkoxy, 2,3-Dimethoxypropoxy, Methoxybenzyloxy, Hydroxybenzyloxy, Phenethyloxy, Methylendioxybenzyloxy, Dioxolanyl-C₁₋₆₋alkoxy, Cyclopropyl-C₁₋₆-alkoxy, Hydroxy-C₁₋₆-alkoxy, Pyridyl-carbamoyloxy-C₁₋₆-alkoxy, 3-Morpholino-2-hydroxypropoxy, Benzyloxy-C₁₋₆-alkoxy, Picolyloxy, C₁₋₆-Alkoxy-carbonyl, C₁₋₆-Alkoxy-C₁₋₆-alkoxy-C₁₋₆-alkyl, C₀₋₆-Alkylcarbonylamino, C₀₋₆-Alkylcarbonylamino-C₁₋₆₋alkyl, C₀₋₆-Alkylcarbonylamino-C₁₋₆-alkoxy, (N-C₁₋₆-Alkyl)-C₀₋₆-alkylcarbonylamino-C₁₋₆-alkyl, (N-C₁₋₆-Alkyl)-C₀₋₆-alkylcarbonylamino-C₁₋₆-alkoxy, C₃₋₈-Cycloalkylcarbonylamino-C₁₋₆-alkyl, C₃₋₈-Cycloalkylcarbonylamino-C₁₋₆-alkoxy, C₁₋₆-Alkoxy-C₁₋₆-alkyl, Hydroxy-C₁₋₆-alkyl, Hydroxy-C₁₋₆-alkoxy-C₁₋₆-alkyl, Hydroxy-C₁₋₆-alkoxy-C₁₋₆-alkoxy, C₁₋₆-Alkoxycarbonylamino-C₁₋₆-alkyl, C₁₋₆-Alkoxycarbonylamino-C₁₋₆-alkoxy, C₁₋₆-Alkylaminocarbonylamino-C₁₋₆-alkyl, C₁₋₆-Alkyl-aminocarbonylamino-C₁₋₆-alkoxy, C₁₋₆-Alkylaminocarbonyl-C₁₋₆-alkyl, C₁₋₆-Alkylaminocarbonyl-C₁₋₆-alkoxy, C₁₋₆-Alkylaminocarbonyl-C₁₋₆-alkoxy-C₁₋₆-alkyl, Di-C₁₋₆-alkylaminocarbonyl-C₁₋₆-alkyl, Di-C₁₋₆-alkylaminocarbonyl-C₁₋₆-alkoxy, C₁₋₆-Alkylcarbonyloxy-C₁₋₆-alkyl, C₁₋₆-Alkylcarbonyloxy-C₁₋₆-alkoxy, Cyano-C₁₋₆-alkyl, Cyano-C₁₋₆-alkoxy, 2-Oxo-oxazolidinyl-C₁₋₆-alkyl, 2-Oxo-oxazolidinyl-C₁₋₆-alkoxy, C₁₋₆-Alkoxycarbonyl-C₁₋₆-alkyl, C₁₋₆-Alkoxycarbonyl-C₁₋₆-alkoxy, C₁₋₆-Alkylsulfonylamino-C₁₋₆-alkyl, C₁₋₆-Alkylsulfonylamino-C₁₋₆-alkoxy, (N-C₁₋₆₋Alkyl)-C₁₋₆-alkylsulfonylamino-C₁₋₆-alkyl, (N-C₁₋₆-Alkyl)-C₁₋₆-alkylsulfonylamino-C₁₋₆-alkoxy, Amino-C₁₋₆-alkyl, Amino-C₁₋₆-alkoxy, C₁₋₆-Alkylamino-C₁₋₆-alkyl, C₁₋₆-Alkylamino-C₁₋₆-alkoxy, Di-C₁₋₆-alkylamino-C₁₋₆-alkyl, Di-C₁₋₆-alkylamino-C₁₋₆-alkoxy, C₁₋₆-Alkylsulfonyl-C₁₋₆-alkyl, C₁₋₆₋Alkylsulfonyl-C₁₋₆-alkoxy, Carboxy-C₁₋₆-alkyl, Carboxy-C₁₋₆-alkoxy, Carboxy-C₁₋₆-alkoxy-C₁₋₆₋alkyl, C₁₋₆-Alkoxy-C₁₋₆-alkyl-carbonyl, Acyl-C₁₋₆-alkoxy-C₁₋₆-alkyl, (N-C₁₋₆-Alkyl)-C₁₋₆-alkoxycarbonylamino, (N-Hydroxy)-C₁₋₆-alkylaminocarbonyl-C₁₋₆-alkyl, (N-Hydroxy)-C₁₋₆-alkylaminocarbonyl-C₁₋₆-alkoxy, (N-Hydroxy)-aminocarbonyl-C₁₋₆-alkyl, (N-Hydroxy)-aminocarbonyl-C₁₋₆₋alkoxy, C₁₋₆-Alkoxy-aminocarbonyl-C₁₋₆-alkyl, C₁₋₆-Alkoxy-aminocarbonyl-C₁₋₆-alkoxy, (N-C₁₋₆₋Alkoxy)-C₁₋₆-alkylaminocarbonyl-C₁₋₆-alkyl, (N-C₁₋₆-Alkoxy)-C₁₋₆-alkylaminocarbonyl-C₁₋₆₋alkoxy, (N-Acyl)-C₁₋₆-alkoxy-C₁₋₆-alkylamino, C₁₋₆-Alkoxy-C₁₋₆-alkyl-carbamoyl, (N-C₁₋₆-Alkyl)-C₁₋₆-alkoxy-C₁₋₆-alkyl-carbamoyl, C₁₋₆-Alkoxy-C₁₋₆-alkyl-carbonyl, C₁₋₆-Alkoxy-C₁₋₆-alkylcarbonylamino, (N-C₁₋₆-Alkyl)-C₁₋₆-alkoxy-C₁₋₆-alkyl-carbonylamino, 1-C₁₋₆-Alkoxy-C₁₋₆-alkylimidazol-2-yl, 1-C₁₋₆-Alkoxy-C₁₋₆-alkyl-tetrazol-5-yl, 5-C₁₋₆-Alkoxy-C₁₋₆-alkyl-tetrazol-1-yl, 2-C₁₋₆-Alkoxy-C₁₋₆-alkyl-4-oxo-imidazol-1-yl, Carbamoyl-C₁₋₆-alkyl, Carbamoyl-C₁₋₆-alkoxy, C₁₋₆₋Alkyl-carbamoyl, Di-C₁₋₆-alkyl-carbamoyl, C₁₋₆-Alkyl-sulfonyl, Piperidinoalkyl, Piperidinoalkoxy, Piperidinoalkoxyalkyl, Morpholinoalkyl, Morpholinoalkoxy, Morpholinoalkoxyalkyl, Piperazinoalkyl, Piperazinoalkoxy, Piperazinoalkoxyalkyl, [1,2,4]-Triazol-1-yl-alkyl, [1,2,4]-Triazol-1-yl-alkoxy, [1,2,4]-Triazol-4-yl-alkyl, [1,2,4]-Triazol-4-yl-alkoxy, [1,2,4]-Oxadiazol-5-yl-alkyl, [1,2,4]-Oxadiazol-5-yl-alkoxy, 3-Methyl-[1,2,4]-oxadiazol-5-yl-alkyl, 3-Methyl-[1,2,4]-oxadiazol-5-yl-alkoxy, 5-Methyl-[1,2,4]-oxadiazol-3-yl-alkyl, 5-Methyl-[1,2,4]-oxadiazol-3-yl-alkoxy, Tetrazol-1-yl-alkyl, Tetrazol-1-yl-alkoxy, Tetrazol-2-yl-alkyl, Tetrazol-2-yl-alkoxy, Tetrazol-5-yl-alkyl, Tetrazol-5-yl-alkoxy, 5-Methyl-tetrazol-1-yl-alkyl, 5-Methyl-tetrazol-1-yl-alkoxy, Thiazol-4-yl-alkyl, Thiazol-4-yl-alkoxy, Oxazol-4-yl-alkyl, Oxazol-4-yl-alkoxy, 2-Oxo-pyrrolidinyl-alkyl, 2-Oxo-pyrrolidinyl-alkoxy, Imidazolyl-alkyl, Imidazolyl-alkoxy, 2-Methyl-imidazolyl-alkyl, 2-Methyl-imidazolyl-alkoxy, N-Methylpiperazinoalkyl, N-Methylpiperazinoalkoxy, N-Methylpiperazinoalkoxyalkyl, Pyrrolidinyl, Piperidinyl, Piperazinyl, Pyrrolyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 3-Acetamidomethylpyrrolidinyl, 3-C₁₋₆-Alkoxy-C₁₋₆-alkyl-pyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl und 2-Oxo-tetrahydro-pyrimidinyl substituiertes Naphthyl; Tetrahydronaphthyl oder durch Methyl substituiertes Tetrahydronaphthyl und Indanyl.

Eine Gruppe besonders bevorzugter Reste R¹ umfasst die oben genannten substituierten Phenyl- und Naphthylreste, sowie Tetrahydronaphthyl und durch Methyl substituiertes Tetrahydronaphthyl.

Ebenfalls bevorzugte Reste R¹, insbesondere in Verbindungen der Gruppen (D), (E), (F) oder (G), sind Pyridyl, Benzoimidazolyl, Di-C₁₋₆-alkoxypyrimidinyl, 2- und 5-Benzo[b]thienyl, 6- und 7-Chinolyl, 6- und 7-Isochinolyl, 6- und 7-Tetrahydrochinolyl, 6- und 7-Tetrahydroisochinolyl, 6-Chinoxalinyl, 6- und 7-Chinazolinyl, Indolyl, Dihydro-2H-benzo[1,4]oxazinyl, 3-Oxo-4H-benzo[1,4]oxazinyl, 2-Oxo-benzooxazolyl, 2-Oxo-1,3-dihydroindolyl, 2,3-Dihydroindolyl, Indazolyl und Benzofuranyl sowie durch 1-3 Reste ausgewählt aus Hydroxy, Oxo, Halogen, Carbamoyl, Carboxy, C₁₋₆-Alkoxy, Hydroxy-C₁₋₆-alkoxy, C₁₋₆-Alkoxy-C₁₋₆-alkoxy, Di-C₁₋₆-Alkylamino, 2,3-Dihydroxypropoxy, 2,3-Dihydroxypropoxy-C₁₋₆-alkoxy, 2,3-Dimethoxypropoxy, Methoxybenzyloxy, Hydroxybenzyloxy, Phenethyloxy, Methylendioxybenzyloxy, Dioxolanyl-C₁₋₆-alkoxy, Cyclopropyl-C₁₋₆-alkoxy, Hydroxy-C₁₋₆-alkoxy, Pyridyl-carbamoyloxy-C₁₋₆-alkoxy, 3-Morpholino-2-hydroxypropoxy, Benzyloxy-C₁₋₆-alkoxy, Picolyloxy, C₁₋₆-Alkoxycarbonyl, C₁₋₆-Alkoxy-C₁₋₆-alkoxy-C₁₋₆-alkyl, C₀₋₆-Alkylcarbonylamino, C₀₋₆-Alkylcarbonylamino-C₁₋₆-alkyl, C₀₋₆-Alkylcarbonylamino-C₁₋₆-alkoxy, (N-C₁₋₆-Alkyl)-C₀₋₆-alkylcarbonylamino-C₁₋₆-alkyl, (N-C₁₋₆-Alkyl)-C₀₋₆-alkylcarbonylamino-C₁₋₆-alkoxy, C₃₋₈-Cycloalkylcarbonylamino-C₁₋₆-alkyl, C₃₋₈-Cycloalkylcarbonylamino-C₁₋₆-alkoxy, C₁₋₆-Alkoxy-C₁₋₆-alkyl, Hydroxy-C₁₋₆₋alkyl, Hydroxy-C₁₋₆-alkoxy-C₁₋₆-alkyl, Hydroxy-C₁₋₆-alkoxy-C₁₋₆-alkoxy, C₁₋₆-Alkoxycarbonylamino-C₁₋₆-alkyl, C₁₋₆-Alkoxycarbonylamino-C₁₋₆-alkoxy, C₁₋₆-Alkylaminocarbonylamino-C₁₋₆₋alkyl, C₁₋₆-Alkylaminocarbonylamino-C₁₋₆-alkoxy, C₁₋₆-Alkylaminocarbonyl-C₁₋₆-alkyl, C₁₋₆₋Alkylaminocarbonyl-C₁₋₆-alkoxy, C₁₋₆-Alkylaminocarbonyl-C₁₋₆-alkoxy-C₁₋₆-alkyl, Di-C₁₋₆₋alkylaminocarbonyl-C₁₋₆-alkyl, Di-C₁₋₆-alkylaminocarbonyl-C₁₋₆-alkoxy, C₁₋₆-Alkylcarbonyloxy-C₁₋₆-alkyl, C₁₋₆-Alkylcarbonyloxy-C₁₋₆-alkoxy, Cyano-C₁₋₆-alkyl, Cyano-C₁₋₆-alkoxy, 2-Oxo-oxazolidinyl-C₁₋₆-alkyl, 2-Oxo-oxazolidinyl-C₁₋₆-alkoxy, C₁₋₆-Alkoxycarbonyl-C₁₋₆-alkyl, C₁₋₆₋Alkoxycarbonyl-C₁₋₆-alkoxy, C₁₋₆-Alkylsulfonylamino-C₁₋₆-alkyl, C₁₋₆-Alkylsulfonylamino-C₁₋₆₋alkoxy, (N-C₁₋₆-Alkyl)-C₁₋₆-alkylsulfonylamino-C₁₋₆-alkyl, (N-C₁₋₆-Alkyl)-C₁₋₆-alkylsulfonylamino-C₁₋₆-alkoxy, Amino-C₁₋₆-alkyl, Amino-C₁₋₆-alkoxy, C₁₋₆-Alkylamino-C₁₋₆-alkyl, C₁₋₆-Alkylamino-C₁₋₆-alkoxy, Di-C₁₋₆-alkylamino-C₁₋₆-alkyl, Di-C₁₋₆-alkylamino-C₁₋₆-alkoxy, C₁₋₆-Alkylsulfonyl-C₁₋₆-alkyl, C₁₋₆-Alkylsulfonyl-C₁₋₆-alkoxy, Carboxy-C₁₋₆-alkyl, Carboxy-C₁₋₆-alkoxy, Carboxy-C₁₋₆-alkoxy-C₁₋₆-alkyl, C₁₋₆-Alkoxy-C₁₋₆-alkyl-carbonyl, Acyl-C₁₋₆-alkoxy-C₁₋₆-alkyl, (N-C₁₋₆₋Alkyl)-C₁₋₆-alkoxy-carbonylamino, (N-Hydroxy)-C₁₋₆-alkylaminocarbonyl-C₁₋₆-alkyl, (N-Hydroxy)-C₁₋₆-alkylaminocarbonyl-C₁₋₆-alkoxy, (N-Hydroxy)-aminocarbonyl-C₁₋₆-alkyl, (N-Hydroxy)-aminocarbonyl-C₁₋₆-alkoxy,C₁₋₆-Alkoxy-aminocarbonyl-C₁₋₆-alkyl, C₁₋₆-Alkoxyaminocarbonyl-C₁₋₆-alkoxy, (N-C₁₋₆-Alkoxy)-C₁₋₆-alkylaminocarbonyl-C₁₋₆-alkyl, (N-C₁₋₆₋Alkoxy)-C₁₋₆-alkylaminocarbonyl-C₁₋₆-alkoxy, (N-Acyl)-C₁₋₆-alkoxy-C₁₋₆-alkylamino, C₁₋₆₋Alkoxy-C₁₋₆-alkyl-carbamoyl, (N-C₁₋₆-Alkyl)-C₁₋₆-alkoxy-C₁₋₆-alkyl-carbamoyl, C₁₋₆-Alkoxy-C₁₋₆₋alkyl-carbonyl, C₁₋₆-Alkoxy-C₁₋₆-alkyl-carbonylamino, (N-C₁₋₆-Alkyl)-C₁₋₆-alkoxy-C₁₋₆-alkylcarbonylamino, 1-C₁₋₆-Alkoxy-C₁₋₆-alkyl-imidazol-2-yl, 1-C₁₋₆-Alkoxy-C₁₋₆-alkyl-tetrazol-5-yl, 5-C₁₋₆-Alkoxy-C₁₋₆-alkyl-tetrazol-1-yl, 2-C₁₋₆-Alkoxy-C₁₋₆-alkyl-4-oxo-imidazol-1-yl, Carbamoyl-C₁₋₆-alkyl, Carbamoyl-C₁₋₆-alkoxy, C₁₋₆-Alkyl-carbamoyl, Di-C₁₋₆-alkyl-carbamoyl, C₁₋₆-Alkylsulfonyl, Piperidinoalkyl, Piperidinoalkoxy, Piperidinoalkoxyalkyl, Morpholinoalkyl, Morpholinoalkoxy, Morpholinoalkoxyalkyl, Piperazinoalkyl, Piperazinoalkoxy, Piperazinoalkoxyalkyl, [1,2,4]-Triazol-1-yl-alkyl, [1,2,4]-Triazol-1-yl-alkoxy, [1,2,4]-Triazol-4-yl-alkyl, [1,2,4]-Triazol-4-yl-alkoxy, [1,2,4]-Oxadiazol-5-yl-alkyl, [1,2,4]-Oxadiazol-5-yl-alkoxy, 3-Methyl-[1,2,4]-oxadiazol-5-yl-alkyl, 3-Methyl-[1,2,4]-oxadiazol-5-yl-alkoxy, 5-Methyl-[1,2,4]-oxadiazol-3-yl-alkyl, 5-Methyl-[1,2,4]-oxadiazol-3-yl-alkoxy, Tetrazol-1-yl-alkyl, Tetrazol-1-yl-alkoxy, Tetrazol-2-yl-alkyl, Tetrazol-2-yl-alkoxy, Tetrazol-5-yl-alkyl, Tetrazol-5-yl-alkoxy, 5-Methyl-tetrazol-1-yl-alkyl, 5-Methyl-tetrazol-1-yl-alkoxy, Thiazol-4-yl-alkyl, Thiazol-4-yl-alkoxy, Oxazol-4-yl-alkyl, Oxazol-4-yl-alkoxy, 2-Oxo-pyrrolidinyl-alkyl, 2-Oxo-pyrrolidinyl-alkoxy, Imidazolyl-alkyl, Imidazolyl-alkoxy, 2-Methyl-imidazolyl-alkyl, 2-Methyl-imidazolyl-alkoxy, N-Methylpiperazinoalkyl, N-Methylpiperazinoalkoxy, N-Methylpiperazinoalkoxyalkyl, Pyrrolidinyl, Piperidinyl, Piperazinyl, Pyrrolyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 3-Acetamidomethylpyrrolidinyl, 3-C₁₋₆-Alkoxy-C₁₋₆-alkyl-pyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl und 2-Oxo-tetrahydro-pyrimidinyl substituiertes 6- und 7-Chinolyl, 6- und 7-Isochinolyl, 6- und 7-Tetrahydrochinolyl, 6- und 7-Tetrahydroisochinolyl, 6-Chinoxalinyl, 6- und 7-Chinazolinyl, Indolyl, Dihydro-3H-benzo[1,4]oxazinyl, 3-Oxo-4H-benzo[1,4]oxazinyl, 2-Oxo-benzooxazolyl, 2-Oxo-1,3-dihydroindolyl, 2,3-Dihydroindolyl, Indazolyl und Benzofuranyl.

Bevorzugte Reste R² sind Phenyl und, durch Halogen, Hydroxy, Cyano, Trifluormethyl, C₁₋₆₋Alkyl, Halo-C₁₋₆-alkyl, Hydroxy-C₁₋₆-alkyl, C₁₋₆-Alkoxy-C₁₋₆-alkyl, Cyano-C₁₋₆-alkyl, Carboxy-C₁₋₆-alkyl, C₁₋₆-Alkylcarbonyloxy-C₁₋₆-alkyl, C₁₋₆-Alkoxycarbonyloxy-C₁₋₆-alkyl, C₁₋₆-Alkoxycarbonyl, C₁₋₆-Alkoxy oder C₁₋₆-Alkylendioxy substituiertes Phenyl.

Ebenfalls bevorzugte Reste R² sind durch einen Rest L1-T1-L2-T2-L3-T3-L4-T4-L5-U substituiertes Phenyl, wobei L1 und L2 vorzugsweise abwesend oder C₁₋₈-Alkylen sind und L3 abwesend ist und U Wasserstoff, C₁₋₆-Alkyl, C₃₋₈-Cycloalkyl, Phenyl-piperidinyl, Phenyl-piperazinyl, Phenyl-pyrrolidinyl, Phenyl, durch C₁₋₆-Alkyl, C₁₋₆-Alkoxy, C₁₋₆-Alkylthio, C₁₋₆₋Alkylsulfinyl, C₁₋₆-Alkylendioxy, Halogen, Benzoyl-C₁₋₆-alkyl, Halogen-C₁₋₆-alkyl, C₁₋₆₋Alkanoyloxy oder Hydroxy substituiertes Phenyl oder Phenyl-pyrrolidinyl, Naphthyl, Pyridyl, Thienyl, Pyrazinyl, Triazolyl, Imidazolyl, Phenyl-oxadiazolyl, Thienyl-oxadiazolyl, Furyloxadiazolyl, Phenyloxazolyl, Benzthiazolyl, Furyl, Pyrimidinyl, Nitrobenzthiazolyl, Phenyl-tetrazolyl, Piperidinyl, Tetrahydropyranyl, Morpholinyl oder Indolyl darstellt.

Bei den Gruppen T1-T4 sind die Bedeutungen (a)-(c), (e)-(h), (k)-(n) und (r)-(t) bevorzugt. Beispiele besonders bevorzugter Reste R² sind Phenyl oder durch 2-Benzothiazolyl-thio-C₁₋₆-alkyl, 2-Benzyloxy-3-methoxypropoxy, 2-Benzoyloxy-3-methoxypropoxy, 2,3-Dihydroxypropoxy, 2-Hydroxy-3-benzylamino-propoxy, 2-Hydroxy-3-phenoxypropoxy, 2-Hydroxy-3-phenylthiopropoxy, 2-Methoxy-3-phenoxypropoxy, 2-Methoxy-3-benzyloxypropoxy, 2-Methyl-3-fluor-phenylbutyryloxy-C₁₋₆-alkoxy, 2-Methyl-3-phenoxypropoxy, 2-C₁₋₆-Alkenyloxy-4-phenylbutyl, 3,4,5-Trimethoxyphenyl-oxadiazolyl-C₁₋₆₋alkoxy, 6-Nitro-2-benzothiazolyl-thio-C₁₋₆-alkyl, Benzamido-C₁₋₆-alkoxy, Benzamido-C₁₋₆-alkyl, Benzo[1,3]dioxo)y)oxy-C₁₋₆-alkoxy, Benzoyl-C₁₋₆-alkoxy und Ketale davon, Benzoyl-C₁₋₆-alkyl und Ketale dayon, Benzoyl-C₁₋₆-alkyl-aminocarbonyl-C₁₋₆-alkyl, Benzoyl-C₁₋₆-alkoxycarbonyl-C₁₋₆-alkyl, Benzoyl-C₁₋₆-alkyl-aminocarbonyl, Benzoyloxy, Benzoyloxy-C₁₋₆-alkyl-benzoyloxy-C₁₋₆-alkoxy, Benzoyloxy-C₁₋₆-alkoxy, Benzoyloxy-C₁₋₆-alkyl, Benzthiazolylthio-C₁₋₆-alkoxy, Benzthiazolylthio-C₁₋₆-alkyl, Benzylcarbamoyl-C₁₋₆-alkoxy, Benzyloxy-C₁₋₆-alkoxycarbonyloxy-C₁₋₆-alkyl, Benzyloxy-C₁₋₆-alkoxy, Benzylthio-C₁₋₆-alkoxy, Carbamoyloxy-C₁₋₆-alkoxy, Carbamoyloxy-C₁₋₆-alkyl, Carboxy-C₁₋₆-alkoxy, Carboxy-C₁₋₆-alkyl, Cyano, Cyano-C₁₋₆-alkoxy, Cyano-C₁₋₆-alkyl, Cyanophenyl-C₁₋₆-alkoxy, Cyclohexylcarbonyloxy-C₁₋₆-alkyl, Cyclohexyloxy-C₁₋₆-alkoxy, Cyclopropylcarbonyloxy-C₁₋₆-alkyl, Dioxolanyl-C₁₋₆-alkoxy, Furyl-oxadiazolyl-C₁₋₆₋alkoxy, Furoyloxy-C₁₋₆-alkoxy, Halo-phenoxy-C₁₋₆-alkyl, Halobenzoyl-C₁₋₆-alkoxy, Halobenzoyloxy-C₁₋₆-alkyl, Halobenzoyloxy-C₁₋₆-alkoxy, Halobenzyloxy-C₁₋₆-alkoxy, Halogen, Halogen-C₁₋₆-alkyl, Halophenoxy, Halophenoxy-C₁₋₆-alkoxy, Halophenyl-oxadiazolyl-C₁-₆₋alkoxy, Hydroxy, Hydroxy-benzoyloxy-C₁₋₆-alkyl, Hydroxy-benzoyloxy-C₁₋₆-alkoxy, Hydroxy-C₁₋₆-alkoxy, Hydroxy-C₁₋₆-alkyl, Imidazolylcarbonyloxy-C₁₋₆-alkyl, Methoxybenzoyl-C₁₋₆-alkyl, Methoxybenzyl-oxy-C₁₋₆-alkoxy, Methylendioxybenzoyl-C₁₋₆-alkoxy, Morpholino-C₁₋₆-alkoxy, Morpholinocarbonyloxy-C₁₋₆-alkoxy, Morpholinocarbonyloxy-C₁₋₆-alkyl, N-Methylaminophenylcarbonyloxy-C₁₋₆-alkyl, N-Methyl-benzylamino-C₁₋₆-alkoxy, N-Methylpyrrolylcarbonyloxy-C₁₋₆₋alkoxy, N-C₁₋₆-Alkylbenzamido-C₁₋₆-alkyl, Naphthyl-C₁₋₆-alkoxy, Nicotinoyloxy-C₁₋₆-alkoxy, Nicotinoyloxy-C₁₋₆-alkyl, C₁₋₆-Alkanoylbenzoyloxy-C₁₋₆-alkyl, C₁₋₆-Alkanoyloxy-C₁₋₆-alkoxy, C₁₋₆-Alkanoyloxy-C₁₋₆-alkyl, C₁₋₆-Alkenyl-benzyloxy-C₁₋₆-alkoxy, C₁₋₆-Alkenyloxy, C₁₋₆₋Alkenyloxy-benzyloxy-C₁₋₆-alkoxy, C₁₋₆-Alkoxy, C₁₋₆-Alkoxy-benzoyloxy-C₁₋₆-alkyl, C₁₋₆-Alkoxycarbonyl, C₁₋₆-Alkoxy-C₁₋₆-alkyl, C₁₋₆-Alkoxybenzoylamino-C₁₋₆-alkyl, C₁₋₆-Alkoxybenzylcarbonyloxy-C₁₋₆-alkyl, C₁₋₆-Alkoxy-benzyloxy-C₁₋₆-alkoxy, C₁₋₆-Alkoxy-benzylthio-C₁₋₆-alkoxy, C₁₋₆-Alkoxy-carbonyl-C₁₋₆-alkoxy, C₁₋₆-Alkoxy-carbonyl-C₁₋₆-alkyl, C₁₋₆-Alkoxyphenyloxadiazolyl-C₁₋₆-alkoxy, C₁₋₆-Alkoxy-phenyloxy-C₁₋₆-alkoxy, C₁₋₆-Alkyl, C₁₋₆-Alkyl-benzyloxy-C₁₋₆-alkoxy, C₁₋₆-Alkyl-phenoxy-C₁₋₆-alkoxy, C₁₋₆-Alkylendioxy, C₁₋₆-Alkylendioxybenzyloxy-C₁₋₆-alkoxy, C₁₋₆-Alkylsulfonylbenzoyl-C₁₋₆-alkoxy, C₁₋₆-Alkylthiobenzoyloxy-C₁₋₆-alkoxy, C₁₋₆₋Alkylthio-benzyloxy-C₁₋₆-alkoxy, Benzoyloxybenzyl-C₁₋₆-alkoxy, Hydroxybenzyl-C₁₋₆-alkoxy, C₁₋₆-Alkoxybenzyl-C₁₋₆-alkoxy, C₁₋₆-Alkoxybenzylcarbonyloxy-C₁₋₆-alkoxy, Phenoxybenzyloxy-C₁₋₆-alkoxy, Phenoxycarbonyl-C₁₋₆-alkyl, Phenoxy-C₁₋₆-alkenyloxy, Phenoxy-C₁₋₆₋alkinyloxy, Phenyl-C₁₋₆-alkanoylamino-C₁₋₆-alkyl, Phenyl-C₁₋₆-alkenyloxy, Phenyl-C₁₋₆-alkoxy, Phenyl-C₁₋₆-alkyl, Phenyl-C₁₋₆-alkylaminocarbonyl, Phenyl-C₁₋₆-alkylcarbonyl-C₁₋₆-alkoxy, Phenyl-C₁₋₆-alkylaminocarbonyl-C₁₋₆-alkyl, Phenylaminocarbonyloxy-C₁₋₆-alkoxy, Phenylaminocarbonyloxy-C₁₋₆-alkyl, Phenyl-hydroxy-C₁₋₆-alkyl, Phenyl-oxadiazolyl-C₁₋₆-alkoxy, Phenyl-oxadiazolyl-C₁₋₆-alkyl, Phenyl-oxazolyl-C₁₋₆-alkoxy, Phenyloxy-C₁₋₆-alkoxy, Phenylsulfamoyl-C₁₋₆-alkyl, Phenylsulfinyl-C₁₋₆-alkyl, Phenylsulfonyl-C₁₋₆-alkoxy, Phenylsulfonyl-C₁₋₆-alkyl, Phenyltetrazolyl-thio-C₁₋₆-alkyl, Phenylthio-C₁₋₆-alkoxy, Phenylthio-C₁₋₆₋alkyl, Pyrazinylcarbonyloxy-C₁₋₆-alkyl, Pyridylaminocarbonyloxy-C₁₋₆-alkoxy, Pyridylaminocarbonyloxy-C₁₋₆-alkyl, Pyridylcarbamoyloxy, Pyridyl-C₁₋₆-alkoxy-C₁₋₆-alkoxy, Pyridyl-C₁₋₆-alkoxy-C₁₋₆-alkyl, Pyridyl-oxadiazolyl-C₁₋₆-alkoxy, Pyridylthio-C₁₋₆-alkyl, Pyrimidinyloxy-C₁₋₆-alkoxy, Pyrimidinylthio-C₁₋₆-alkyl, Thienoyloxy-C₁₋₆-alkoxy, Thienoyloxy-C₁₋₆-alkyl, Thienyl-oxadiazolyl-C₁₋₆-alkoxy, Triazolyl-C₁₋₆-alkoxy, Trifluormethylbenzyloxy-C₁₋₆-alkoxy, oder Trifluormethyl substituiertes Phenyl.

Beispiele besonders bevorzugter Reste R¹, insbesondere in Verbindungen der Gruppen (D), (E), (F) oder (G), sind Acetamido-phenyl, C₁₋₆-Alkoxy-phenyl, C₁₋₆-Alkylamino-phenyl, C₁₋₆₋Alkyl-phenyl, 1 H-Benzoimidazolyl, Carbamoyl-phenyl, Di-C₁₋₆-alkylamino-phenyl, 3,4-Dihydro-1 H-chinolin-2-onyl, 2-Furanyl-phenyl, Halo-phenyl, 2-Methyl-pyrimidinyl, Morpholinyl-phenyl, Phenyl, Pyrrolidinonyl-phenyl und Pyrrolidinyl-phenyl, welches durch 1-3 Wasserstoff, C₁₋₆-Alkoxy-C₁₋₆-alkoxy, C₁₋₆-Alkoxy-C₁₋₆-alkyl, N-Acetyl-C₁₋₆-alkoxy-C₁₋₆₋alkyl-amino, C₁₋₆-Alkanoylamido-C₁₋₆-alkyl, N-C₁₋₆-Alkyl-C₁₋₆-alkanoylamido-C₁₋₆-alkyl, C₁₋₆₋Alkoxy-C₁₋₆-alkoxy-C₁₋₆-alkyl, Triazol-1-yl-C₁₋₆-alkyl, Tetrazol-1-yl-C₁₋₆-alkyl, Tetrazol-2-yl-C₁₋₆₋alkyl, Tetrazol-5-yl-C₁₋₆-alkyl, C₁₋₆-Alkoxy-carboxyl-C₁₋₆-alkyl, Pyrrolidinonyl-C₁₋₆-alkyl, Imidazolyl-C₁₋₆-alkyl, Cyano-C₁₋₆-alkyl, Carboxy-C₁₋₆-alkyl, Carboxy-C₁₋₆-alkoxy, C₁₋₆₋Alkoxycarbonyl-C₀₋₆-alkyl, C₁₋₆-Alkylsulfonamidyl-C₁₋₆-alkyl, C₁₋₆-Alkoxy-C₁₋₆-alkanoylamido, C₁₋₆-Alkoxy-C₁₋₆-alkanoylamido-C₁₋₆-alkyl, N-(C₁₋₆-Alkyl)-C₁₋₆-alkoxy-C₁₋₆-alkanoylamido, N-C₁₋₆-Alkyl-carbamoyl-C₁₋₆-alkyl, C₃₋₈-Cycloalkanoylamido-C₁₋₆-alkyl, C₁₋₆-Alkylamino-carbonylamino-C₁₋₆-alkyl, C₁₋₆-Alkanoylamidomethyl-pyrrolidinyl, N-(C₁₋₆-Alkoxy-C₁₋₆-alkyl)-carbamoyl, N-(C₁₋₆-Alkoxy-C₁₋₆-alkyl)-N-(C₁₋₆-alkyl)-carbamoyl, N-(C₁₋₆-Alkoxy-C₁₋₆-alkyl)-imidazol-2-yl, Hydroxy-C₁₋₆-alky), Hydroxy-C₁₋₆-alkoxy, Hydroxy-C₁₋₆-alkoxy-C₁₋₆-alkyl, C₁₋₆-Alkoxycarbonylamido-C₁₋₆-alkyl, Amino-C₁₋₆-alkyl oder C₁₋₆-Alkylamino-C₁₋₆-alkyl substituiert ist.

Beispiele besonders bevorzugter Reste R¹, insbesondere in Verbindungen der Gruppen (A), (D), (E), (F) oder (G), sind 3-C₁₋₆-Alkyl-indolyl, Benzofuranyl, 4H-Benzo[1,4]oxazin-3-onyl, 3,4-Dihydro-2H-benzo[1,4]oxazinyl, 3,4-Dihydro-2H-benzo[1,4]thiazinyl, 3,3-Di-C₁₋₆-alkyl-1,3-dihydro-indol-2-onyl, 3,3-Di-C₁₋₆-alkyl-1,3-dihydro-indolyl, Indolyl, 3-Methyl-indolyl und Spiro[cyclopropan-1,3']-2,3-dihydro-1 H-indolyl, welches wie oben angegeben substituiert sein kann, insbesondere durch mindestens einen Substituenten ausgewählt aus C₁₋₆-Alkoxy-C₁₋₆-alkoxy, C₁₋₆-Alkoxy-C₁₋₆-alkyl, N-Acetyl-C₁₋₆-alkoxy-C₁₋₆-alkyl-amino, C₁₋₆-Alkanoylamido-C₁₋₆-alkyl, N-C₁₋₆-Alkyl-C₁₋₆-alkanoylamido-C₁₋₆-alkyl, C₁₋₆-Alkoxy-C₁₋₆-alkoxy-C₁₋₆-alkyl, Triazol-1-yl-C₁₋₆-alkyl, Tetrazol-1-yl-C₁₋₆-alkyl, Tetrazol-2-yl-C₁₋₆-alkyl, Tetrazol-5-yl-C₁₋₆-alkyl, C₁₋₆-Alkoxy-carboxyl-C₁₋₆-alkyl, Pyrrolidinonyl-C₁₋₆-alkyl, Imidazolyl-C₁₋₆-alkyl, Cyano-C₁₋₆₋alkyl, Carboxy-C₁₋₆-alkyl, Carboxy-C₁₋₆-alkoxy, C₁₋₆-Alkoxycarbonyl-C₁₋₆-alkyl, C₁₋₆-Alkylsulfonamidyl-C₁₋₆-alkyl, C₁₋₆-Alkoxy-C₁₋₆-alkanoylamido, C₁₋₆-Alkoxy-C₁₋₆₋alkanoylamido-C₁₋₆₋alkyl, N-(C₁₋₆-Alkyl)-C₁₋₆-alkoxy-C₁₋₆-alkanoylamido, N-C₁₋₆-Alkyl-carbamoyl-C₁₋₆-alkyl, C₃₋₈₋Cycloalkanoylamido-C₁₋₆-alkyl, C₁₋₆-Alkylamino-carbonylamino-C₁₋₆-alkyl, C₁₋₆-Alkanoylamidomethyl-pyrrolidinyl, N-(C₁₋₆-Alkoxy-C₁₋₆-alkyl)-carbamoyl, N-(C₁₋₆-Alkoxy-C₁₋₆-alkyl)-N-(C₁₋₆-alkyl)-carbamoyl, N-(C₁₋₆-Alkoxy-C₁₋₆-alkyl)-imidazol-2-yl, Hydroxy-C₁₋₆-alkyl, Hydroxy-C₁₋₆-alkoxy, Hydroxy-C₁₋₆-alkoxy-C₁₋₆-alkyl, C₁₋₆-Alkoxycarbonylamido-C₁₋₆-alkyl, Amino-C₁₋₆₋alkyl und C₁₋₆-Alkylamino-C₁₋₆-alkyl.

Beispiele ganz besonders bevorzugter Reste R¹, insbesondere in Verbindungen der Gruppen (A), (D), (E), (F) oder (G), sind 4H-Benzo[1,4]oxazin-3-on-6-yl, 3,4-Dihydro-2H-benzo[1,4]oxazin-6-yl, 3,4-Dihydro-2H-benzo[1,4]thiazin-6-yl, 2,2-Dimethyl-3,4-dihydro-2H-benzo[1,4]oxazin-6-yl, 2,2-Dimethyl- 4H-benzo[1,4]oxazin-3-on-6-yl, 4,4-Dimethyl-1,4-dihydro-benzo[d][1,3]oxazin-2-on-8-yl, 3,3-Dimethyl-1,3-dihydro-indol-2-on-6-yl, 3,3-Dimethyl-1,3-dihydro-indol-2-on-7-yl, 3,3-Dimethyl-1,3-dihydro-indol-6-yl, 3-Methyl-indol-6-yl und Spiro[cyclopropan-1,3']-2,3-dihydro-1 H-indol-6-yl, welches wie oben angegeben substituiert sein kann, insbesondere durch mindestens einen Substituenten ausgewählt aus C₁₋₆-Alkoxy-C₁₋₆-alkoxy, C₁₋₆-Alkoxy-C₁₋₆-alkyl, N-Acetyl-C₁₋₆-alkoxy-C₁₋₆-alkyl-amino, C₁₋₆₋Alkylcarbonylamino-C₁₋₆-alkyl, C₁₋₆-Alkylcarbonylamino-C₁₋₆-alkoxy, N-C₁₋₆-Alkyl-C₁₋₆₋alkanoylamido-C₁₋₆-alkyl, C₁₋₆-Alkoxy-C₁₋₆-alkoxy-C₁₋₆-alkyl, Triazol-1-yl-C₁₋₆-alkyl, Tetrazol-1-yl-C₁₋₆-alkyl, Tetrazol-2-yl-C₁₋₆-alkyl, Tetrazol-5-yl-C₁₋₆-alkyl, C₁₋₆-Alkoxy-carboxyl-C₁₋₆-alkyl, Pyrrolidinonyl-C₁₋₆-alkyl, Imidazolyl-C₁₋₆-alkyl, Cyano-C₁₋₆-alkyl, Carboxy-C₁₋₆-alkyl, Carboxy-C₁₋₆-alkoxy, C₁₋₆-Alkoxycarbonyl-C₁₋₆-alkyl, C₁₋₆-Alkylsulfonamidyl-C₁₋₆-alkyl, C₁₋₆-Alkoxy-C₁₋₆₋alkanoylamido, C₁₋₆-Alkoxy-C₁₋₆-alkanoylamido-C₁₋₆-alkyl, N-(C₁₋₆-Alkyl)-C₁₋₆-aIkOXy-C₁₋₆₋alkanoylamido, N-C₁₋₆-Alkyl-carbamoyl-C₁₋₆-alkyl, C₃₋₈-Cycloalkanoylamido-C₁₋₆-alkyl, C₁₋₆₋Alkylamino-carbonylamino-C₁₋₆-alkyl, C₁₋₆-Alkanoylamidomethyl-pyrrolidinyl, N-(C₁₋₆-Alkoxy-C₁₋₆-alkyl)-carbamoyl, N-(C₁₋₆-Alkoxy-C₁₋₆-alkyl)-N-(C₁₋₆-alkyl)-carbamoyl, N-(C₁₋₆-Alkoxy-C₁₋₆₋alkyl)-imidazol-2-yl, Hydroxy-C₁₋₆-alkyl, Hydroxy-C₁₋₆-alkoxy, Hydroxy-C₁₋₆-alkoxy-C₁₋₆-alkyl, C₁₋₆-Alkoxycarbonylamido-C₁₋₆-alkyl, Amino-C₁₋₆-alkyl und C₁₋₆-Alkylamino-C₁₋₆-alkyl.

Weitere Beispiele ganz besonders bevorzugter Reste R¹, insbesondere in Verbindungen der Gruppen (B), (C), (D), (E), (F) oder (G), sind 3,4-Dihydro-1 H-chinolin-2-on-7-yl, 4,4-Dimethyl-3,4-dihydro-1 H-chinolin-2-on-8-yl und Phenyl, welches wie weiter oben angegeben substituiert ist, insbesondere durch mindestens einen Substituenten ausgewählt aus N-Acetyl-C₁₋₆-alkoxy-C₁₋₆-alkyl-amino, C₁₋₆-Alkylcarbonylamino-C₁₋₆-alkyl, C₁₋₆-Alkylcarbonylamino-C₁₋₆₋alkoxy, N-C₁₋₆-Alkyl-C₁₋₆-alkanoylamido-C₁₋₆-alkyl, C₁₋₆-Alkoxy-C₁₋₆-alkoxy-C₁₋₆-alkyl, Triazol-1-yl-C₁₋₆-alkyl, Tetrazol-1-yl-C₁₋₆-alkyl, Tetrazol-2-yl-C₁₋₆-alkyl, Tetrazol-5-yl-C₁₋₆-alkyl, C₁₋₆₋Alkoxy-carboxyl-C₁₋₆-alkyl, Pyrrolidinonyl-C₁₋₆-alkyl, Imidazolyl-C₁₋₆-alkyl, Cyano-C₁₋₆-alkyl, Carboxy-C₁₋₆-alkyl, Carboxy-C₁₋₆-alkoxy, C₁₋₆-Alkoxycarbonyl-C₁₋₆-alkyl, C₁₋₆-Alkylsulfonamidyl-C₁₋₆-alkyl, C₁₋₆-Alkoxy-C₁₋₆-alkanoylamido, C₁₋₆-Alkoxy-C₁₋₆-alkanoylamido-C₁₋₆-alkyl, N-(C₁₋₆-Alkyl)-C₁₋₆-alkoxy-C₁₋₆-alkanoylamido, N-C₁₋₆-Alkyl-carbamoyl-C₁₋₆-alkyl, C₃₋₈-Cycloalkanoylamido-C₁₋₆-alkyl, C₁₋₆-Alkylamino-carbonylamino-C₁₋₆-alkyl, C₁₋₆-Alkanoylamidomethyl-pyrrolidinyl, N-(C₁₋₆-Alkoxy-C₁₋₆-alkyl)-carbamoyl, N-(C₁₋₆-Alkoxy-C₁₋₆-alkyl)-N-(C₁₋₆₋alkyl)-carbamoyl, N-(C₁₋₆-Alkoxy-C₁₋₆-alkyl)-imidazol-2-yl, Hydroxy-C₁₋₆-alkyl, Hydroxy-C₁₋₆₋alkoxy-C₁₋₆-alkyl, C₁₋₆-Alkoxycarbonylamido-C₁₋₆-alkyl, Amino-C₁₋₆-alkyl und C₁₋₆-Alkylamino-C₁₋₆-alkyl.

Beispiele besonders bevorzugter Reste R² sind durch (C₁₋₆-Alkanoyl)-amlnophenoxy-C₁₋₆-alkyl, C₁₋₆-Alkoxy, C₁₋₆-Alkoxy-C₁₋₆-alkoxy, C₁₋₆-Alkoxy-C₁₋₆-alkoxy-C₁₋₆-alkyl, C₁₋₆-Alkoxy-C₁₋₆-alkyl, C₁₋₆-Alkoxybenzyl-C₁₋₆-alkoxy, C₁₋₆-Alkoxybenzyloxy-C₁₋₆-alkoxy, C₁₋₆-Alkoxybenzyloxy-C₁₋₆-alkoxy-C₁₋₆-alkyl, C₁₋₆-Alkoxy-methylbenzyloxy-C₁₋₆-alkoxy, C₁₋₆-Alkoxyphenoxy-C₁₋₆-alkoxy, C₁₋₆-Alkoxyphenoxy-C₁-₆-al kyl, C₁₋₆₋Alkoxyphenyl-C₁₋₆-alkoxy-C₁₋₆-alkoxy, N-(C₁₋₆-Alkoxyphenyl-C₁₋₆-alkyl)-amino-C₁₋₆-alkoxy, N-(C₁₋₆-Alkoxyphenyl)-azetidinyloxy, N-(C₁₋₆-Alkoxyphenyl)-pyrrolidinyloxy, C₁₋₆-Alkoxypyrimidinyl-amino-C₁₋₆-alkoxy, C₁₋₆-Alkoxy-pyridyl-amino-C₁₋₆-alkoxy, (N-(C₁₋₆-alkyl)-N-(benzoyl)-amino)-pyrrolidin-1-yl, C₁₋₆-Alkyl-benzyloxy-C₁₋₆-alkoxy, N-(C₁₋₆-Alkyl)-carbamoyl-C₁₋₆-alkyl, (N-(C₁₋₆-Alkyl)-carbamoyl-C₁₋₆-alkyl)-phenoxy-C₁₋₆-alkyl, C₁₋₆-Alkyl-indolyl-C₁₋₆₋alkoxy, N-(C₁₋₆-Alkyl)-indol-4-yloxy-C₁₋₆-alkyl, C₁₋₆-Alkylphenoxy, C₁₋₆-Alkylphenoxy-C₁₋₆₋alkoxy, C₁₋₆-Alkylphenoxy-C₁₋₆-alkyl, N-(C₁₋₆-Alkyl)-N-(phenoxy-C₁₋₆-alkyl)-amino-carbonyl, Azetidin-1-yl, Benzo[1,3]dioxolyloxy-C₁₋₆-alkoxy, N-(Benzoyl)-azetidinyloxy, N-(Benzyl)-azetidinyloxy, Benzyloxy, (Carbamoyl-C₁₋₆-alkyl)-phenoxy-C₁₋₆-alkyl, Cyanophenoxy-C₁₋₆₋alkoxy, N-(C₃₋₈-Cycloalkanoyl)-pyrrolidinyloxy, N-(C₃₋₈-Cycloal kyl-C₁₋₆-alkanoyl)-pyrrolidinyloxy, C₃₋₈-Cycloalkoxy, C₃₋₈-Cycloalkoxy-C₁₋₆-alkoxy, C₃₋₈-Cycloalkyl-C₁₋₆-alkoxy, C₃₋₈-Cycloalkyl-C₁₋₆-alkoxy-C₁₋₆-alkyl, N-(C₃₋₈-Cycloalkyl-C₀₋₆-alkyl)-amino-C₁₋₆-alkoxy, N-(C₃₋₈₋Cycloalkyl)-pyrrolidinyloxy, N,N-(Di-C₁₋₆-alkyl)-amino-C₁₋₆-alkoxy, 3,4-Dihydro-chinolin-2-on-5-yloxy-C₁₋₆-alkyl, 3,4-Dihydro-1H-isochinolin-2-yl-C₁₋₆-alkoxy, Halobenzoylamido-C₁₋₆-alkoxy, N-(Halobenzyl)-amino-C₁₋₆-alkoxy, Halobenzyloxy-C₁₋₆-alkoxy, Halophenoxy, Halophenoxy-C₁₋₆-alkoxy, Halophenoxy-C₁₋₆-alkoxy-C₁₋₆-alkyl, Halophenoxy-C₁₋₆-alkyl, Halophenoxy-C₁₋₆₋alkylamino, N-(Halophenoxy-C₁₋₆-alkyl)-amino-C₁₋₆-alkyl, Halophenyl-C₁₋₆-alkoxy-C₁₋₆-alkoxy, N-(Halophenyl-C₁₋₆-alkyl)-amino-C₁₋₆-alkoxy, N-(Halophenyl)-amino-C₁₋₆-alkoxy, N-(Halophenyl)-azetidinyloxy, N-(Halophenyl)-pyrrolidinyloxy, N-(Heteroaryl)-azetidinyloxy, N-(Heteroaryl)-pyrrolidinyloxy, Hydroxy-C₁₋₆-alkoxy, (Hydroxy)-phenoxy-C₁₋₆-alkoxy, Imidazol-1-y)-C₁₋₆-alky), Imidazol-4-yl-phenoxy-C₁₋₆-alkyl, Indol-4-yloxy-C₁₋₆-alkyl, Phenoxy, Phenoxy-C₁₋₆-alkyl, Phenoxy-C₁₋₆-alkoxy, Phenoxy-(C₁₋₆-alkoxy)-C₁₋₆-alkoxy, Phenooy-(C₁₋₆-alkyl)-C₁₋₆₋alkoxy, N-(Phenoxy-C₁₋₆-alkyl)-amino-carbonyl, (Phenoxy)-azetidin-1-yl-carbonyl, Phenoxy-(halo)-C₁₋₆-alkoxy, Phenooy-(hydrooy)-C₁₋₆-alkooy, (Phenooy)-pyrrolidin-1-yl-C₁₋₆-alkyl, (Phenooy)-pyrrolidin-1-yl-carbonyl, Phenyl-(amino)-C₁₋₆-alkoxy, N-(Phenyl)-C₁₋₆-alkylpyrrolidinyloxy, Phenyl-azetidinyl-C₁₋₆-alkyl, Phenyl-azetidinyloxy, N-(Phenyl)-azetidinyloxy-C₁₋₆-alkyl, Phenyl-(C₃₋₈-cycloalkyl)-C₁₋₆-alkoxy-C₁₋₆-alkoxy, N4-(Pheny))-piperazin-1-yl-C₁₋₆₋alkyl, N4-(Phenyl)-piperazin-1-yl-carbonyl, Phenyl-piperidinyloxy, Phenyl-pyrrolidin-1-yl-C₁₋₆₋alkoxy, Phenyl-pyrrolidin-1-yl-C₁₋₆-alkyl, N-(N-Phenyl-pyrrolidinyl)-amino-C₁₋₆-alkyl, (Phenyl)-pyrrolidin-1-yl-carbonyl, (Phenyl)-pyrrolidin-1-yl-carbonyl-C₁₋₆-alkyl, (Phenyl)-pyrrolidin-1-yl-carbonyloxy, N-(Phenyl)-pyrrolidinonyloxy, N-(Phenyl)-pyrrolidinyloxy, N-(Phenyl)-pyrrolidinyloxy-C₁₋₆-alkyl, Piperidinyloxy-C₁₋₆-alkoxy, Pyridyloxy-C₁₋₆-alkoxy, N-(Pyridyl)-pyrrolidinyloxy, Pyrrolidin-1-yl, Tetrahydrofuranyloxy, Tetrahydrofuranyloxy-C₁₋₆-alkyl, N-(Tetrahydropyranyl-carbonyl)-piperidinyloxy, Tetrahydropyranyloxy, Tetrahydropyranyloxy-C₁₋₆-alkoxy, Tetrahydropyranyloxy-C₁₋₆-alkyl oder Trifluormethyl-phenoxy-C₁₋₆-alkoxy-substituiertes Phenyl bzw. Halophenyl.

Beispiele ganz besonders bevorzugter Reste R² sind durch C₁₋₆-Alkoxybenzyloxy-C₁₋₆-alkoxy, C₁₋₆-Alkoxyphenyl-C₁₋₆-alkoxy-C₁₋₆-alkoxy, C₁₋₆-Alkylphenoxy-C₁₋₆-alkoxy, Halobenzyloxy-C₁₋₆₋alkoxy, Halophenoxy-C₁₋₆-alkoxy, Halophenoxy-C₁₋₆-alkoxy-C₁₋₆-alkyl, N-(Halophenyl)-pyrrolidin-3-yloxy und Indol-4-yloxy-C₁₋₆-alkyl substituiertes Phenyl bzw. Halophenyl.

Beispiele bevorzugter Reste X sind Sauerstoff, -O-CH₂-CO-NH-, -O-CH₂-CO-N(CH₃)- und -O-CH(CH₃)-CO-NH-.

Besonders bevorzugt für X ist Sauerstoff.

Beispiele bevorzugter Reste Z sind Methylen, -(CH₂)₂-O- und -CH(CH₃)-

Ganz besonders bevorzugt sind die Verbindungen der Beispielsnummern 2, 5, 13, 14, 58, 59, 60, 64, 76, 79, 100, 108, 109, 110, 111, 112, 113, 115, 119, 121, 140, 141, 142, 143, 180, 182, 185, 187, 194, 196, 245, 270, 273, 278 und 346.

Die Verbindungen der Formel (I) oder (II) können in analoger Weise zu aus der Literatur bekannten Herstellungsverfahren hergestellt werden. Ähnliche Herstellungsverfahren werden beispielsweise in WO 97/09311 beschrieben. Einzelheiten zu den spezifischen Herstellungsvarianten können den Beispielen entnommen werden.

Die Verbindungen der Formel (I) oder (II) können auch in optisch reiner Form dargestellt werden. Die Trennung in Antipoden kann nach an sich bekannten Methoden erfolgen entweder vorzugsweise auf einer synthetisch frühen Stufe durch Salzbildung mit einer optisch aktiven Säure wie zum Beispiel (+)- oder (-)-Mandelsäure und Trennung der diasteromeren Salze durch fraktionierte Kristallisation oder vorzugsweise auf einer eher späten Stufe durch Derivatisierung mit einem chiralen Hilfsbaustein, wie zum Beispiel (+)- oder (-)-Camphansäurechlorid, und Trennung der diastereomeren Produkte durch Chromatographie und/oder Kristallisation und anschließende Spaltung der Bindung zum chiralen Hilfsstoff. Die reinen diastereomeren Salze und Derivate können zur Bestimmung des absoluten Konfiguration des enthaltenen Piperidines mit gängigen spektroskopischen Methoden analysiert werden, wobei die X-Ray Spektroskopie an Einkristallen eine besonders geeignete Methode darstellt.

Prodrug Derivate der vorliegend beschriebenen Verbindungen sind Derivate derselben, die bei *in vivo* Anwendung die ursprüngliche Verbindung durch einen chemischen oder physiologischen Prozess freisetzen. Ein Prodrug kann beispielsweise bei Erreichen eines physiologischen pH Wertes oder durch enzymatische Konversion in die ursprüngliche Verbindung umgesetzt werden. Prodrug Derivate können zum Beispiel Ester freiverfügbarer Carbonsäuren, S- und O-Acylderivate von Thiolen, Alkoholen oder Phenolen darstellen, wobei die Acylgruppe wie vorliegend definiert wird. Bevorzugt werden pharmazeutisch verwendbare Esterderivate, die durch Solvolyse in physiologischem Medium in die ursprüngliche Carbonsäure umgesetzt werden, wie beispielsweise niedere Alkylester, Zykloalkylester, niedere Alkenylester, Benzylester, mono- oder disubstituierte niedere Alkylester, wie niedere ω-(Amino, Mono- oder Dialkylamino, Carboxy, niedere Alkoxycarbonyl) - Alkylester oder wie niedere α-(Alkanoyloxy, Alkoxycarbonyl oder Dialkylaminocarbonyl) - Alkylester; als solche werden Pivaloyloxymethylester and ähnliche Ester konventioneller Weise benützt.

Aufgrund der nahen Verwandschaft zwischen einer freien Verbindung, einem Prodrug Derivat und einer Salzverbindung, umfasst eine bestimmte Verbindung in dieser Erfindung auch dessen Prodrug Derivat und Salzform, sofern diese möglich und angebracht ist.

Die Verbindungen der Formel (I) oder (II), bzw. bevorzugt der Formel (IA) oder (IIA), und ihre pharmazeutisch verwendbaren Salze weisen hemmende Wirkung hinsichtlich Beta-Sekretase, Cathepsin D, Plasmepsin II und/oder HIV-Protease auf.

Die Wirkung von Beta-Sekretase-, Cathepsin D-, Plasmepsin II- und/oder HIV-Protease-Hemmern wird unter anderem experimentell mittels in vitro-Tests nachgewiesen.

Der Protease hemmende Vorgang von chemischen Verbindungen kann anhand von Testsystemen, die auf der Fluoreszenz-Resonanz Energietransfer (FRET) Technologie basiert sind und ein rekombinantes, zum Beispiel durch ein Baculovirus exprimiertes Enzympräparat beigeben, ermittelt werden. Die FRET Technologie erlaubt die Messung der proteolytische Teilung von Peptidsubstraten. Das Prinzip der FRET Technologie beruht auf einem messbaren Energiewechsel, der quantitative von dem Vorliegen einer Peptidsequenz abhängig ist. Das Peptidsubstrat für ein bestimmtes Enzym wird mit zwei terminalen Fluorophoren versehen. Das eine Fluorophor stellt den Fluoreszenz-Donor dar und das andere den Fluoreszenz Akzeptor. Die Distanz dieser beiden Gruppen ist so gewählt, das die lichtinduzierte Donorfluoreszenz vom Akzeptor durch FRET gelöscht wird. Bei proteolytischer Substratspaltung, wird das Donorfluorophor vom Akzeptorfluorophor getrennt, wodurch der Fluoreszenzgehalt des Donors nicht gelöscht wird. Auf diese Weise wird ein schwach fluoreszierendes Peptidsubstrat nach enzymischer Spaltung zu einem hoch fluoreszierendem Peptid. Die Steigerung der Fluoreszenz hängt linear von der Proteolyserate ab.

Das FRET Testsystem wird mit weissen Polysorp-Platten durchgeführt. Das Assaymedium besteht aus einer 50 mM Natriumazetat Pufferlösung von pH 5.0, einer Natriumkonzentration von 392 mM, einer Glyceringehalt von 12.5% und einem BSA-Gehalt von 0.1%. Die Inkubate setzen sich aus 160 µl Pufferlösung, 10 µl Inhibitoren in DMSO, 10 µl Peptidsubstrat in DMSO und 20 µl Enzympräparatslösung zusammen. Die Inhibitoren werden in einem Konzentrationsbereich von 0.001 pM zu 10 µM getested. Die zugefügte Peptidsubstratmenge ergibt eine Konzentration im Inkubat von 1 µM. Die fluorophoren Donor und Akzeptor markierten Peptidsubstrate werden durch Solidphasenpeptidsynthese (Applied Biosystems) hergestellt. Das beta-Sekretase Peptidsubstrat Rh-Glu-Val-Asn-Leu-Asp-Ala-Glu-Phe-Lys-Quencher wird von Invitrogen, Carlsbad, CA, USA erhalten. Das Cathepsin D Peptidsubstrat mit der Sequenz DABCYL-Pro-Thr-Glu-Phe-Phe-Arg-Leu-OXL wird von der AnaSpec Inc, San Jose, CA, USA erhalten. Das Plasmepsin Peptidsubstrat mit der Sequenz DABCYL-Glu-Arg-Nle-Phe-Leu-Ser-Phe-Pro-OXL wird von der AnaSpec Inc, San Jose, CA, USA erhalten. Das HIV Protease Peptidsubstrat mit der Sequenz DABCYL-His-Lys-Ala-Arg-Val-Leu-Tyr-Glu-Ala-Nle-Ser-EDANS wird von der AnaSpec Inc, San Jose, CA, USA erhalten. Die rekombinant exprimierten Enzympräparate werden in unterschiedlichen Mengen hinzugegeben. Die Beta-Sekretase Konzentration beträgt 1 unit/ml im Inkubat. Die Cathepsin D Konzentration beträgt 100 ng/ml. Die HIV Protease Konzentration beträgt 500 ng/ml. Die Plasmepsin II Konzbetration im Inkubat ist 50 ng/ml.

Die Reaktionen werden durch Zuführung der Enzymlösung eingeleitet. Der Test wird bei einer Temperatur von 37°C während 30-120 Minuten durchgeführt. Für den Beta-Sekretase Assay beträgt die Inkubationszeit 60 min, für Cathepsin D 120 min, für Plasmepsin II 40 min und für die HIV Protease 40 min. Die Reaktionen werden durch die Zugabe von 20 µl einer 1.0 M Tris Basis Lösung gestoppt. Die enzymatische Substrat zu Produkt Umwandlung wird durch Messung der Fluoreszenz bei 460 nm Wellenlänge gemessen.

### In vitro Enzymhemmungswerte

Für die erfindungsgemässen Verbindungen werden strukturabhängige und enzymabhängige Hemmungsaktivitäten gemessen. Die Enzymhemmungen wurden als IC50 Werte ausgedrückt. Die gemessenen IC50 -Werte zur Inhibierung der Beta-Sekretase belaufen sich zwischen 0.01 pM und 100 nM. Die IC50 -Werte zur Inhibierung von Cathepsin D sind zwischen 0.01 pM und 100 nM. Die IC50-Werte zur Inhibierung von Plasmepsin II liegen zwischen 0.01 pM und 100 nM. Die gemessenen IC50 -Werte zur Inhibierung der HIV-Peptidase liegen zwischen 0.01 pM und 100 nM.

Die Verbindungen der Formel (I) oder (II), bzw. bevorzugt der Formel (IA) oder (IIA), und ihre pharmazeutisch verwendbaren Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, rektal, z.B. in Form von Suppositorien, oder transdermal, z.B. in Form von Salben oder Pflastern, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können die Verbindungen der Formel (I) oder (II), bzw. bevorzugt der Formel (IA) oder (IIA), sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser. Polyole, Sacharose, Invertzucker, Glukose etc.

Für Injektionslösungen eignen sich als Excipientien z.B. Wasser, Alkohole, Polyole, Glycerin, vegetabile Oele, Gallensäuren, Lecithin etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro erwachsene Person (70 kg), verteilt auf vorzugsweise 1-3 Einzeldosen, die z.B. gleich gross sein können, angemessen sein, wobei aber die angegebene obere Grenze auch überschritten werden kann, wenn sich dies als angezeigt erweisen sollte, üblicherweise erhalten Kinder eine ihrem Alter und Körpergewicht entsprechend geringere Dosis.

Die folgenden Beispielverbindungen erläutern die vorliegende Erfindung. Einzelheiten zur Herstellung finden sich in PCT/EP2004/052389.

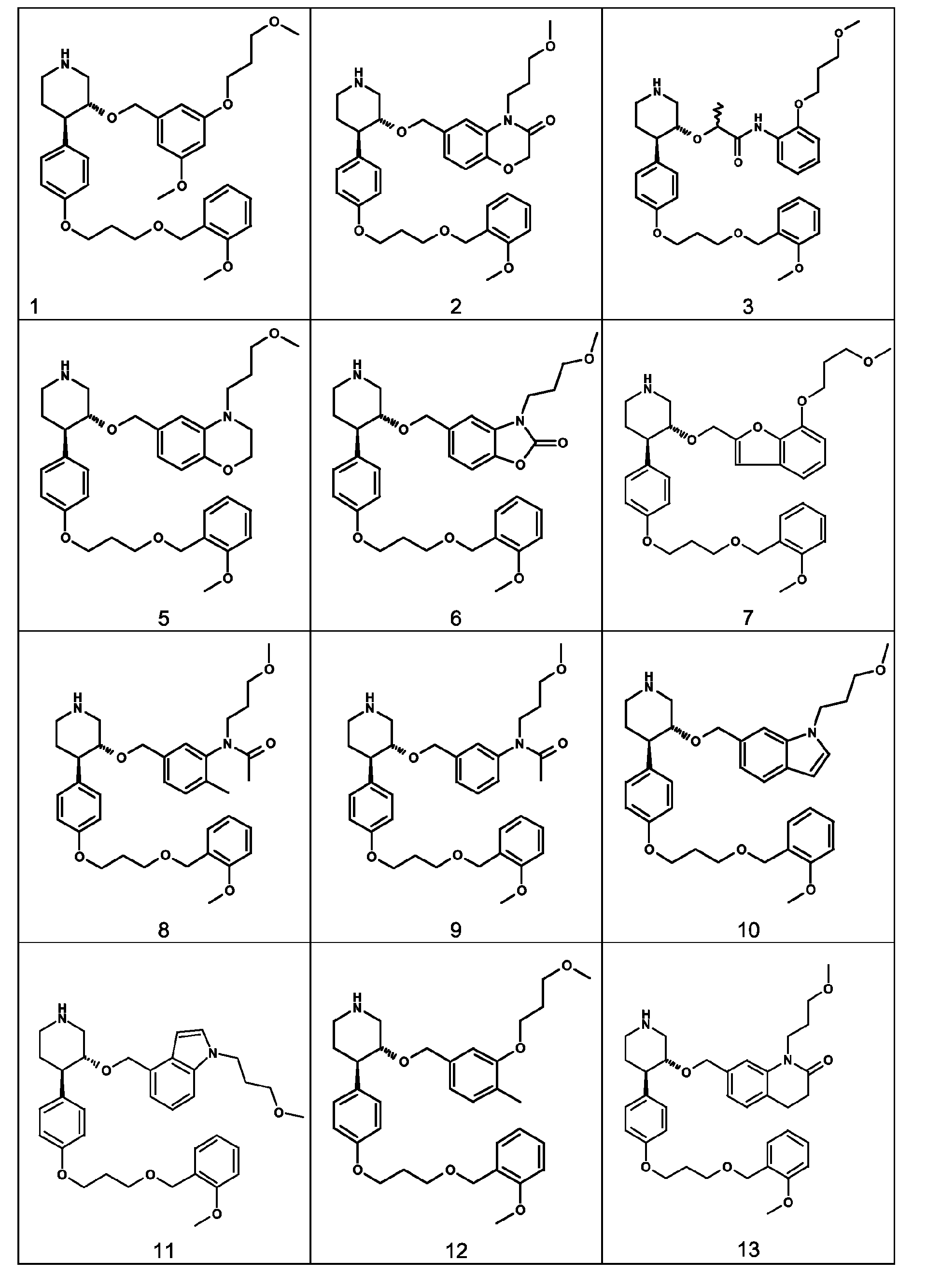

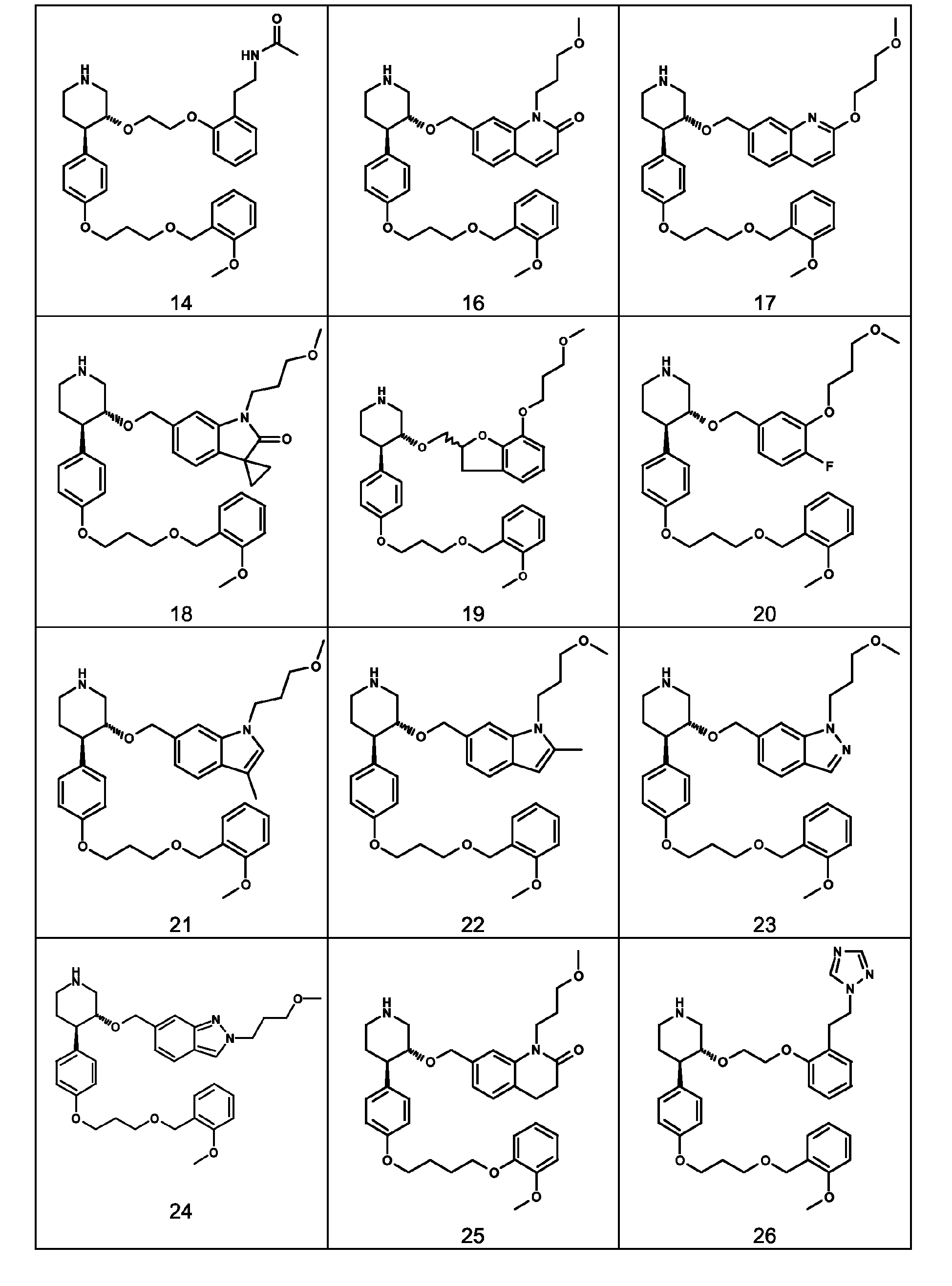

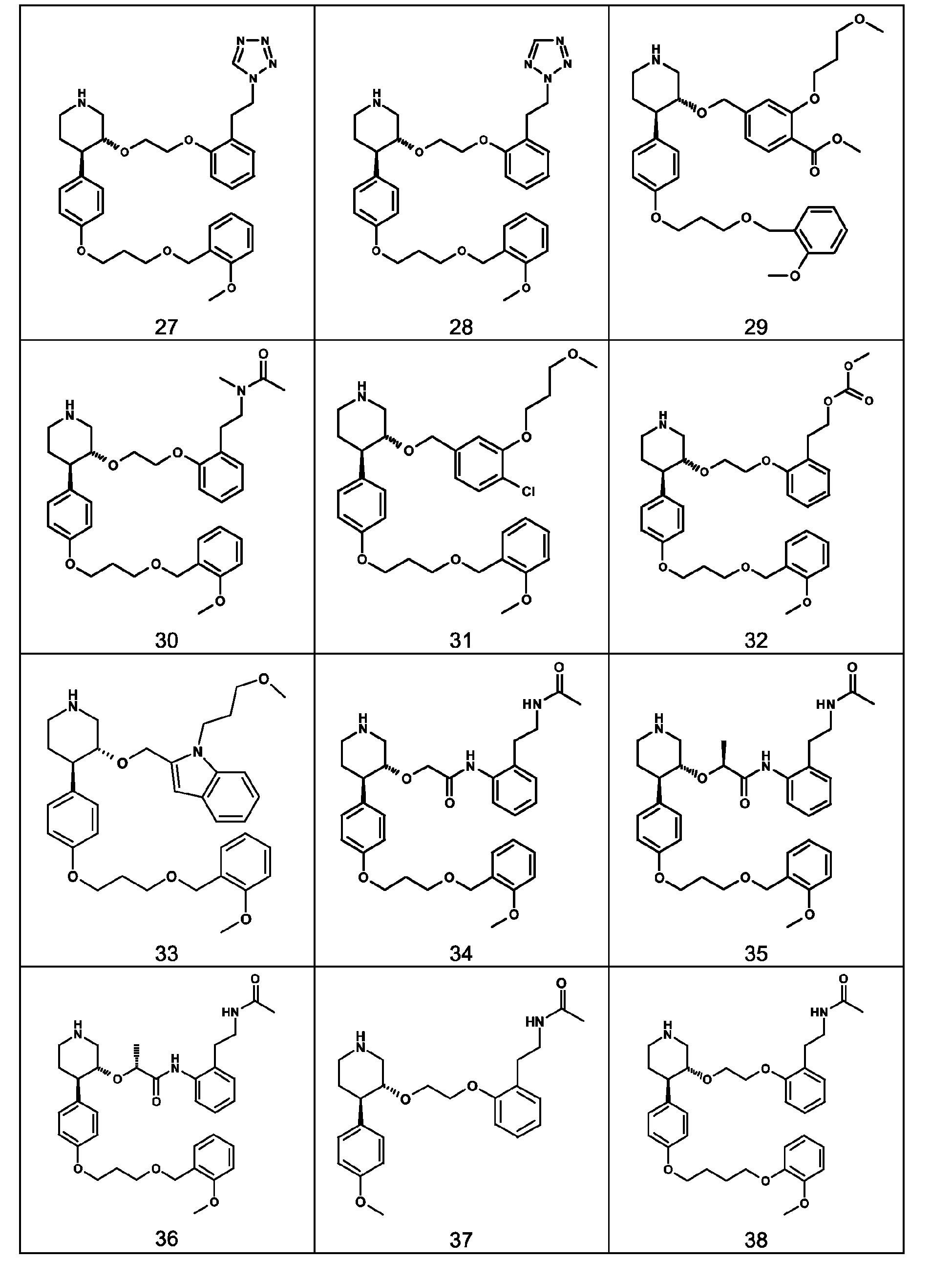

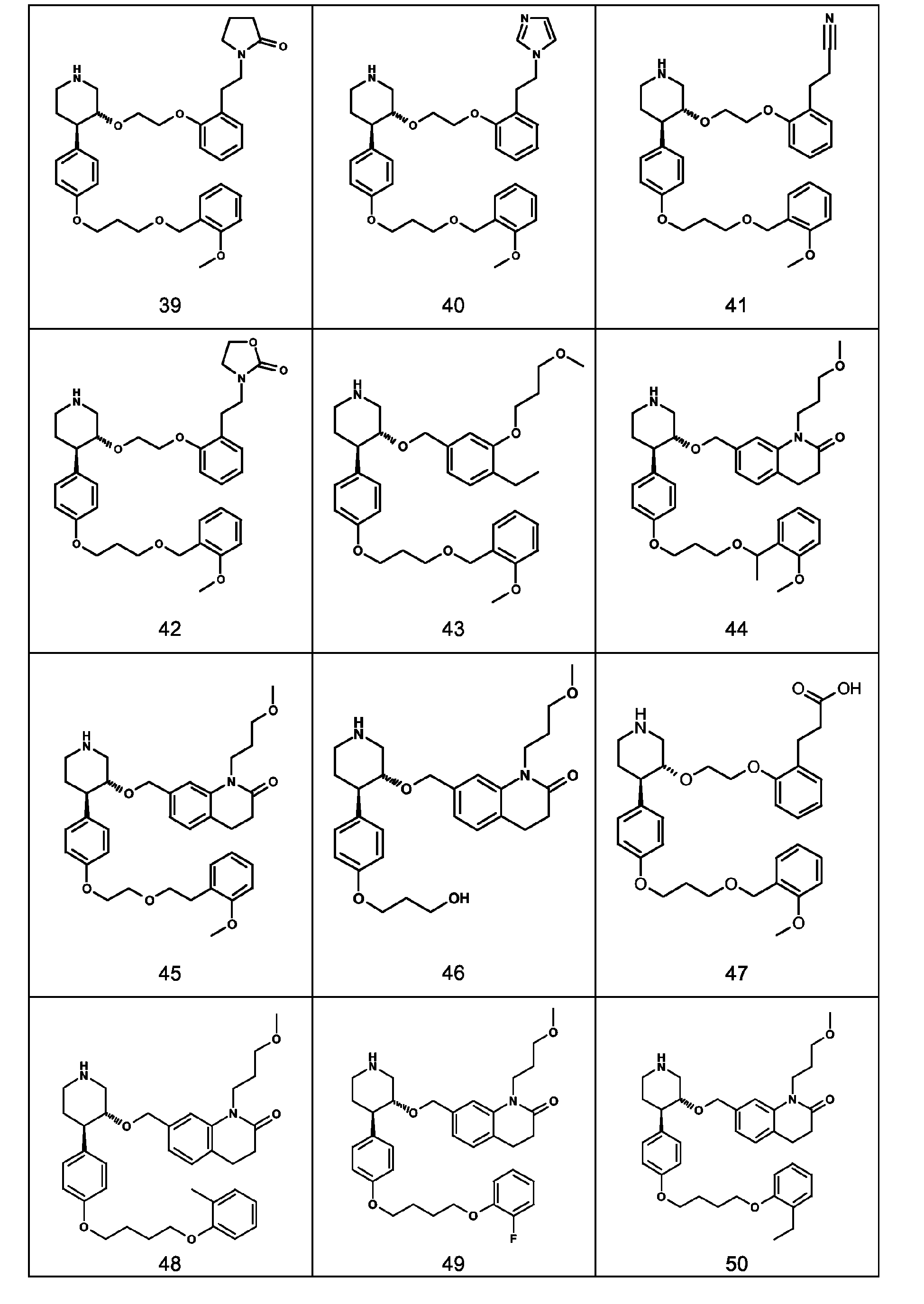

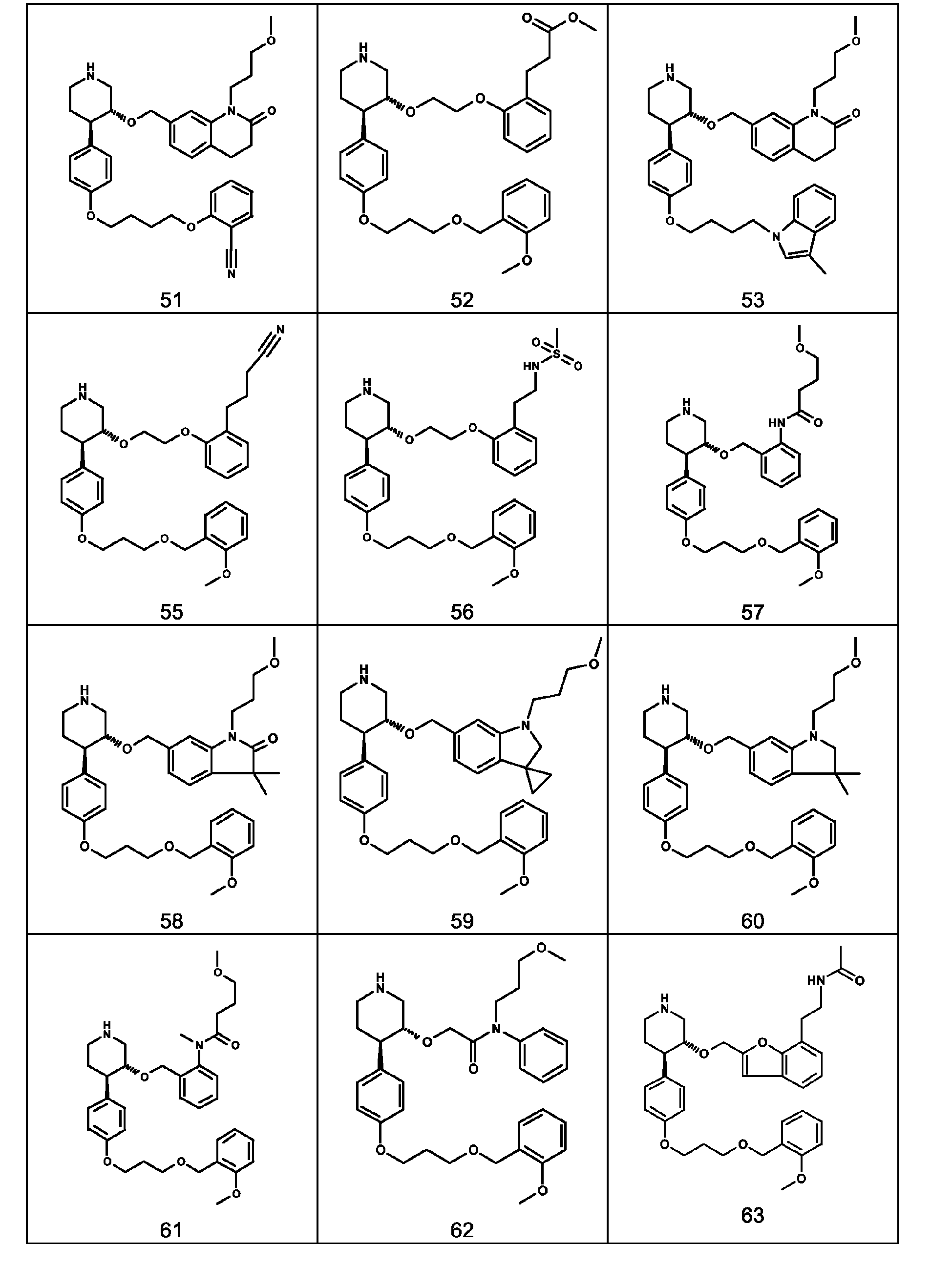

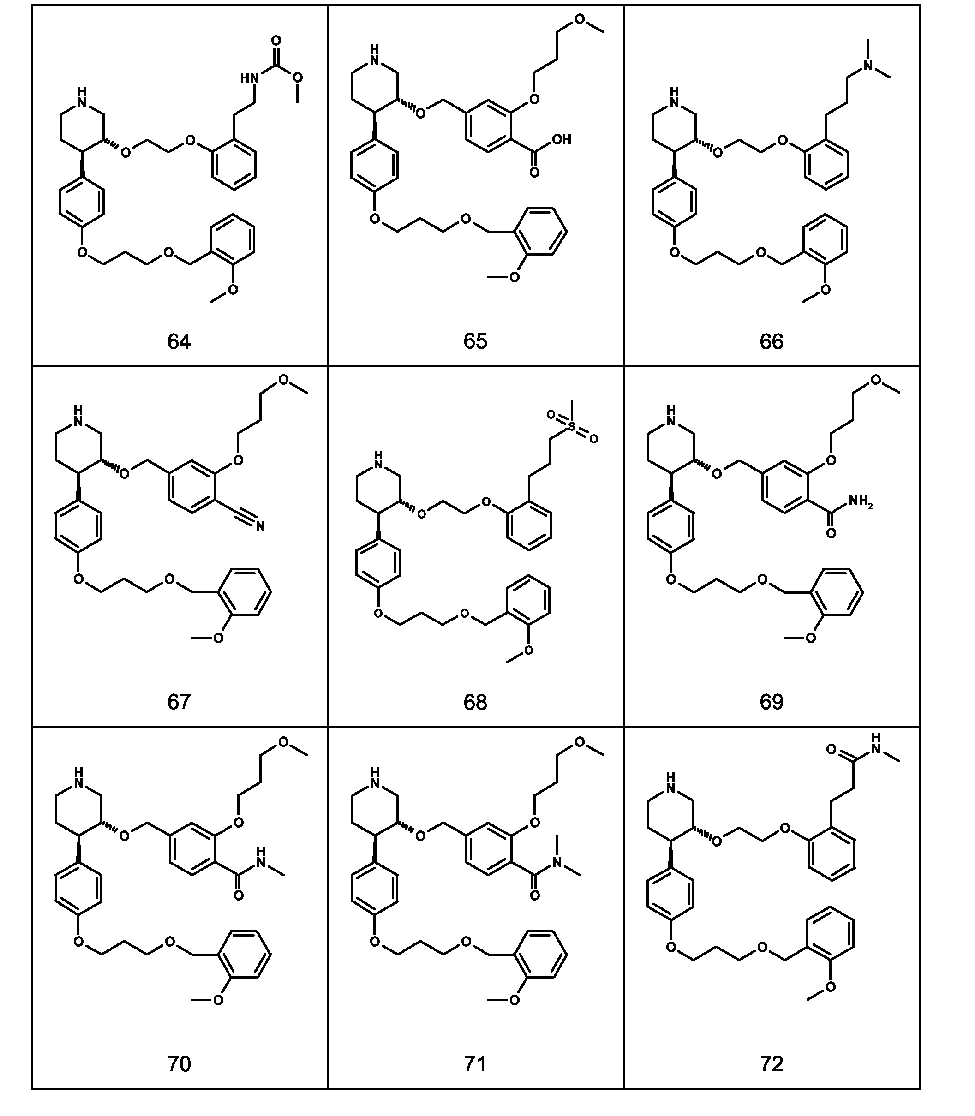

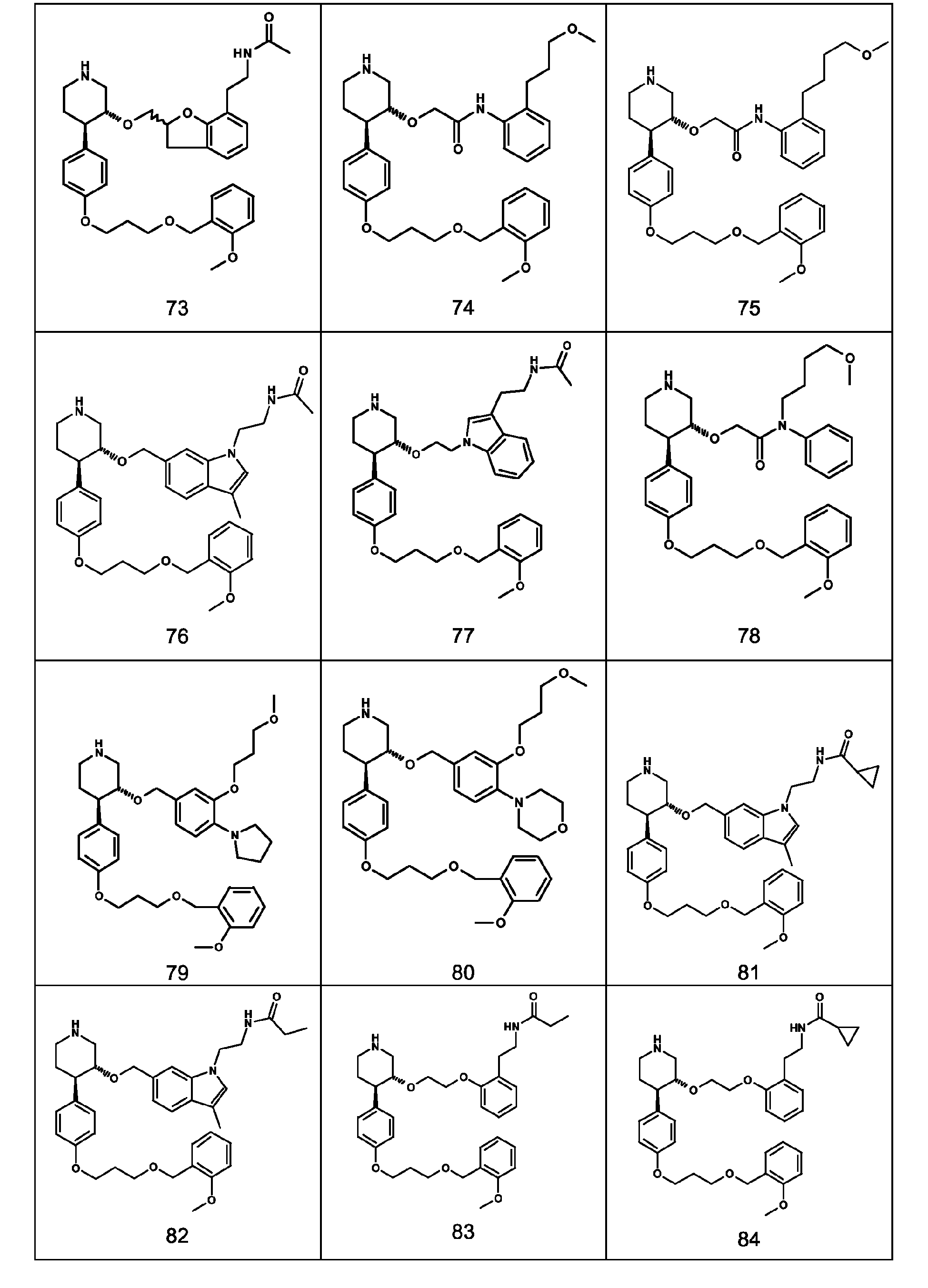

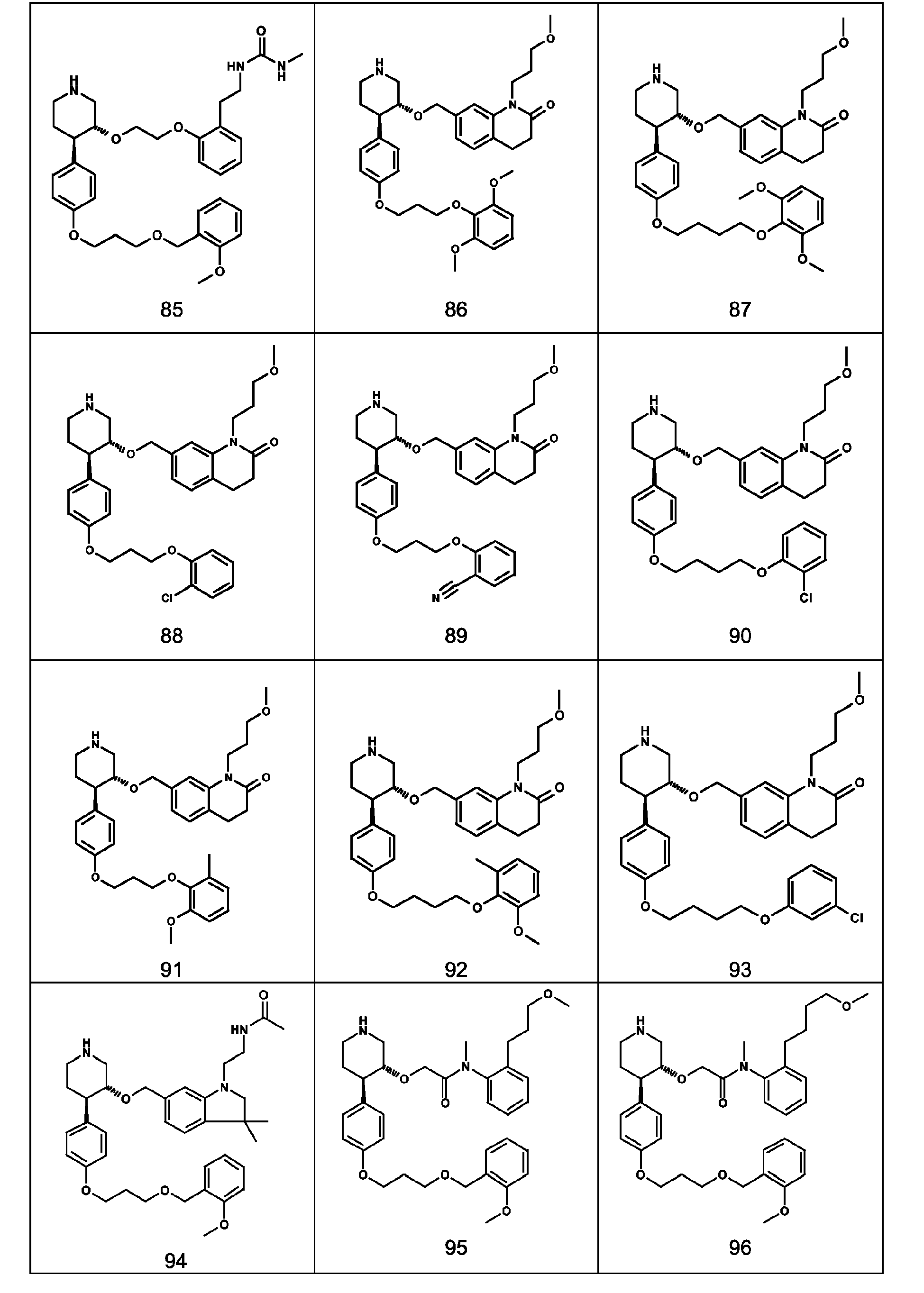

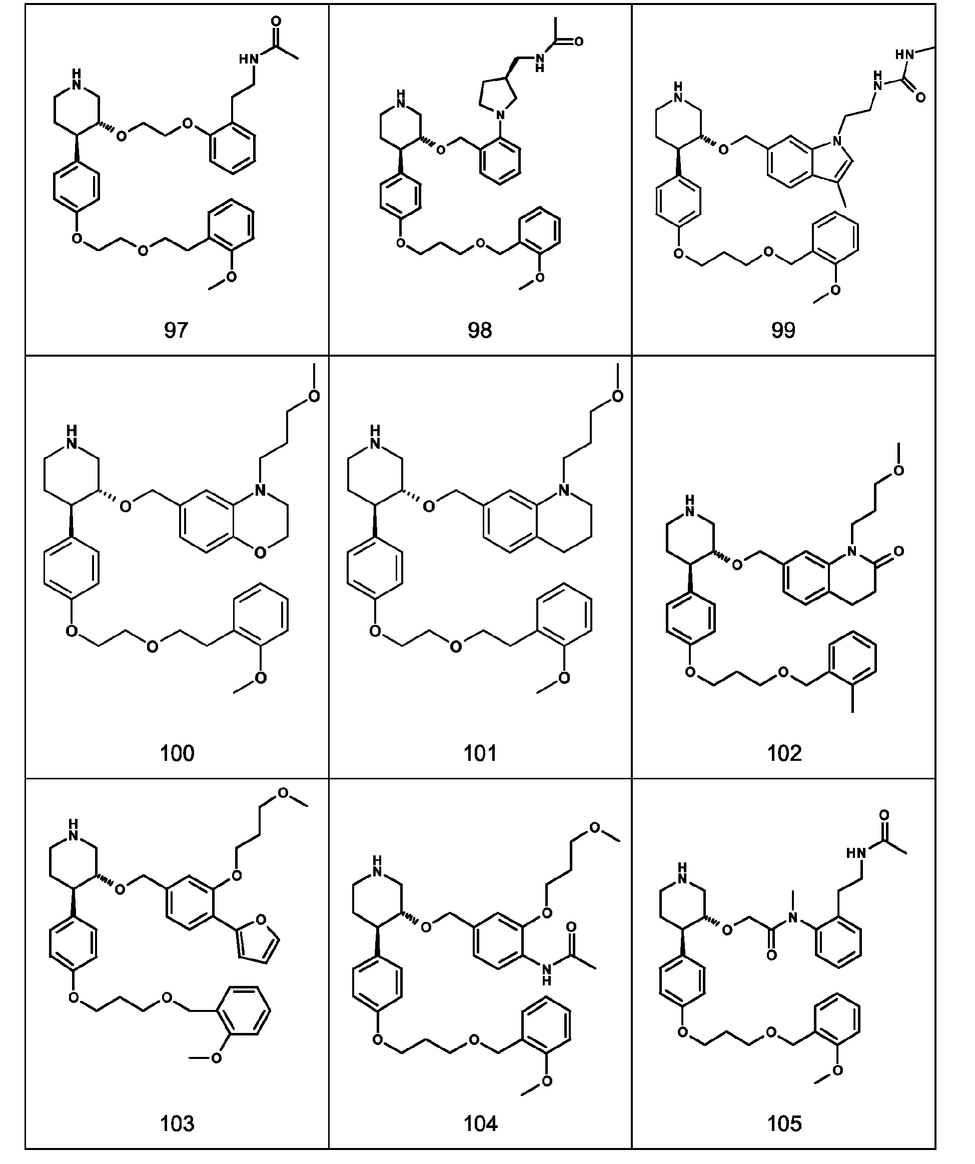

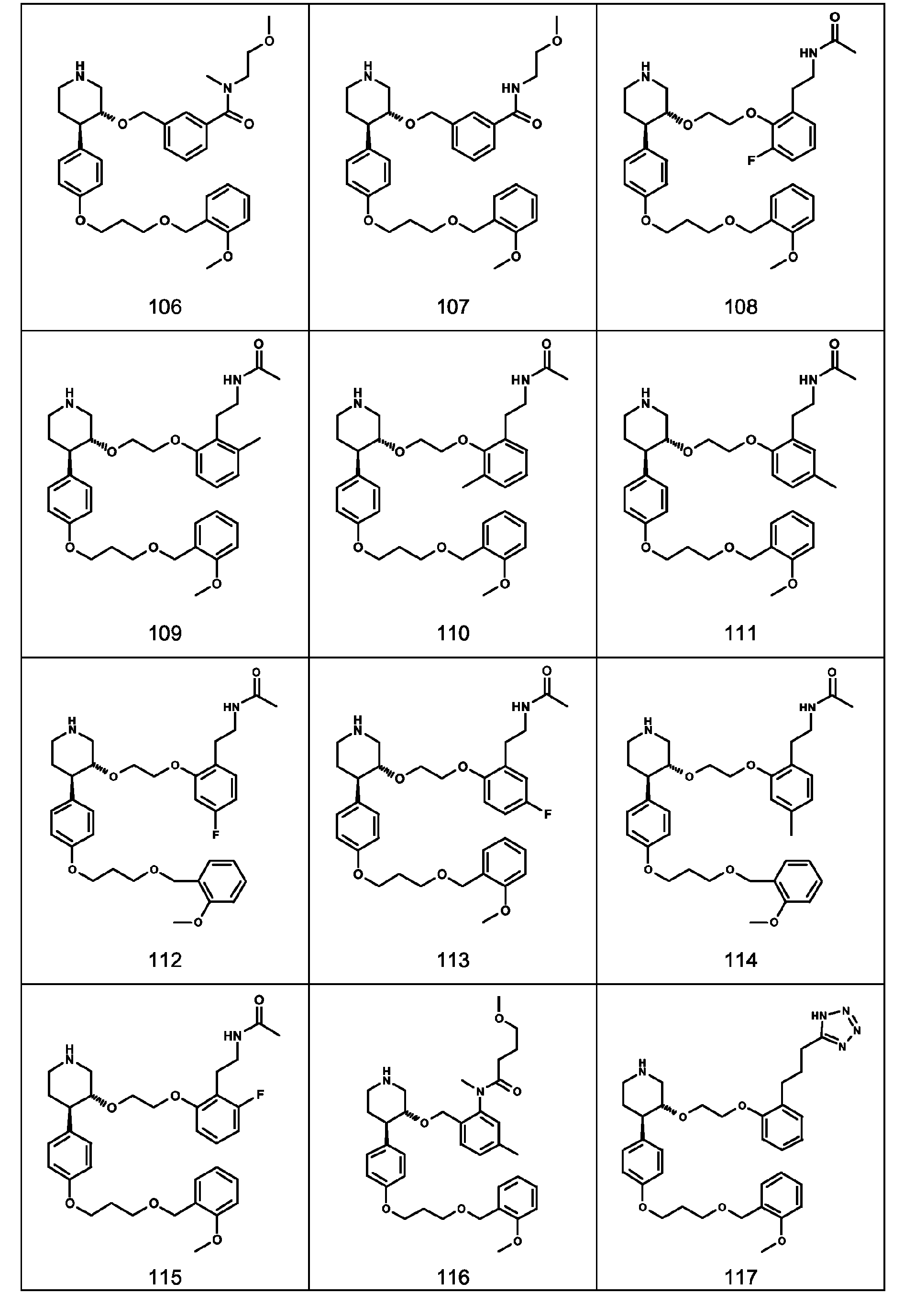

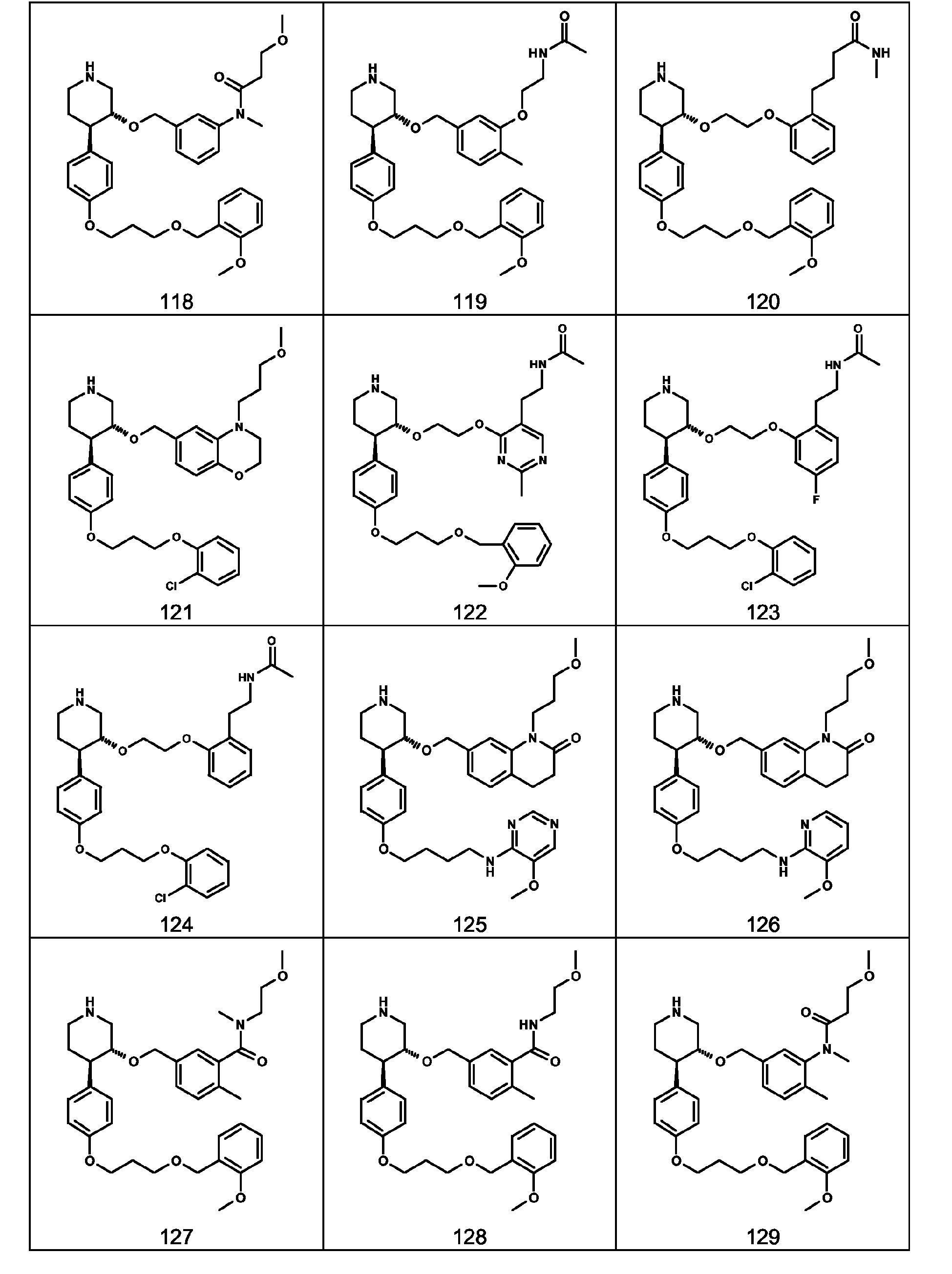

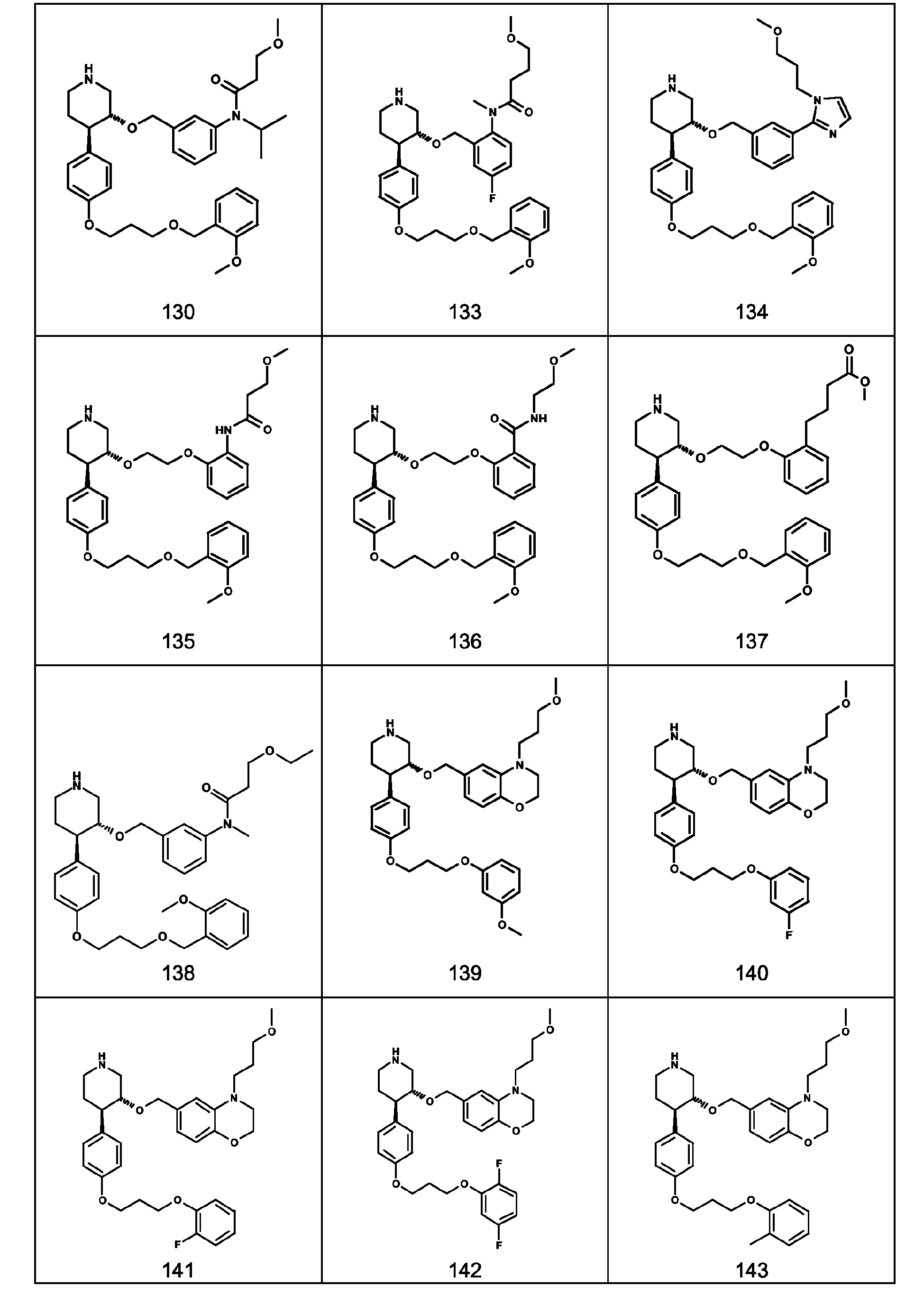

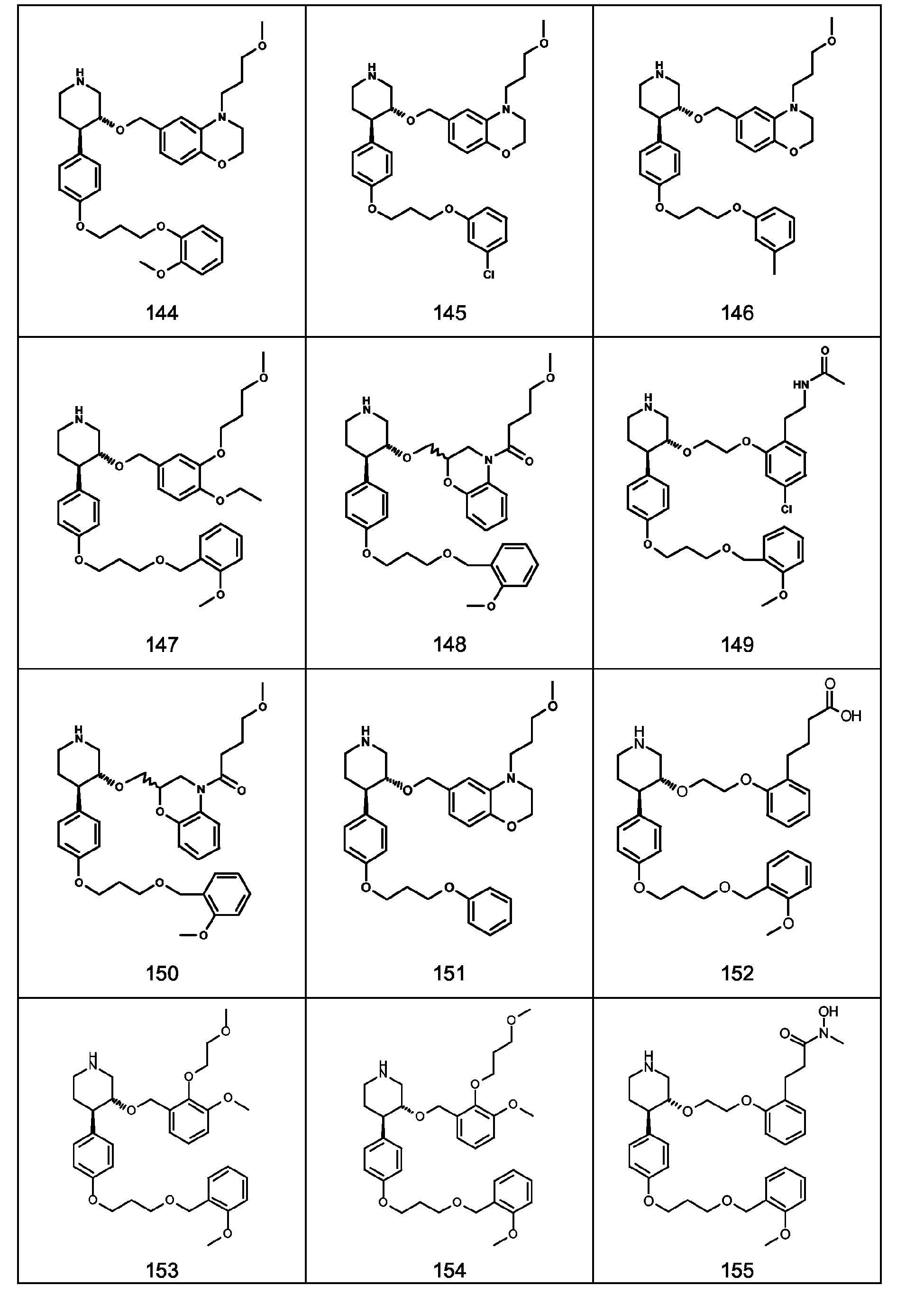

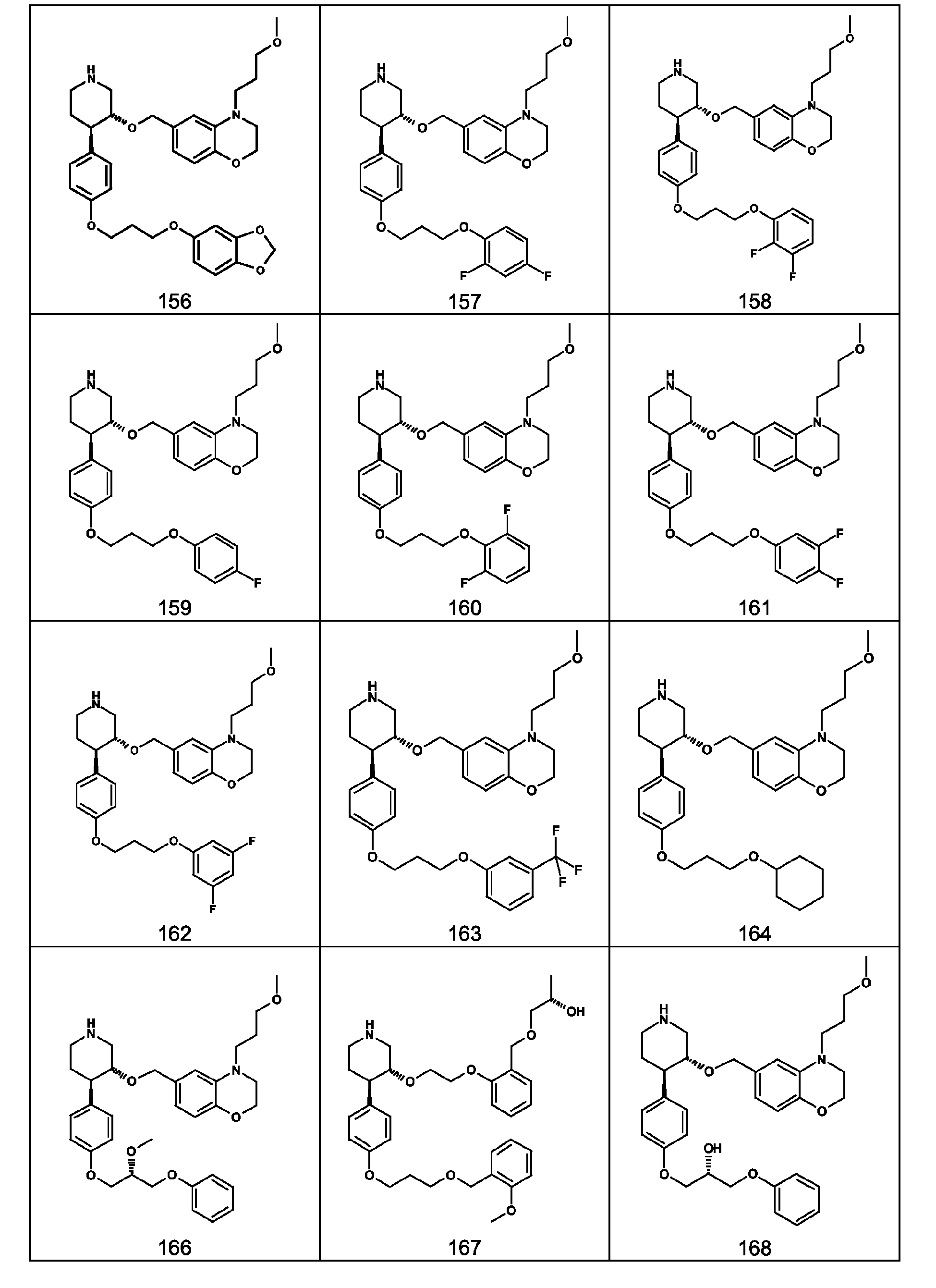

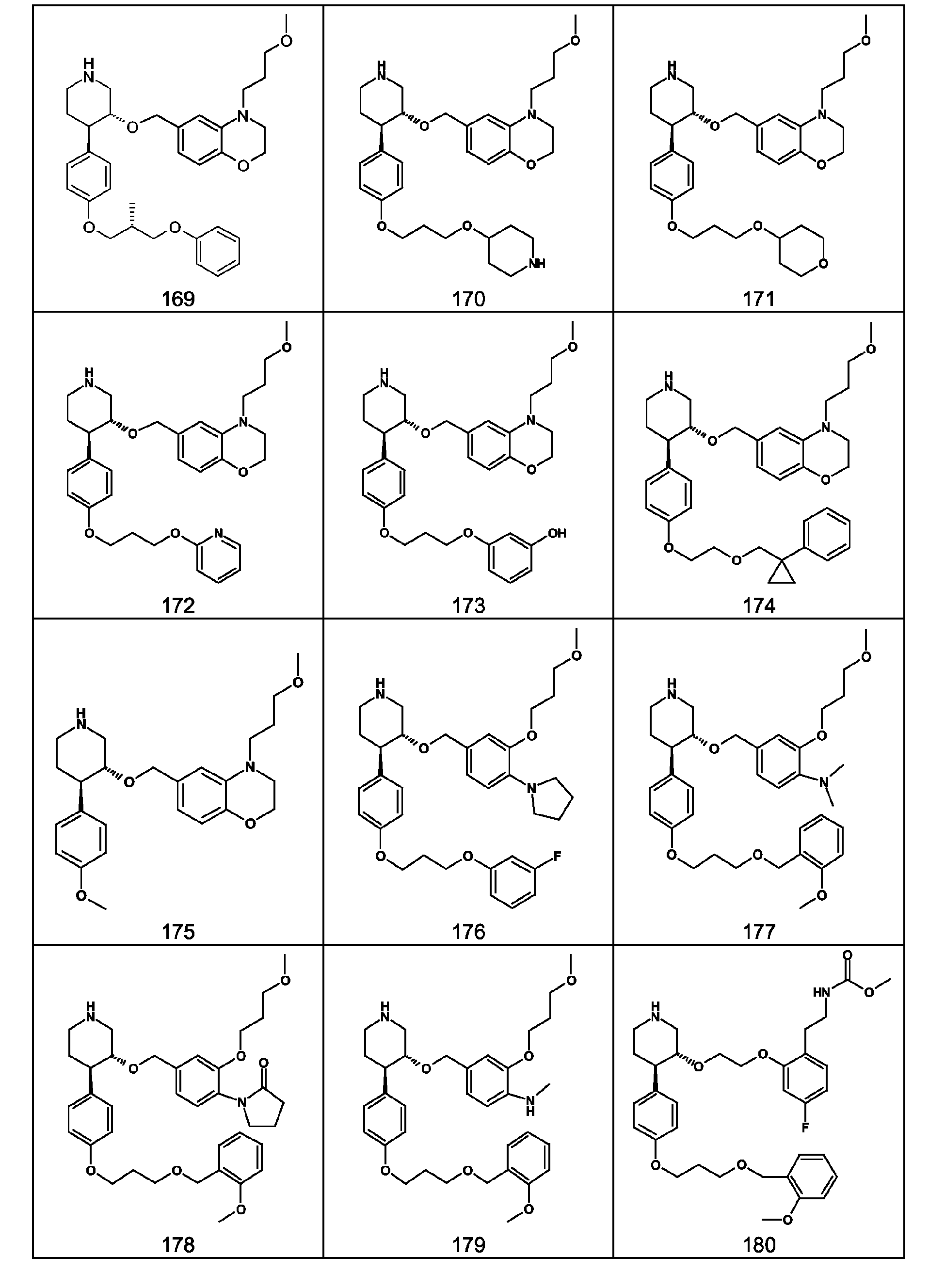

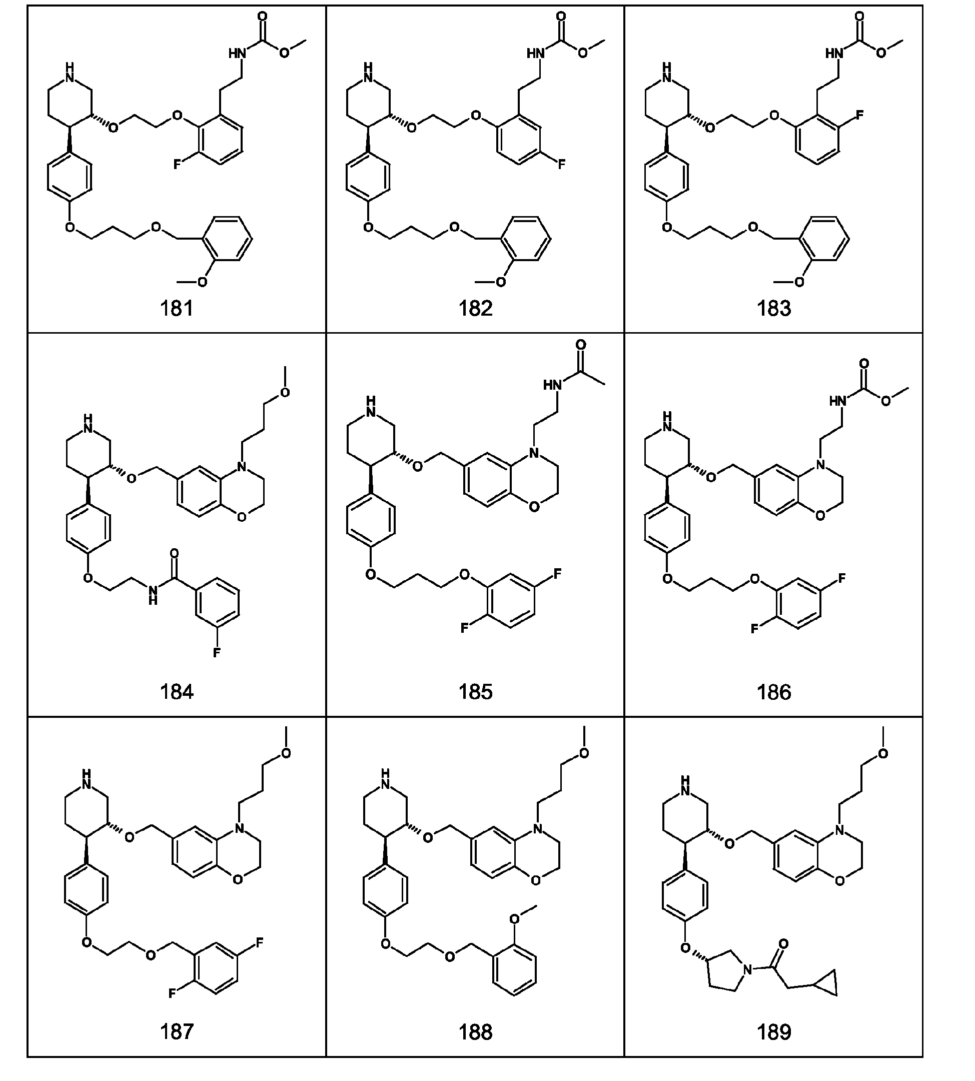

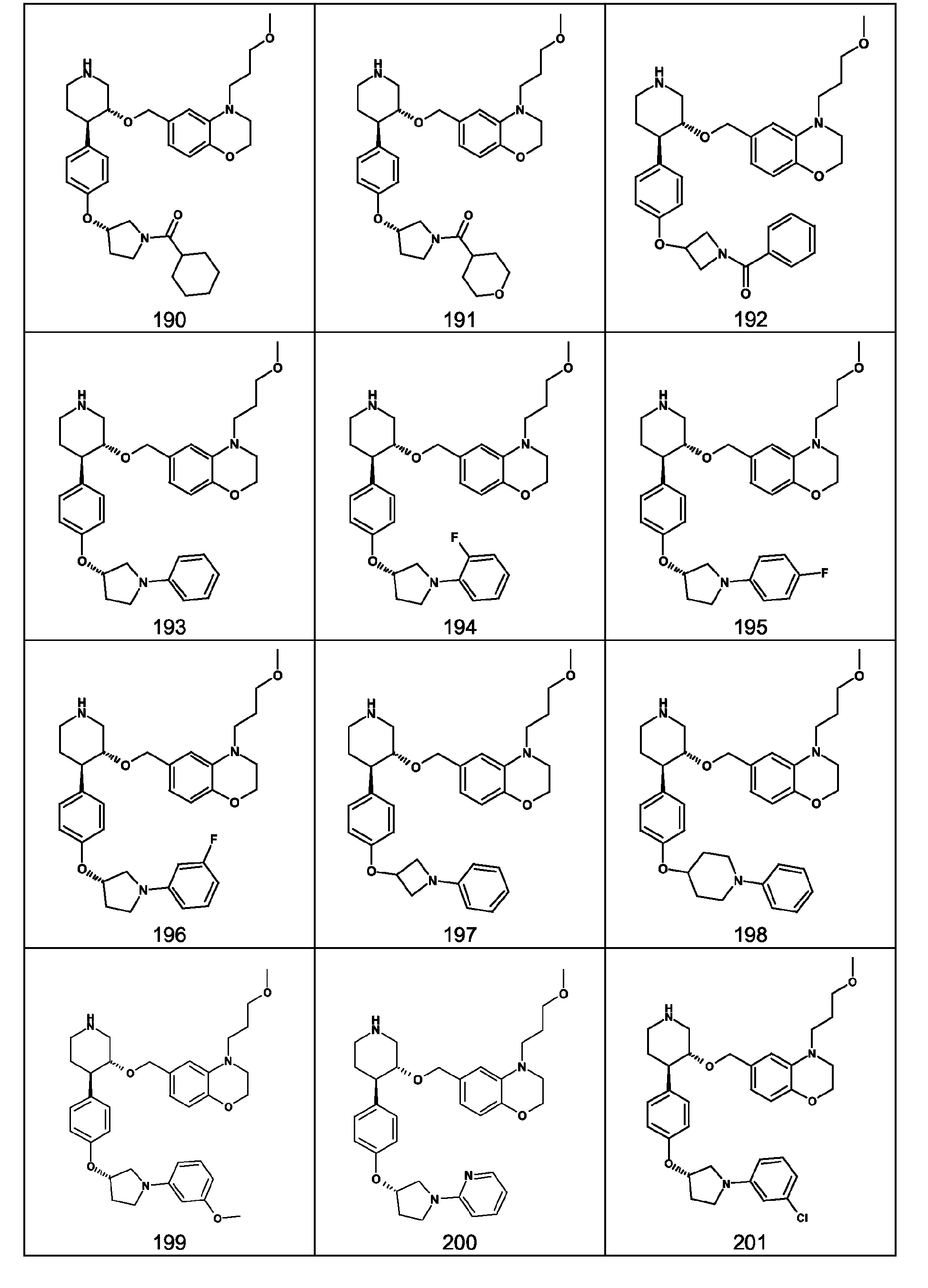

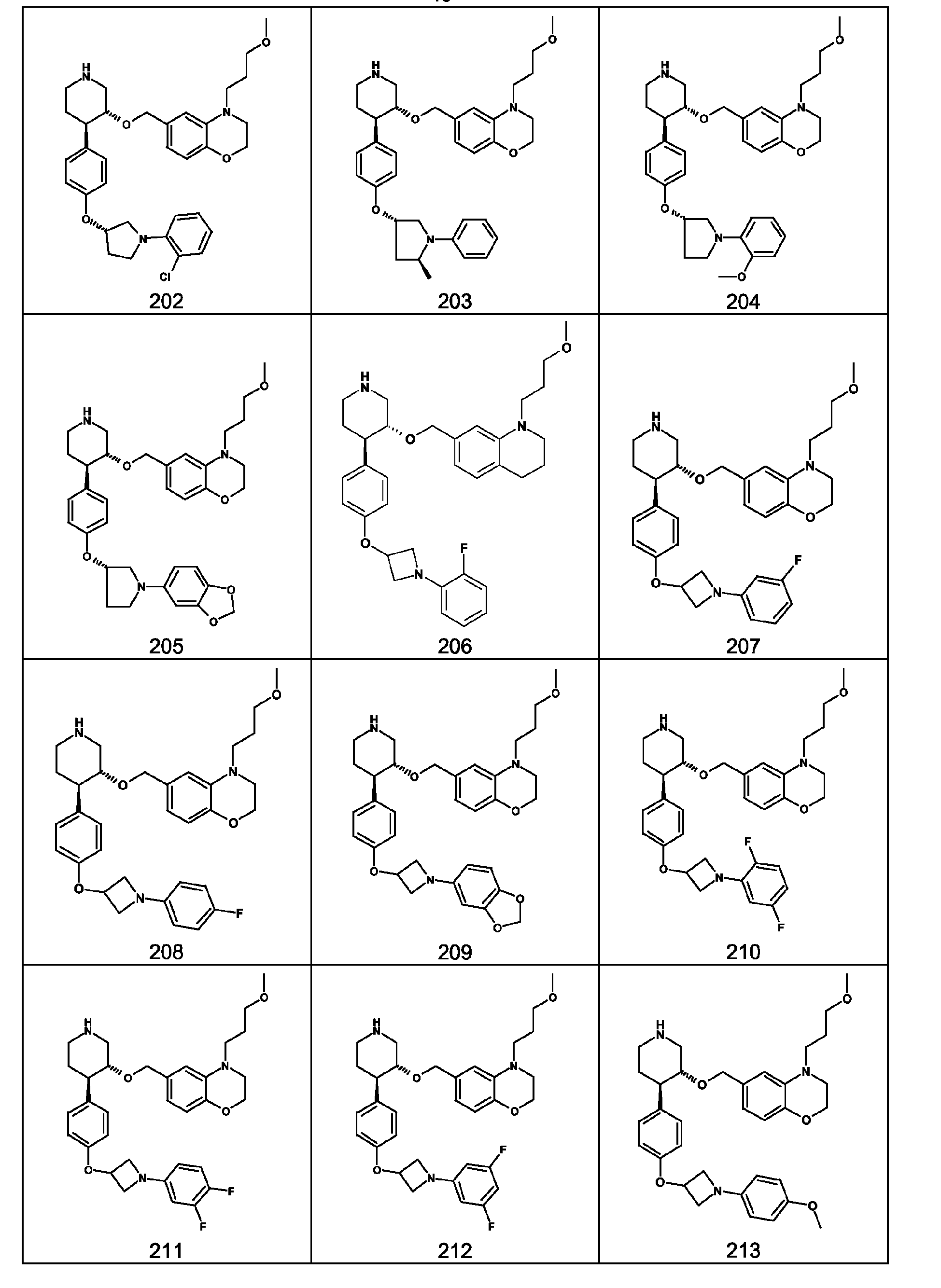

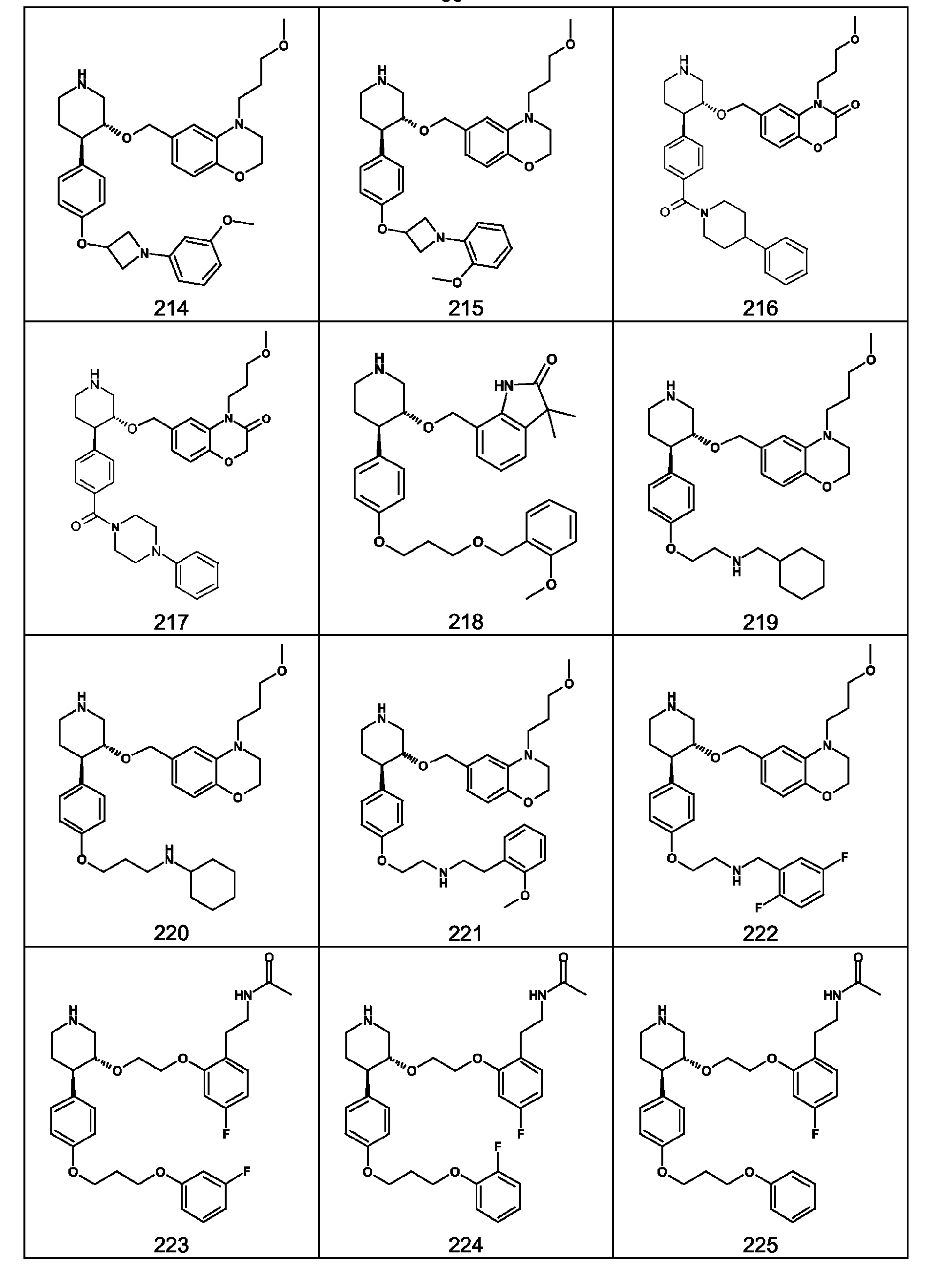

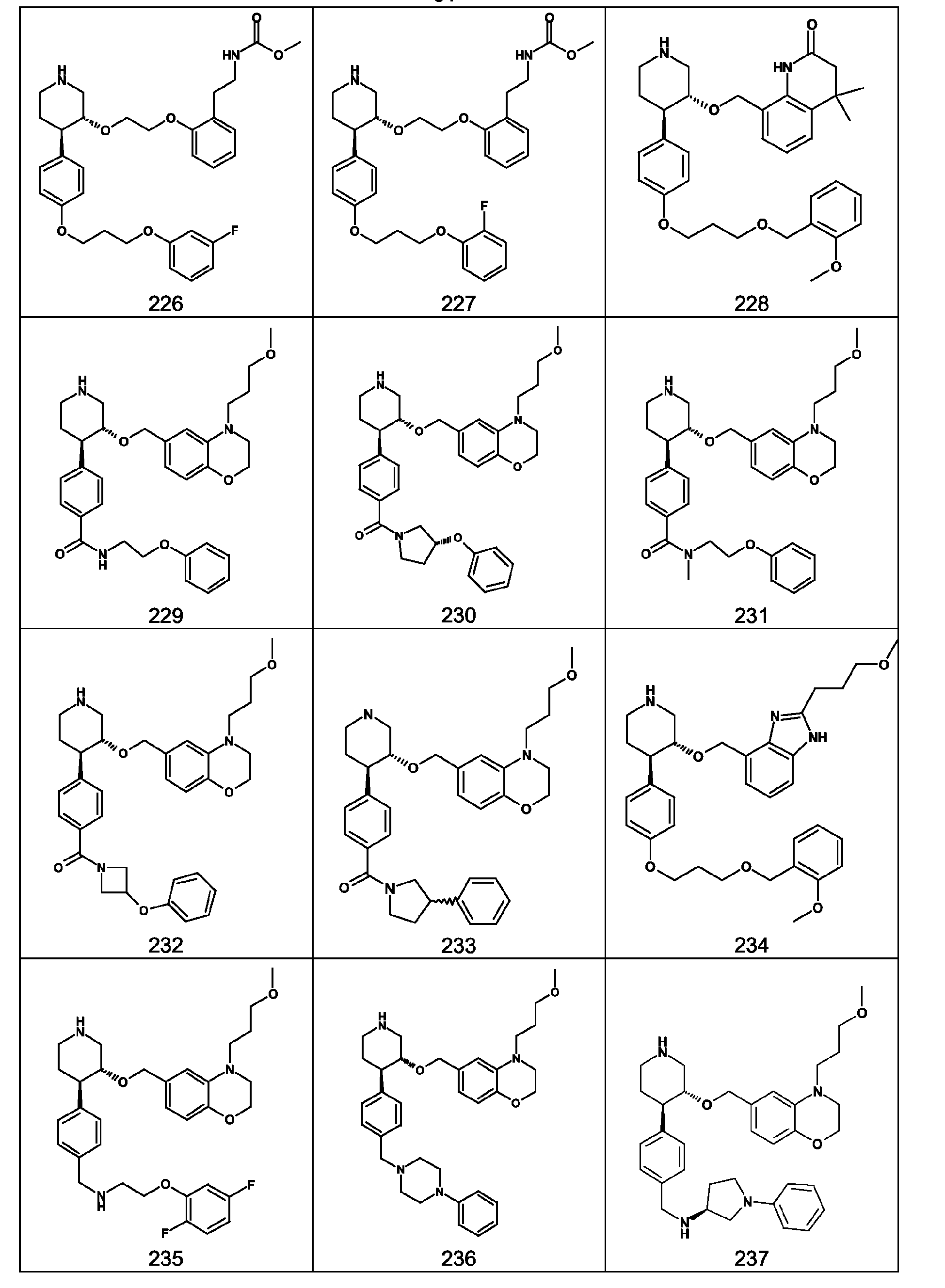

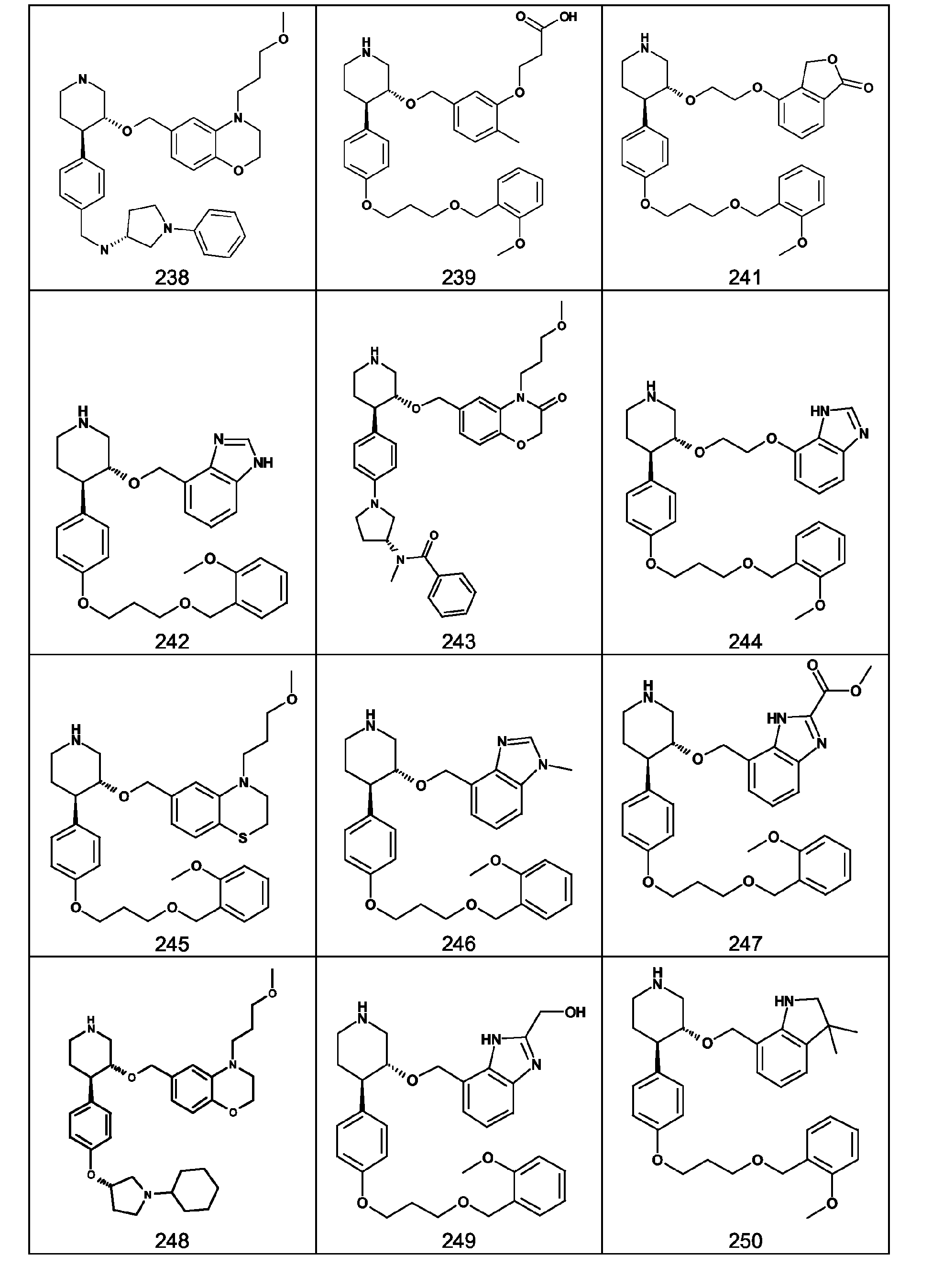

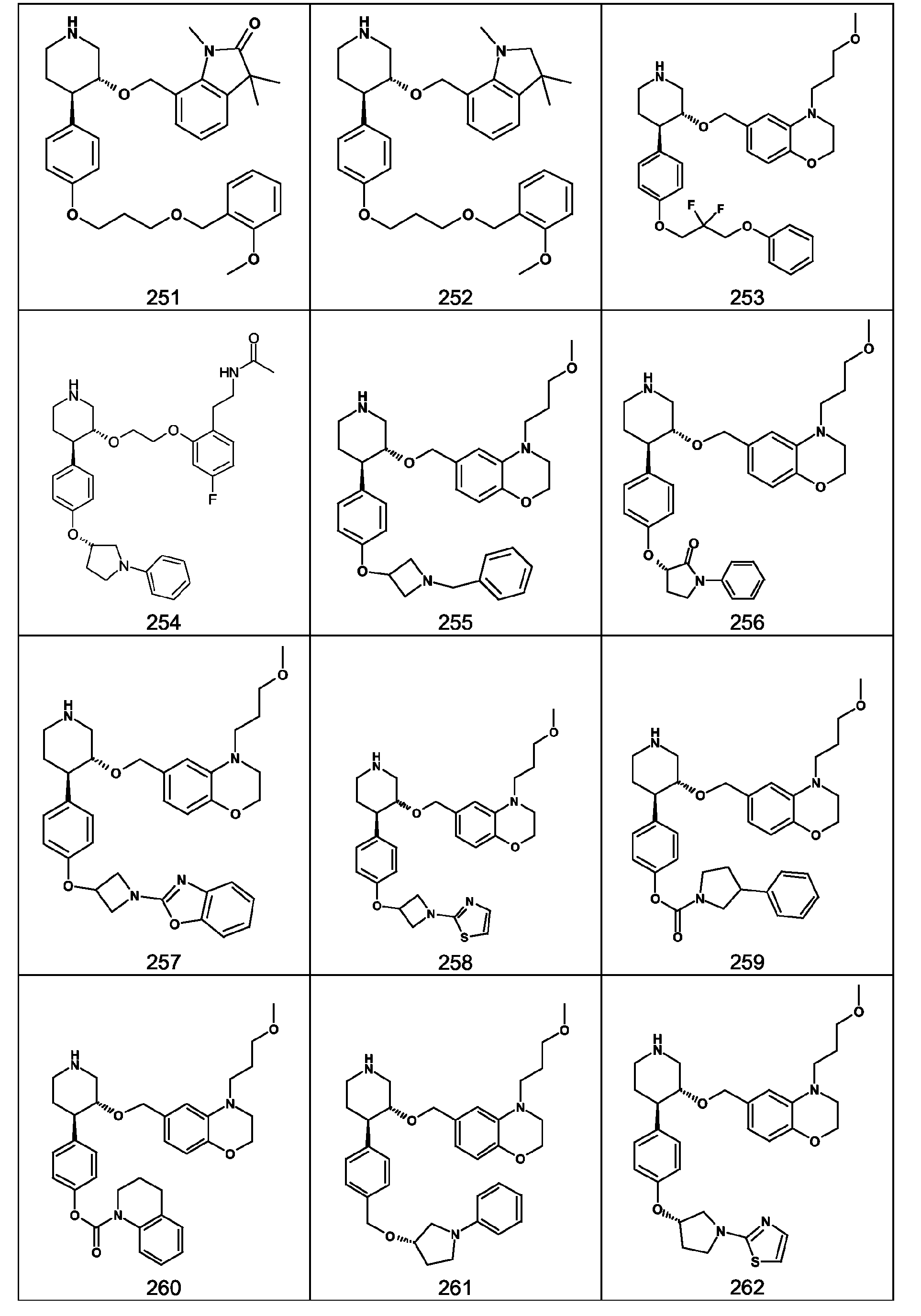

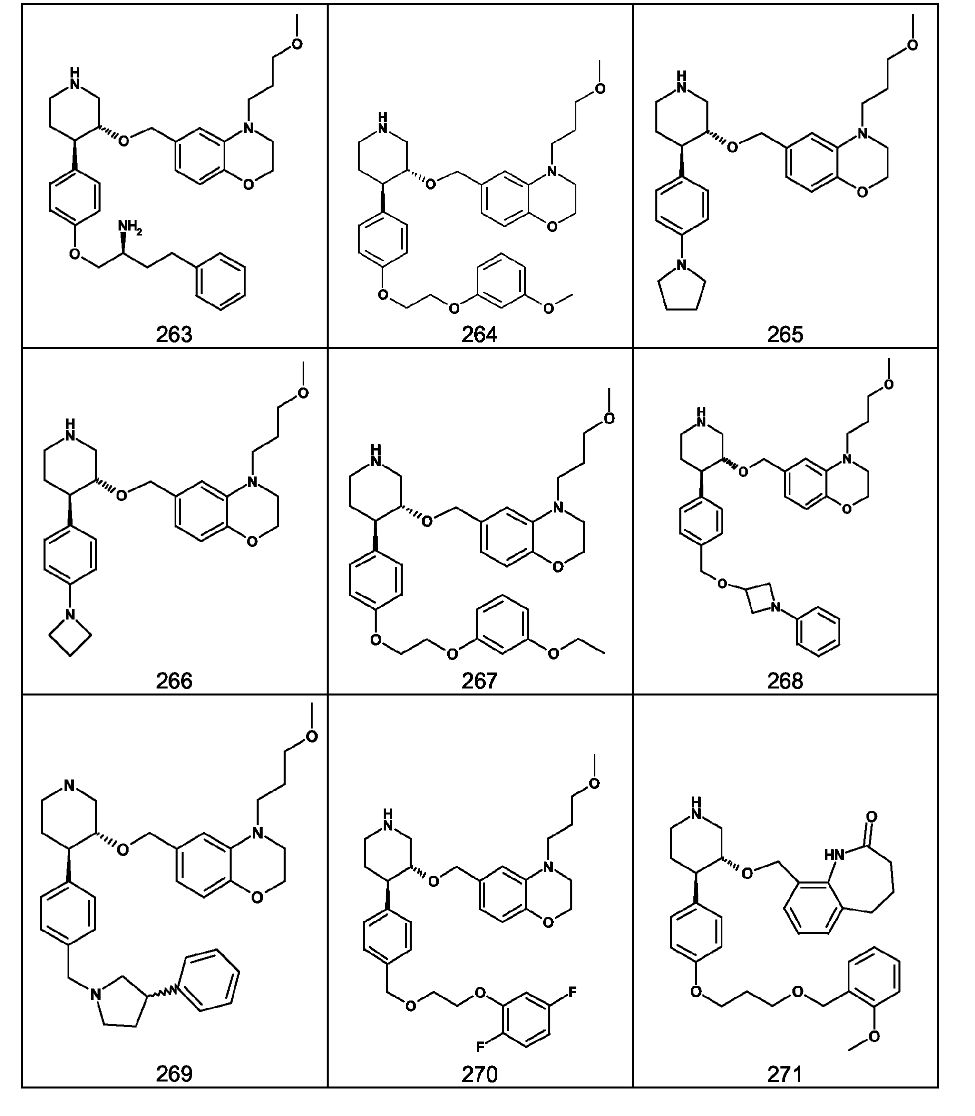

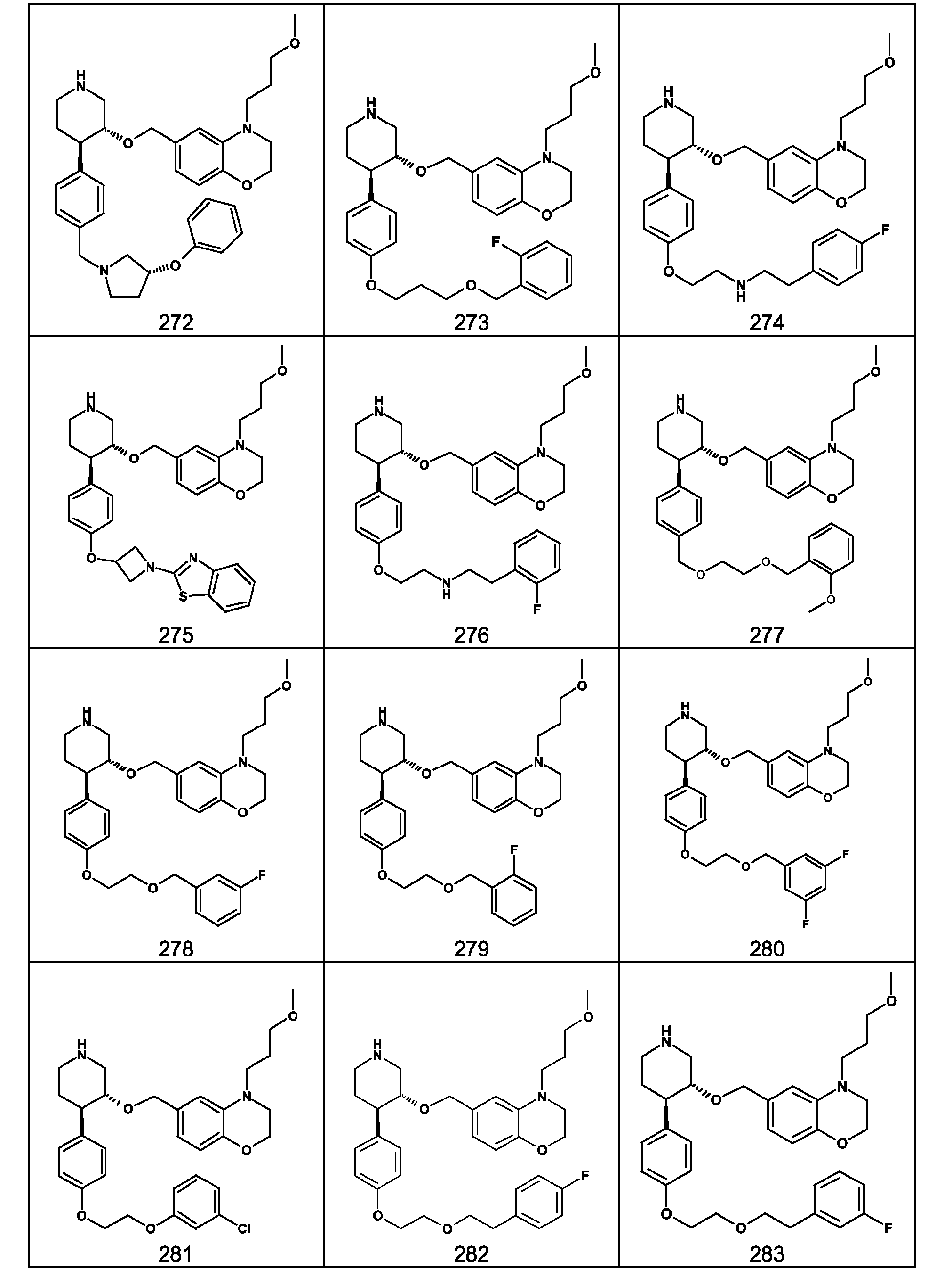

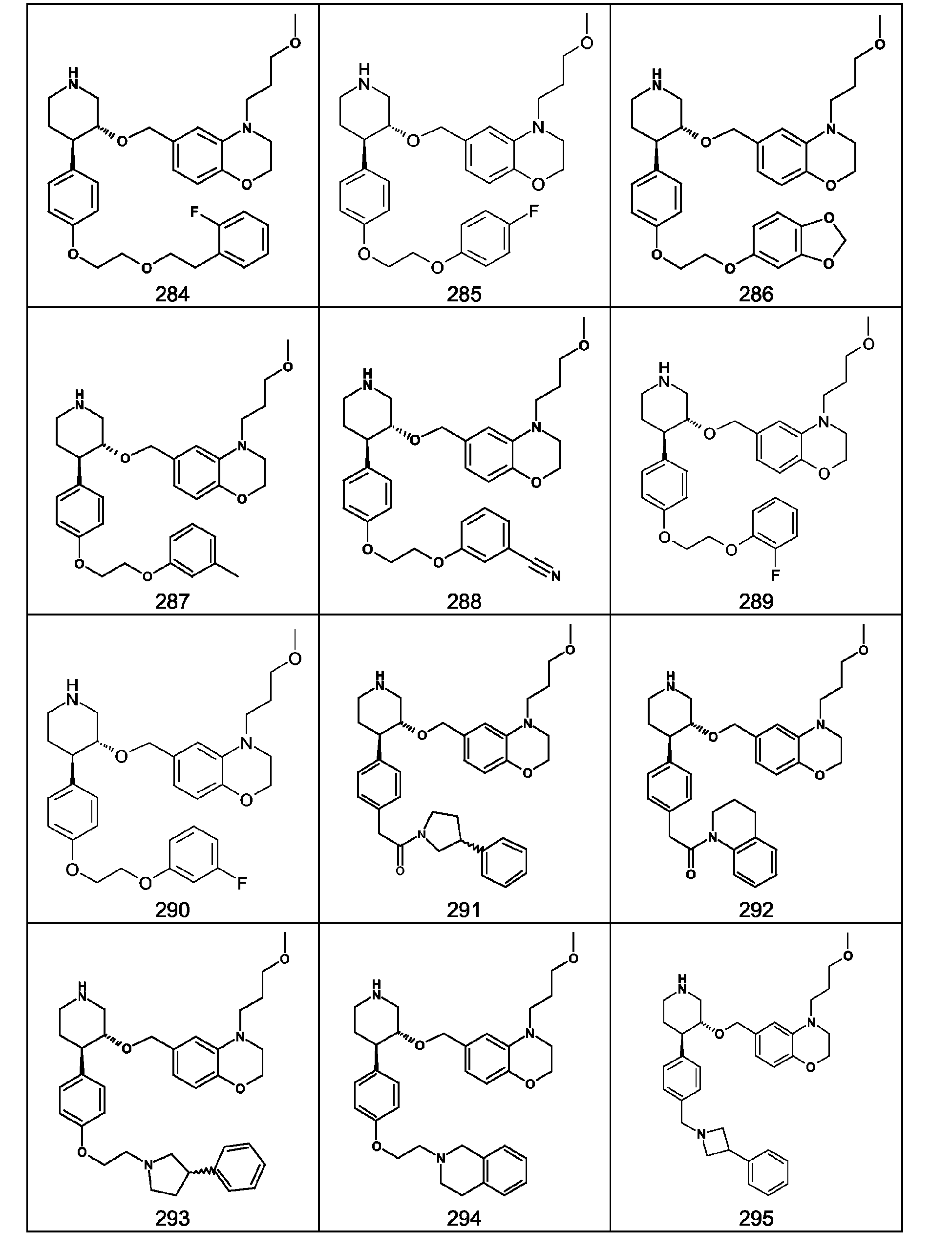

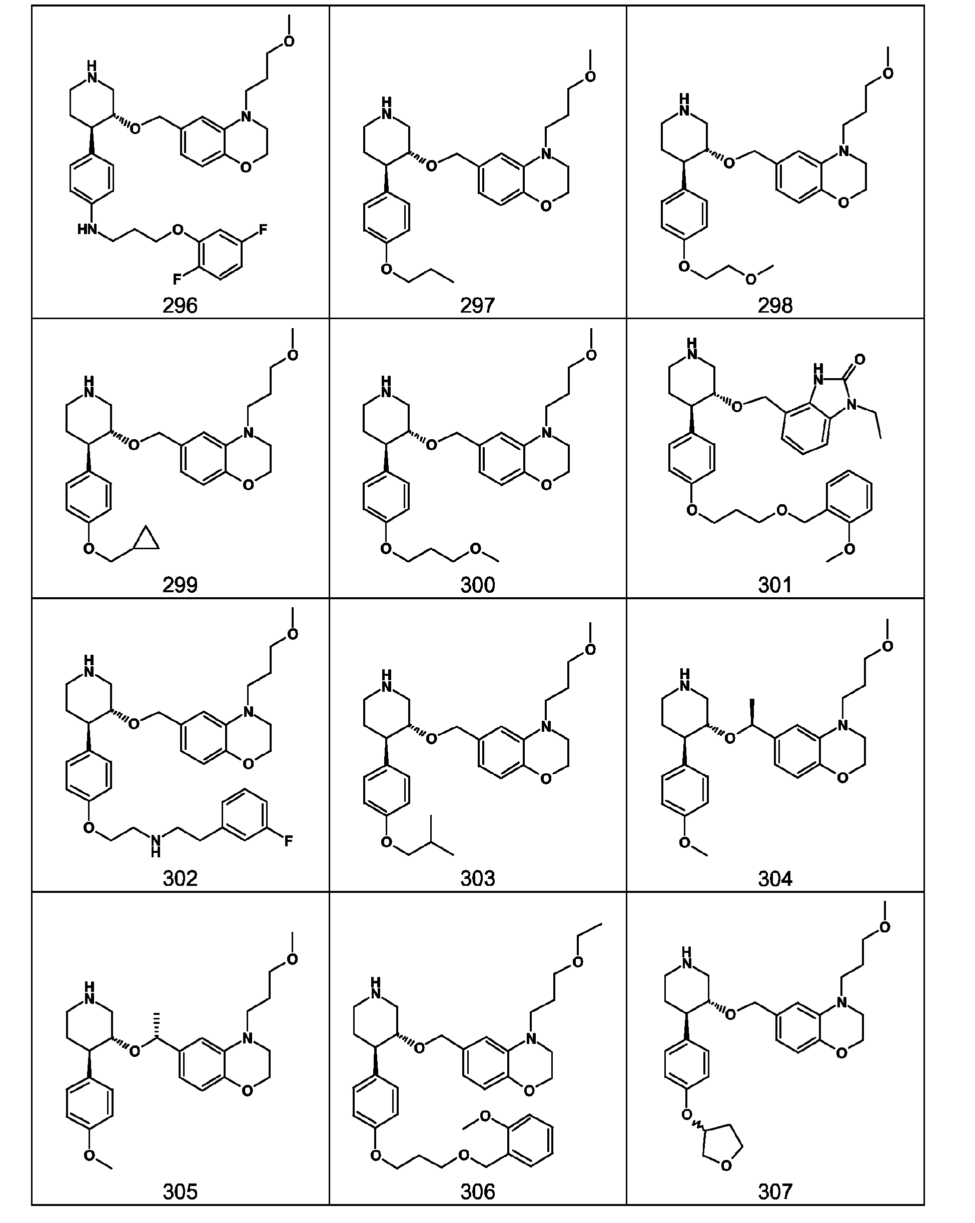

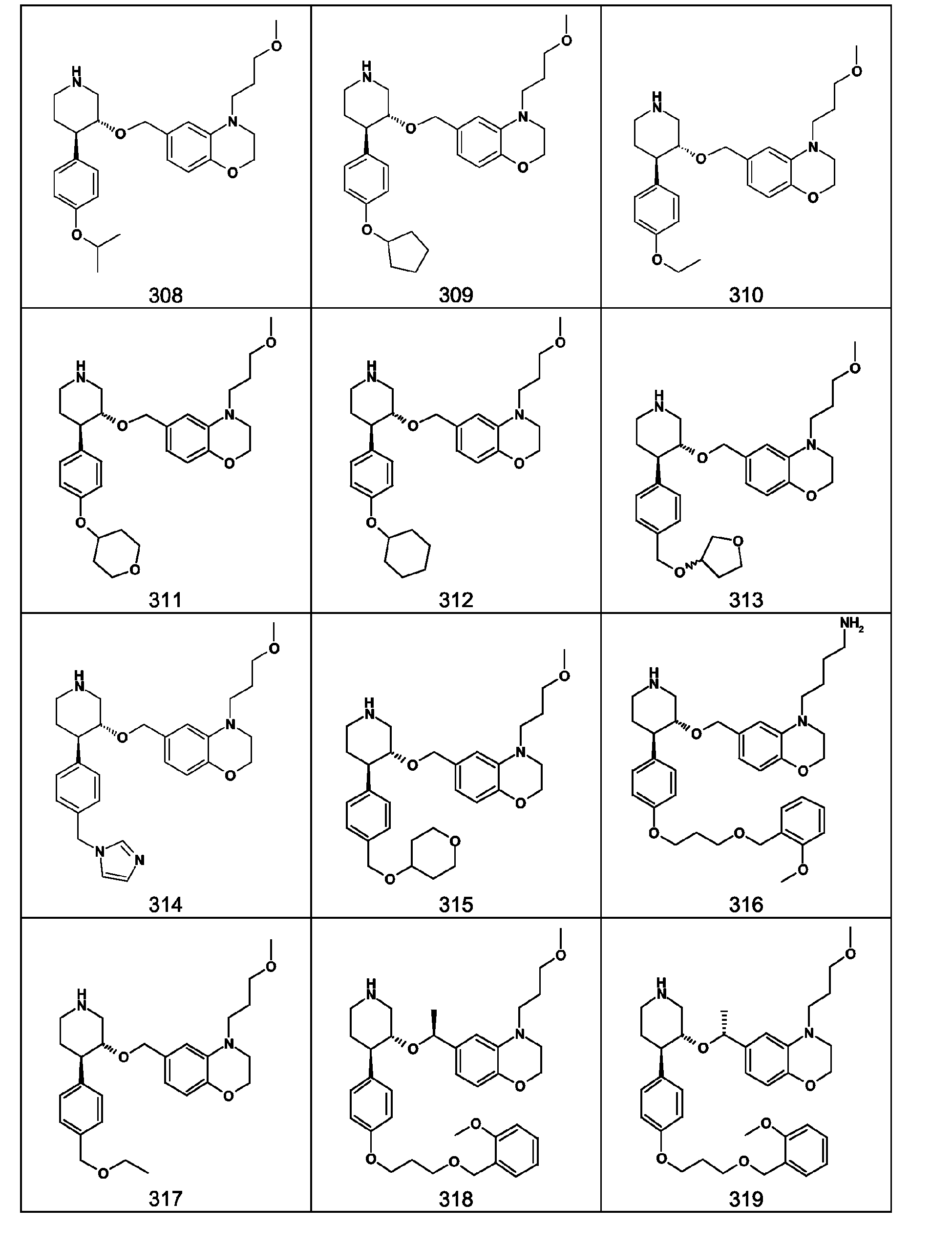

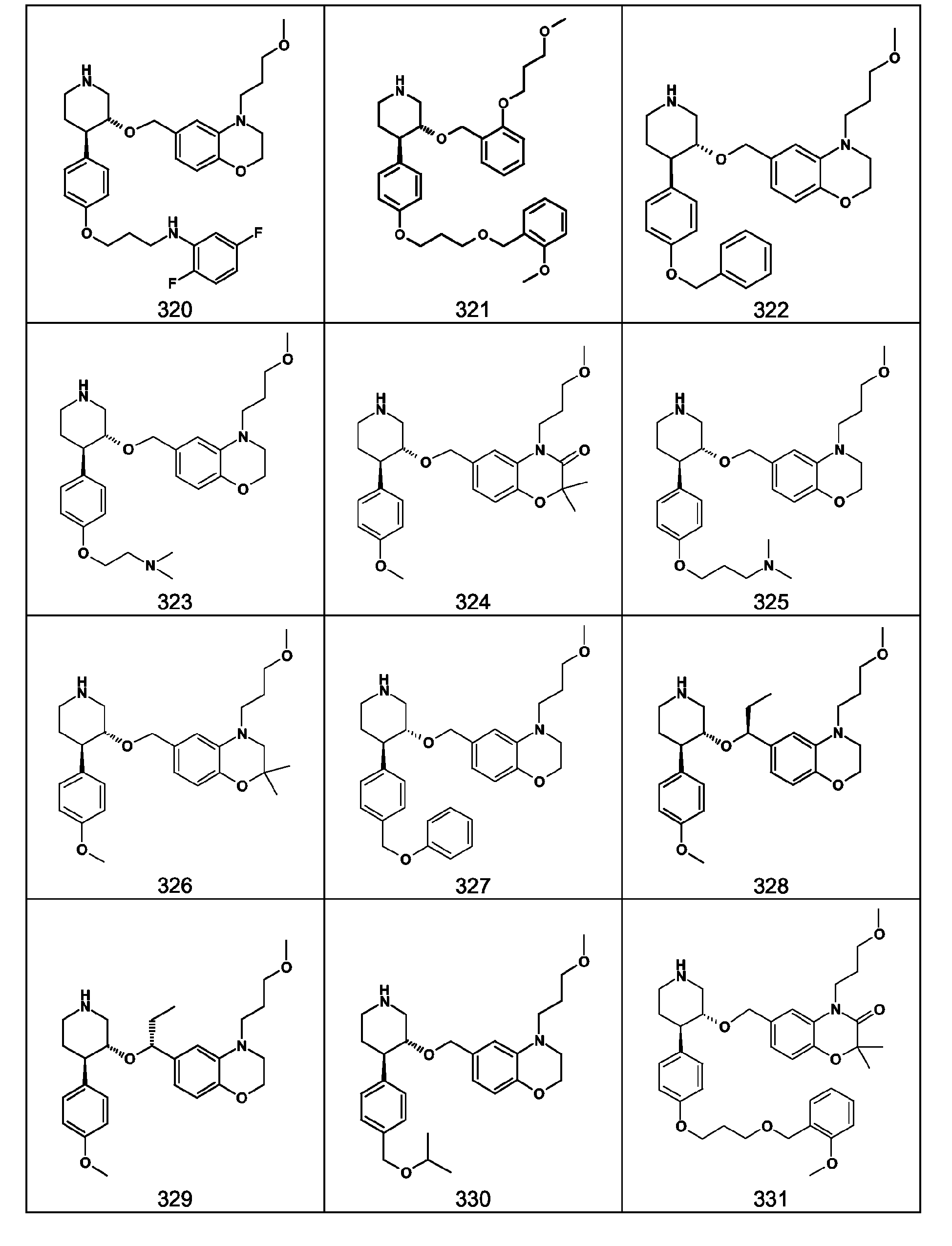

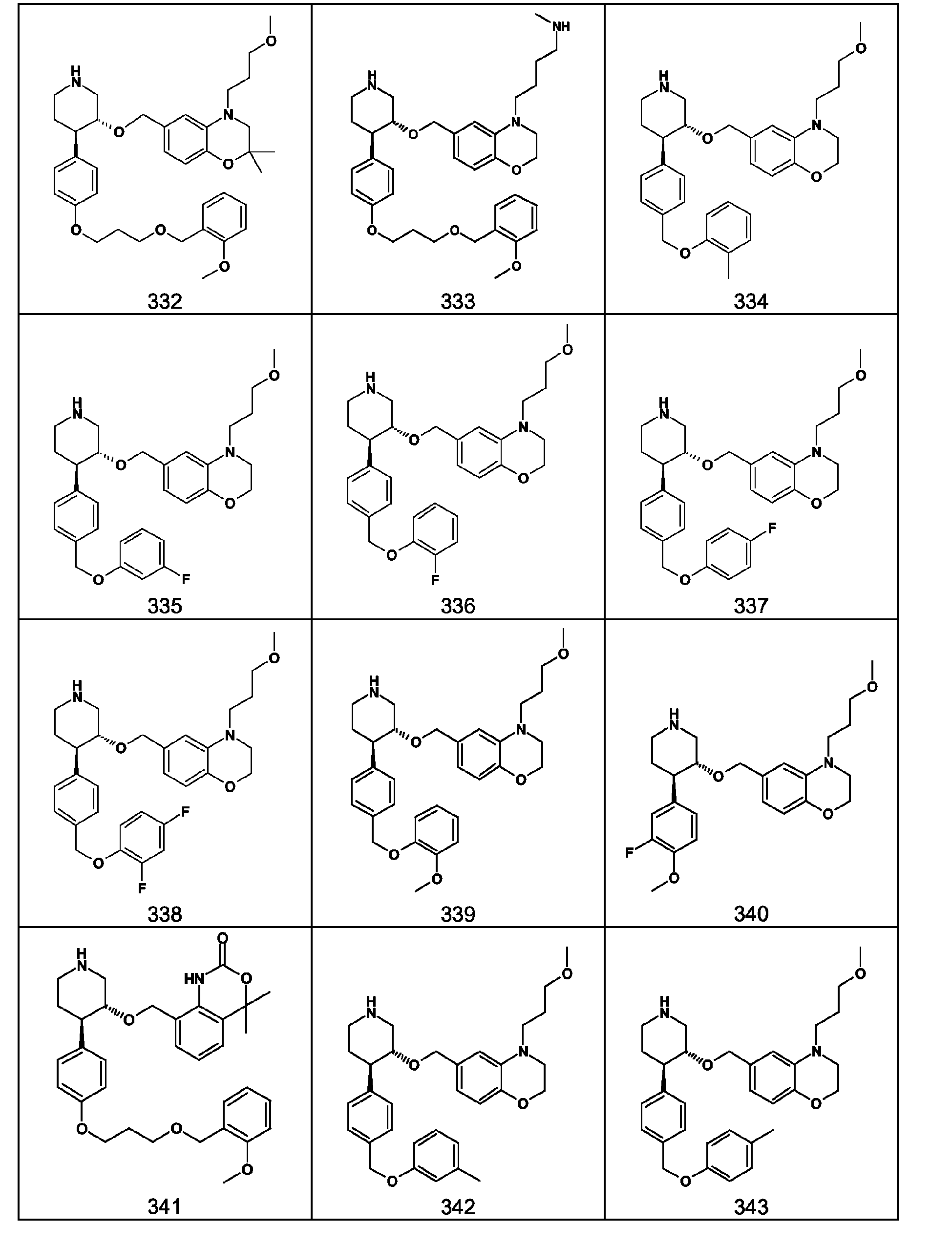

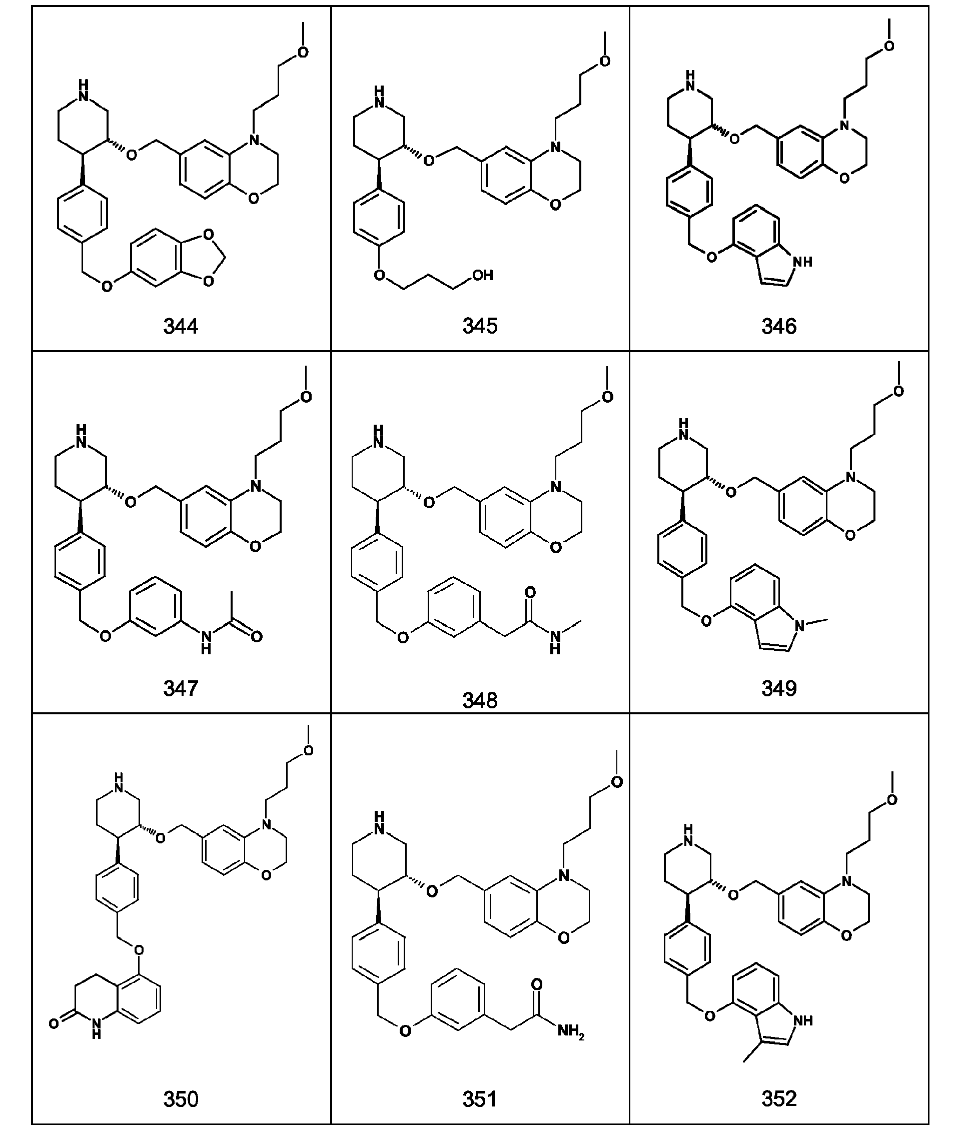

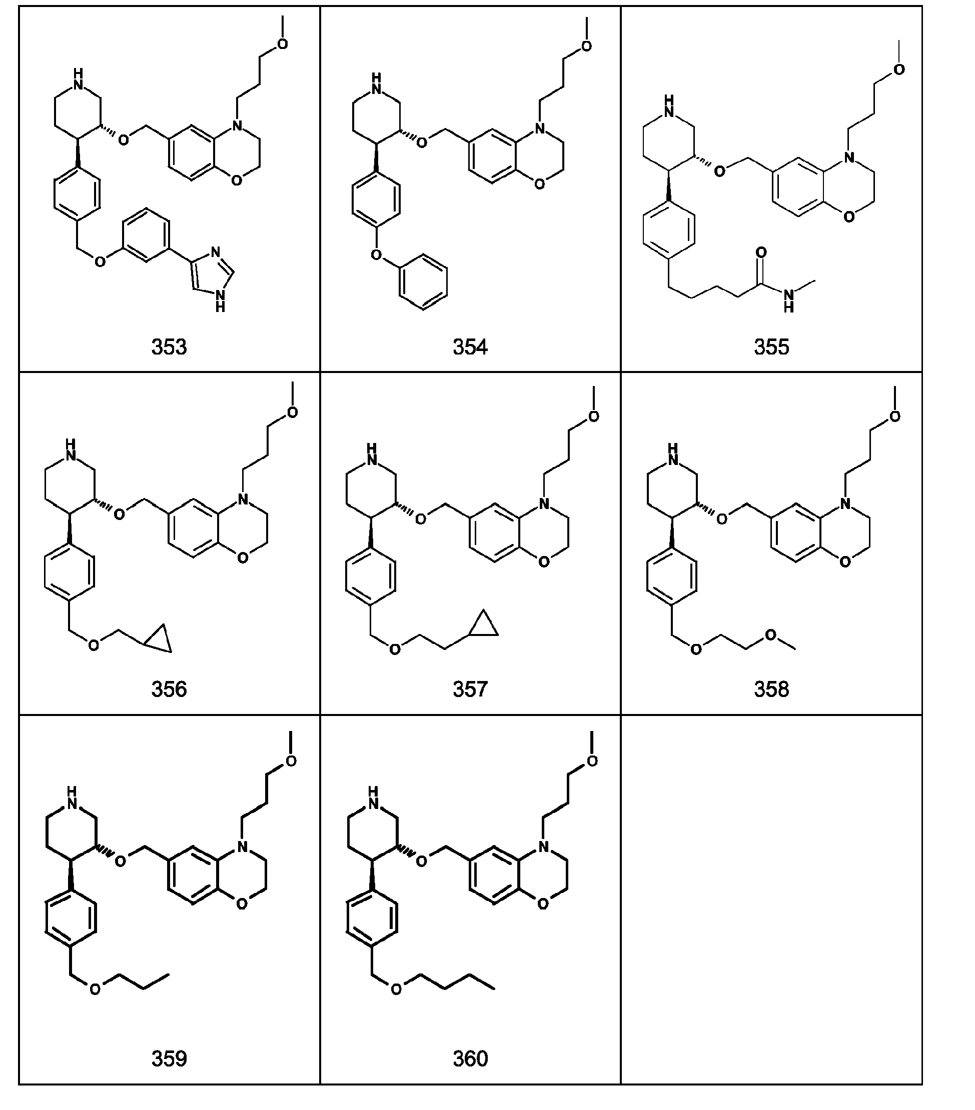

| **Nr.** | **Aspekt** | **R**_{**f**}**(System)** | **Rt (Method)** |
|---|---|---|---|
| 1 | gelbliches Oel | 0.28 (A) | 4.59 (I) |
| 2 | weisser Schaum | 0.44 (B) | 4.20 (I) |
| 3 | gelbes Oel | 0.25 (B) | 4.65 (I) |
| 5 | gelbliches Oel | 0.44 (C) | 4.53 (I) |
| 6 | gelbliches Oel | 0.20 (A) | 4.26 (I) |
| 7 | gelbliches Oel | 0.29 (A) | 4.67 (I) |
| 8 | gelbliches Oel | 0.25 (A) | 4.16 (I) |
| 9 | gelbliches Oel | 0.20 (A) | 4.06 (I) |
| 10 | gelbliches Oel | 0.15 (A) | 19.39(11) |
| 11 | gelbliches Oel | 0.15 (A) | 19.04(11) |
| 12 | gelbes Oel | 0.47 (A) | 4.84 (I) |
| 13 | gelbliches Oel | 0.30 (A) | 4.15 (I) |
| 14 | braunes Oel | 0.10 (A) | 4.25 (I) |
| 16 | gelbliches Oel | 0.13 (A) | 4.00 (I) |
| 17 | gelbliches Oel | 0.16 (A) | 4.59 (I) |
| 18 | gelbliches Oel | 0.31 (A) | 4.23 (I) |
| 19 | gelbliches Oel | 0.31 (A) | 4.71 (I) |
| 20 | gelbliches Oel | 0.20 (A) | 4.67 (I) |
| 21 | gelbliches Oel | 0.23 (A) | 4.96 (I) |
| 22 | gelbliches Oel | 0.30 (A) | 4.92 (I) |
| 23 | farbloses Oel | 0.15 (A) | 4.30 (I) |
| 24 | gelbliches Oel | 0.11 (A) | 4.16 (I) |
| 25 | farbloses Oel | 0.29 (A) | 3.99 (I) |
| 26 | farbloses Oel | 0.14 (A) | 4.07 (I) |
| 27 | farbloses Oel | 0.20 (A) | 4.49 (I) |
| 28 | farbloses Oel | 0.18 (A) | 4.27 (I) |
| 29 | gelbliches Oel | 0.17 (A) | 4.48 (I) |
| 30 | farbloses Oel | 0.24 (A) | 4.35 (I) |
| 31 | gelbliches Oel | 0.20 (A) | 4.91 (I) |
| 32 | farbloses Oel | 0.32 (A) | 4.69 (I) |
| 33 | gelbes Oel | 0.33 (A) | 4.86 (I) |
| 34 | gelbes Oel | 0.26 (D) | 3.81 (I) |
| 35 | gelber Schaum | 0.21 (D) | 4.00 (I) |
| 36 | gelbes Oel | 0.22 (D) | 3.84 (I) |
| 37 | oranges Oel | 0.10 (A) | 3.28 (I) |
| 38 | oranges Oel | 0.10 (A) | 4.27 (I) |
| 39 | farbloses Oel | 0.10 (A) | 4.43 (I) |
| 40 | farbloses Oel | 0.08 (A) | 3.66 (I) |
| 41 | farbloses Oel | 0.24 (A) | 4.48 (I) |
| 42 | farbloses Oel | 0.15 (A) | 4.33 (I) |
| 43 | gelbliches Oel | 0.27 (A) | 5.16 (I) |
| 44 | braunes Oel | 0.24 (A) | 4.36 (I) |
| 45 | gelbliches Oel | 0.21 (A) | 4.15 (I) |
| 46 | gelbliches Oel | 0.13 (A) | 2.81 (I) |
| 47 | gelblicher Feststoff | 0.32 (U) | 4.26 (I) |
| 48 | gelbliches Oel | 0.24 (A) | 4.63 (I) |
| 49 | farbloses Oel | 0.26 (A) | 4.32 (I) |
| 50 | farbloses Oel | 0.28 (A) | 4.89 (I) |
| 51 | farbloses Oel | 0.26 (A) | 4.06 (I) |
| 52 | farbloses Oel | 0.35 (A) | 4.69 (I) |
| 53 | farbloses Oel | 0.20 (A) | 4.69 (I) |
| 55 | farbloses Oel | 0.24 (A) | 4.69 (I) |
| 56 | farbloses Oel | 0.17 (A) | 4.35 (I) |
| 57 | gelbliches Oel | 0.23 (A) | 4.13 (I) |
| 58 | gelbliches Oel | 0.28 (A) | 4.35 (I) |
| 59 | gelbliches Oel | 0.19 (A) | 4.46 (I) |
| 60 | gelbliches Oel | 0.30 (A) | 4.77 (I) |
| 61 | gelbliches Oel | 0.12 (A) | 4.32 (I) |
| 62 | gelbes Oel | 0.25 (A) | 4.38 (I) |
| 63 | gelbes Oel | 0.20 (A) | 4.21 (I) |
| 64 | farbloses Oel | 0.20 (A) | 4.51 (I) |
| 65 | gelbliches Oel | 0.47 (E) | 4.08 (I) |
| 66 | farbloses Oel | 0.22 (A) | 3.83 (I) |
| 67 | farbloses Oel | 0.26 (A) | 4.67 (I) |
| 68 | farbloses Oel | 0.22 (A) | 4.42 (I) |
| 69 | gelbliches Oel | 0.14 (A) | 3.90 (I) |
| 70 | gelbliches Oel | 0.14 (A) | 4.10 (I) |
| 71 | gelbliches Oel | 0.14 (A) | 4.10 (I) |
| 72 | farbloses Oel | 0.16 (A) | 4.26 (I) |
| 73 | gelbes Oel | 0.10 (A) | 4.27 (I) |
| 74 | gelbliches Oel | 0.10 (A) | 4.31 (I) |
| 75 | gelbliches Oel | 0.10 (A) | 4.43 (I) |
| 76 | weisser Schaum | 0.08 (A) | 4.34 (I) |
| 77 | gelbes Oel | 0.12 (A) | 4.24 (I) |
| 78 | gelbes Oel | 0.12 (A) | 4.49 (I) |
| 79 | gelbliches Oel | 0.29 (A) | 3.75 (I) |
| 80 | gelbliches Oel | 0.36 (A) | 3.82 (I) |
| 81 | brauner Schaum | 0.20 (A) | 4.52 (I) |
| 82 | gelbes Oel | 0.35 (A) | 4.44 (I) |
| 83 | farbloses Oel | 0.25 (A) | 4.41 (I) |
| 84 | farbloses Oel | 0.32 (A) | 4.46 (I) |
| 85 | farbloses Oel | 0.10 (A) | 4.33 (I) |
| 86 | gelbliches Oel | 0.36 (A) | 3.95 (I) |
| 87 | gelbliches Oel | 0.31 (A) | 4.09 (I) |
| 88 | gelbliches Oel | 0.35 (A) | 4.31 (I) |
| 89 | gelbliches Oel | 0.36 (A) | 3.90 (I) |
| 90 | gelbliches Oel | 0.40 (A) | 4.49 (I) |
| 91 | gelbliches Oel | 0.43 (A) | 4.28 (I) |
| 92 | gelbliches Oel | 0.43 (A) | 4.48 (I) |
| 93 | gelbliches Oel | 0.35 (A) | 4.34 (I) |
| 94 | gelbliches Oel | 0.19 (A) | 4.39 (I) |
| 95 | gelbes Oel | 0.16 (A) | 4.44 (I) |
| 96 | gelbes Oel | 0.08 (A) | 4.56 (I) |
| 97 | gelbes Oel | 0.23 (A) | 4.27 (I) |
| 98 | gelbes Oel | 0.20 (A) | 3.46 (I) |
| 99 | gelber Schaum | 0.04 (A) | 4.34 (I) |
| 100 | gelbliches Oel | 0.24 (C) | 4.53 (I) |
| 101 | gelbliches Oel | 0.64 (A) | 4.32 (I) |
| 102 | gelbes Oel | 0.30 (A) | 4.34 (I) |
| 103 | gelbliches Oel | 0.42 (A) | 5.00 (I) |
| 104 | gelbliches Oel | 0.43 (A) | 4.13 (I) |
| 105 | gelblicher Feststoff | 0.24 (A) | 3.99 (I) |
| 106 | gelbes Oel | 0.15 (A) | 4.09 (I) |
| 107 | gelbes Oel | 0.15 (A) | 3.95 (I) |
| 108 | gelbes Oel | 0.11 (A) | 4.28 (I) |
| 109 | gelbes Oel | 0.18 (A) | 4.45 (I) |
| 110 | gelbes Oel | 0.56 (A) | 4.36 (I) |
| 111 | gelbes Oel | 0.30 (A) | 4.49 (I) |
| 112 | gelbes Oel | 0.48 (A) | 4.33 (I) |
| 113 | gelbes Oel | 0.32 (A) | 4.30 (I) |
| 114 | gelbes Oel | 0.54 (A) | 4.48 (I) |
| 115 | gelbes Oel | 0.19 (A) | 4.36 (I) |
| 116 | braunes Oel | 0.34 (A) | 4.48 (I) |
| 117 | gelblicher Feststoff | 0.34 (I) | 4.23 (I) |
| 118 | gelbes Oel | 0.16 (A) | 4.06 (I) |
| 119 | gelbliches Oel | 0.27 (A) | 4.13 (I) |
| 120 | gelbes Oel | 0.14 (A) | 4.30 (I) |
| 121 | gelbes Oel | 0.29 (A) | 4.70 (I) |
| 122 | gelbes Oel | 0.20 (A) | 3.14 (I) |
| 123 | gelbes Oel | 0.27 (A) | 4.48 (I) |
| 124 | gelbes Oel | 0.54 (A) | 4.43 (I) |
| 125 | gelbes Oel | 0.15 (A) | 2.89 (I) |
| 126 | gelbes Oel | 0.28 (A) | 2.94 (I) |
| 127 | gelbliches Oel | 0.36 (A) | 4.20 (I) |
| 128 | gelbliches Oel | 0.34 (A) | 4.05 (I) |
| 129 | gelbes Oel | 0.27 (A) | 4.16 (I) |
| 130 | farbloses Oel | 0.23 (A) | 4.20 (I) |
| 133 | gelbes Oel | 0.19 (A) | 4.35 (I) |
| 134 | farbloses Oel | 0.25 (A) | 3.54 (I) |
| 135 | gelbes Oel | 0.50 (F) | 4.17 (I) |
| 136 | gelbes Oel | 0.08 (A) | 4.04 (I) |
| 137 | gelbliches Oel | 0.25 (A) | 4.75 (I) |
| 138 | farbloses Oel | 0.28 (A) | 4.11 (I) |
| 139 | gelbliches Oel | 0.20 (H) | 4.42 (I) |
| 140 | gelbliches Oel | 0.17 (A) | 4.54 (I) |
| 141 | gelbliches Oel | 0.41 (A) | 4.42 (I) |
| 142 | gelbliches Oel | 0.41 (A) | 4.52 (I) |
| 143 | gelbliches Oel | 0.22 (A) | 4.76 (I) |
| 144 | gelbliches Oel | 0.27 (A) | 4.30 (I) |
| 145 | gelbliches Oel | 0.08 (A) | 4.84 (I) |
| 146 | gelbliches Oel | 0.33 (G) | 4.70 (I) |
| 147 | gelbliches Oel | 0.35 (A) | 4.53 (I) |
| 148 | gelbliches Oel | 0.16 (H) | 17.78(11) |
| 149 | gelbes Oel | 0.50 (A) | 4.48 (I) |
| 150 | gelbliches Oel | 0.26 (H) | 18.03(11) |
| 151 | gelbes Oel | 0.23 (A) | 4.52 (I) |
| 152 | gelblicher Feststoff | 0.08 (A) | 4.37 (I) |
| 153 | farbloses Oel | 0.30 (A) | 4.39 (I) |
| 154 | farbloses Oel | 0.29 (A) | 4.49 (I) |
| 155 | oranges Oel | 0.28 (A) | 4.20 (I) |
| 156 | oranges Oel | 0.34 (C) | 4.38 (I) |
| 157 | oranges Oel | 0.31 (C) | 4.54 (I) |
| 158 | gelbes Oel | 0.30 (C) | 4.55 (I) |
| 159 | gelbes Oel | 0.33 (C) | 4.54 (I) |
| 160 | oranges Oel | 0.20 (A) | 4.47 (I) |
| 161 | oranges Oel | 0.10 (A) | 4.55 (I) |
| 162 | oranges Oel | 0.11 (A) | 4.62 (I) |
| 163 | oranges Oel | 0.07 (A) | 4.81 (I) |
| 164 | gelbes Oel | 0.23 (A) | 4.81 (I) |
| 166 | gelbes Oel | 0.29 (C) | 4.34 (I) |
| 167 | gelbliches Oel | 0.11 (A) | 4.22 (I) |
| 168 | gelbliches Oel | 0.35 (A) | 3.88 (I) |
| 169 | farbloses Oel | 0.37 (A) | 4.75 (I) |
| 170 | gelbes Oel | 0.15 (K) | 2.95 (I) |
| 171 | gelbes Oel | 0.21 (A) | 3.73 (I) |
| 172 | gelbes Oel | 0.22 (A) | 3.56 (I) |
| 173 | gelbliches Oel | 0.55 (A) | 3.84 (I) |
| 174 | gelbliches Oel | 0.23 (A) | 4.72 (I) |
| 175 | gelbliches Oel | 0.34 (A) | 3.58 (I) |
| 176 | gelbliches Oel | 0.32 (A) | 3.82 (I) |
| 177 | gelbliches Oel | 0.30 (A) | 3.58 (I) |
| 178 | oranges Oel | 0.30 (A) | 4.10 (I) |
| 179 | gelbliches Oel | 0.09 (A) | 3.59 (I) |
| 180 | gelbes Oel | 0.37 (A) | 4.54 (I) |
| 181 | gelb-oranges Oel | 0.10 (A) | 4.42 (I) |
| 182 | gelbes Oel | 0.17 (A) | 4.40 (I) |
| 183 | gelb-oranges Oel | 0.20 (A) | 4.45 (I) |
| 184 | braun-oranges Oel | 0.33 (A) | 3.72 (I) |
| 185 | gelber Schaum | 0.19 (A) | 4.14 (I) |
| 186 | gelber Schaum | 0.32 (A) | 4.33 (I) |
| 187 | gelbliches Oel | 0.35 (A) | 4.43 (I) |
| 188 | gelbliches Oel | 0.21 (A) | 4.38 (I) |
| 189 | gelbliches Oel | 0.18 (A) | 3.50 (I) |
| 190 | gelbes Oel | 0.10 (P) | 3.85 (I) |
| 191 | gelbes Oel | 0.20 (P) | 3.13 (I) |
| 192 | braunes Oel | 0.20 (S) | 3.59 (I) |
| 193 | gelbes Oel | 0.19 (A) | 4.57 (I) |
| 194 | gelbes Harz | 0.26 (A) | 4.57 (I) |
| 195 | gelbes Harz | 0.37 (A) | 4.46 (i) |
| 196 | gelbes Harz | 0.26 (A) | 4.59 (I) |
| 197 | gelbes Oel | 0.21 (A) | 4.43 (I) |
| 198 | gelbes Oel | 0.37 (A) | 3.26 (I) |
| 199 | gelbes Oel | 0.37 (A) | 4.40 (I) |
| 200 | dunkelgelbes Oel | 0.22 (A) | 2.91 (I) |
| 201 | oranges Oel | 0.17 (A) | 4.84 (I) |
| 202 | braunes Oel | 0.20 (A) | 4.65 (I) |
| 203 | gelbes Oel | 0.26 (A) | 3.88 (I) |
| 204 | gelbes Oel | 0.20 (Q) | 3.23 (I) |
| 205 | braunes Oel | 0.40 (R) | 3.99 (I) |
| 206 | gelbes Oel | 0.29 (A) | 4.58 (I) |
| 207 | gelbes Oel | 0.29 (A) | 4.60 (I) |
| 208 | gelbes Oel | 0.29 (A) | 4.42 (I) |
| 209 | gelbes Oel | 0.18 (A) | 4.04 (I) |
| 210 | gelbliches Oel | 0.30 (A) | 4.66 (I) |
| 211 | gelbliches Oel | 0.24 (A) | 4.61 (I) |
| 212 | gelbliches Oel | 0.28 (A) | 4.74 (I) |
| 213 | gelbes Oel | 0.26 (A) | 3.75 (I) |
| 214 | gelbes Oel | 0.27 (A) | 4.38 (I) |
| 215 | gelbes Oel | 0.25 (A) | 3.89 (I) |
| 216 | gelber Schaum | 0.20 (A) | 3.75 (I) |
| 217 | gelbes Harz | 0.20 (A) | 3.27 (I) |
| 218 | weisser Schaum | 0.33 (A) | 4.33 (I) |
| 219 | gelbes Oel | 0.18 (A) | 3.36 (I) |
| 220 | gelbliches Oel | 0.25 (A) | 3.14 (I) |
| 221 | farbloses Oel | 0.22 (A) | 3.30 (I) |
| 222 | farbloses Oel | 0.23 (A) | 3.13 (I) |
| 223 | gelbliches Oel | 0.22 (A) | 4.29 (I) |
| 224 | gelbliches Oel | 0.21 (A) | 4.19 (I) |
| 225 | gelbliches Oel | 0.12 (A) | 4.23 (I) |
| 226 | gelbliches Oel | 0.60 (N) | 4.58 (I) |
| 227 | gelbliches Oel | 0.20 (A) | 4.47 (I) |
| 228 | gelbliches Oel | 0.22 (A) | 4.31 (I) |
| 229 | oranges Oel | 0.30 (A) | 3.74 (I) |
| 230 | oranges Oel | 0.35 (A) | 3.83 (I) |
| 231 | oranges Oel | 0.37 (A) | 3.85 (I) |
| 232 | gelbliches Oel | 0.16 (A) | 3.85 (I) |
| 233 | gelbliches Oel | 0.21 (A) | 3.88 (I) |
| 234 | gelbliches Oel | 0.3 (T) | 3.42 (I) |
| 235 | gelbliches Oel | 0.14 (A) | 3.30 (I) |
| 236 | gelbliches Oel | 0.25 (A) | 3.30 (I) |
| 237 | gelbes Oel | 0.27 (A) | 3.45 (I) |
| 238 | gelbes Oel | 0.22 (A) | 3.45 (I) |
| 239 | gelbliches Oel | 0.37 (J) | 4.18 (I) |
| 241 | gelbes Oel | 0.36 (A) | 4.12 (I) |
| 242 | gelbes Oel | 0.25 (L) | 3.24 (I) |
| 243 | gelbes Oel | 0.10 (D) | 3.43 (I) |
| 244 | gelber Schaum | 0.05 (M) | 3.31 (I) |
| 245 | gelbes Oel | 0.27 (A) | 4.80 (I) |
| 246 | farbloses Oel | 0.10 (O) | 3.32 (I) |
| 247 | weisser Schaum | 0.23 (A) | 3.74 (I) |
| 248 | gelbes Harz | 0.11 (A) | 3.14 (I) |
| 249 | gelbes Oel | 0.39 (I) | 3.26 (I) |
| 250 | farbloses Harz | 0.09 (A) | 3.53 (I) |
| 251 | gelbliches Oel | 0.27 (A) | 4.25 (I) |
| 252 | farbloses Oel | 0.16 (A) | 4.02 (I) |
| 253 | gelbes Oel | 0.15 (Q) | 4.45 (I) |
| 254 | gelbes Oel | 0.16 (A) | 4.16 (I) |
| 255 | gelbes Oel | 0.15 (A) | 3.12 (I) |
| 256 | farbloses Harz | 0.34 (A) | 3.93 (I) |
| 257 | farbloser Schaum | 0.15 (A) | 3.74 (I) |
| 258 | braun-gelbes Harz | 0.20 (A) | 2.91 (I) |
| 259 | gelbliches Oel | 0.18 (A) | 4.33 (I) |
| 260 | gelbliches Oel | 0.19 (A) | 4.46 (I) |
| 261 | gelbliches Oel | 0.11 (C) | 4.29 (I) |
| 262 | gelbes Harz | 0.20 (A) | 2.87 (I) |
| 263 | gelbliches Oel | 0.20 (A) | 3.28 (I) |
| 264 | farbloses Oel | 0.28 (A) | 4.18 (I) |
| 265 | gelbliches Oel | 0.37 (A) | 3.77 (I) |
| 266 | gelbliches Oel | 0.29 (A) | 2.76 (I) |
| 267 | farbloses Oel | 0.33 (A) | 4.41 (I) |
| 268 | gelbliches Oel | 0.09 (C) | 4.15 (I) |
| 269 | gelbliches Oel | 0.09 (C) | 3.34 (I) |
| 270 | gelbliches Oel | 0.27 (C) | 4.37 (I) |
| 271 | braunes Harz | 0.15 (A) | 4.02 (I) |
| 272 | gelbliches Oel | 0.07 (C) | 3.35 (I) |
| 273 | gelbliches Oel | 0.21 (A) | 4.55 (I) |
| 274 | gelbes Oel | 0.22 (A) | 3.37 (I) |
| 275 | gelblicher Feststoff | 0.15 (A) | 3.63 (I) |
| 276 | farbloses Oel | 0.26 (A) | 3.32 (I) |
| 277 | gelbliches Oel | 0.14 (C) | 4.34 (I) |
| 278 | gelbliches Oel | 0.34 (A) | 4.35 (I) |
| 279 | gelbliches Oel | 0.38 (A) | 4.34 (I) |
| 280 | gelbliches Oel | 0.39 (A) | 4.44 (I) |
| 281 | gelbliches Oel | 0.25 (A) | 4.50 (I) |
| 282 | gelbes Oel | 0.20 (A) | 4.46 (I) |
| 283 | gelbes Oel | 0.20 (A) | 4.47 (I) |
| 284 | gelbes Oel | 0.20 (A) | 4.48 (I) |
| 285 | gelblich-beiges Oel | 0.23 (A) | 4.40 (I) |
| 286 | gelbes Oel | 0.19 (A) | 4.21 (I) |
| 287 | gelbes Oel | 0.15 (A) | 4.62 (I) |
| 288 | gelbliches Oel | 0.23 (A) | 4.15 (I) |
| 289 | gelblich-beiges Oel | 0.23 (A) | 4.34 (I) |
| 290 | gelblich-beiges Oel | 0.23 (A) | 4.43 (I) |
| 291 | gelbliches Oel | 0.11 (C) | 3.95 (I) |
| 292 | gelbliches Oel | 0.18 (C) | 4.06 (I) |
| 293 | farbloses Oel | 0.14 (A) | 3.39 (I) |
| 294 | farbloses Oel | 0.13 (A) | 3.22 (I) |
| 295 | oranges Oel | 0.06 (A) | 3.27 (I) |
| 296 | gelbliches Oel | 0.16 (A) | 3.58 (I) |
| 297 | gelbliches Oel | 0.17 (C) | 4.13 (I) |
| 298 | gelbliches Oel | 0.14 (C) | 3.49 (I) |
| 299 | farbloses Oel | 0.13 (C) | 4.04 (I) |
| 300 | gelbes Oel | 0.14 (C) | 3.72 (I) |
| 301 | gelbes Harz | 0.10 (A) | 4.03 (I) |
| 302 | farbloses Oel | 0.20 (A) | 3.36 (I) |
| 303 | gelbliches Oel | 0.11 (C) | 4.43 (I) |
| 304 | gelbliches Oel | 0.25 (A) | 15.21 (II) |
| 305 | gelbliches Oel | 0.23 (A) | 13.97(11) |
| 306 | gelbes Oel | 0.25 (A) | 4.70 (I) |
| 307 | gelbliches Oel | 0.34 (A) | 3.44 (I) |
| 308 | farbloses Oel | 0.19 (C) | 3.94 (I) |
| 309 | gelbliches Oel | 0.13 (C) | 4.42 (I) |
| 310 | gelbliches Oel | 0.14 (C) | 3.83 (I) |
| 311 | gelbliches Oel | 0.31 (A) | 3.54 (I) |
| 312 | gelbliches Oel | 0.41 (A) | 4.61 (I) |
| 313 | gelbes Oel | 0.14 (A) | 3.44 (I) |
| 314 | farbloses Oel | 0.18 (A) | 2.68 (I) |
| 315 | farbloses Oel | 0.20 (C) | 3.52 (I) |
| 316 | gelbliches Oel | 0.18 (I) | 3.66 (I) |
| 317 | gelbliches Oel | 0.35 (A) | 3.75 (I) |
| 318 | gelbliches Oel | 0.33 (A) | 20.17(11) |
| 319 | gelbliches Oel | 0.31 (A) | 19.20 (11) |
| 320 | gelbes Oel | 0.29 (C) | 4.48 (I) |
| 321 | oranges Oel | 0.21 (C) | 4.56 (I) |
| 322 | gelbliches Oel | 0.11 (C) | 4.21 (I) |
| 323 | gelbliches Oel | 0.06 (C) | 2.62 (I) |
| 324 | gelb-oranges Oel | 0.25 (A) | 3.56 (I) |
| 325 | gelbliches Oel | 0.05 (C) | 2.70 (I) |
| 326 | gelb-oranges Oel | 0.35 (A) | 3.96 (I) |
| 327 | gelbliches Oel | 0.17 (C) | 4.45 (I) |
| 328 | gelbliches Oel | 0.38 (A) | 16.76(11) |
| 329 | gelbliches Oel | 0.41 (A) | 15.37 (II) |
| 330 | gelbliches Oel | 0.18 (C) | 4.13 (I) |
| 331 | gelb-oranges Oel | 0.30 (C) | 4.49 (I) |
| 332 | gelb-oranges Oel | 0.30 (C) | 4.85 (I) |
| 333 | gelbliches Oel | 0.15 (I) | 3.81 (I) |
| 334 | oranges Oel | 0.18 (C) | 4.79 (I) |
| 335 | gelbliches Oel | 0.21 (C) | 4.53 (I) |
| 336 | gelbliches Oel | 0.27 (C) | 4.44 (I) |
| 337 | gelbliches Oel | 0.27 (C) | 4.49 (I) |
| 338 | gelbliches Oel | 0.12 (C) | 4.47 (I) |
| 339 | gelbliches Oel | 0.13 (C) | 4.28 (I) |
| 340 | farbloses Oel | 0.30 (A) | 3.51 (I) |
| 341 | gelbes Oel | 0.23 (A) | 4.14 (I) |
| 342 | oranges Oel | 0.25 (C) | 4.73 (I) |
| 343 | oranges Oel | 0.25 (C) | 4.76 (I) |
| 344 | oranges Oel | 0.14 (C) | 4.29 (I) |
| 345 | gelbliches Oel | 0.29 (A) | 3.15 (I) |
| 346 | weisser Schaum | 0.13 (C) | 4.05 (I) |
| 347 | farbloses Oel | 0.10 (C) | 3.64 (I) |
| 348 | farbloses Oel | 0.25 (A) | 3.41 (I) |
| 349 | oranges Oel | 0.33 (S) | 4.51 (I) |
| 350 | farbloser Schaum | 0.18 (C) | 3.62 (I) |
| 351 | gelbliches Oel | 0.27 (A) | 3.44 (I) |
| 352 | weisser Feststoff | 0.19 (C) | 4.25 (I) |
| 353 | weisser Feststoff | 0.13 (A) | 3.25 (I) |
| 354 | farbloses Harz | 0.10 (C) | 4.46 (I) |
| 355 | gelbliches Oel | 0.23 (C) | 3.35 (I) |
| 356 | gelbliches Oel | 0.30 (A) | 4.04 (I) |
| 357 | gelbliches Oel | 0.32 (A) | 4.40 (I) |
| 358 | gelbliches Oel | 0.39 (A) | 3.46 (I) |
| 359 | gelbliches Oel | 0.39 (V) | 4.56 (I) |
| 360 | gelbliches Oel | 0.39 (V) | 4.28 (I) |

### Dünnschichtchromatographie Fliessmittelsysteme :

- A: Dichlormethan-Methanol-Ammoniak konz. 25% = 200:20:1
- B: Dichlormethan-Methanol-Ammoniak konz. 25% = 200:20:0.5
- C: Dichlormethan-Methanol-Ammoniak konz. 25% = 200:10:1
- D: Dichlormethan-Methanol-Ammoniak konz. 25% = 90:10:1
- E: Dichlormethan-Methanol-Wasser-Essigsäure konz. = 750:270:50:5
- F: Dichlormethan-Methanol = 1:4
- G: Dichlormethan-Methanol-Ammoniak konz. 25% = 200:5:1
- H: Dichlormethan-Methanol = 9:1
- I: Dichlormethan-Methanol-Ammoniak konz. 25% = 40:10:1
- J: Dichlormethan-Methanol-Ammoniak konz. 25% = 80:10:1
- K: Dichlormethan-Methanol-Ammoniak konz. 25% = 60:10:1
- L: Dichlormethan-Methanol-Ammoniak konz. 25% = 90:20:1
- M: Dichlormethan-Methanol-Ammoniak konz. 25% = 200:40:1
- N: Dichlormethan-Methanol-Ammoniak konz. 25% = 200:20:1 + 10% Methanol
- O: Dichlormethan-Methanol-Ammoniak konz. 25% = 200:100:2
- P: Dichlormethan-Methanol-Ammoniak konz. 25% = 95:5:1
- Q: Dichlormethan-Methanol-Ammoniak konz. 25% = 200:15:2
- R: Dichlormethan-Methanol-Ammoniak konz. 25% = 200:20:2
- S: Dichlormethan-Methanol-Ammoniak konz. 25% = 200:15:1
- T: Dichlormethan-Methanol-Ammoniak konz. 25% = 200:50:1
- U: Dichlormethan-Methanol-Wasser-Essigsäure konz. = 150:54:10:1
- V: Dichlormethan-Methanol-Ammoniak konz. 25% = 200:10:0.5

### HPLC Gradienten :

- I: 90% Wasser/10% Acetonitril/0.1% Trifluoressigsäure zu 0% Wasser/100% Acetonitril/0.1% Trifluoressigsäure in 5 Minuten + 2.5 Minuten (1.5 ml/min)
- II: 95% Wasser/5% Acetonitril/0.1 % Trifluoressigsäure zu 0% Wasser/100% Acetonitril/0.1% Trifluoressigsäure in 40 Minuten (0.8 ml/min)

## Patentansprüche

1. Verwendung einer Verbindung der allgemeinen Formel (I) oder (II) 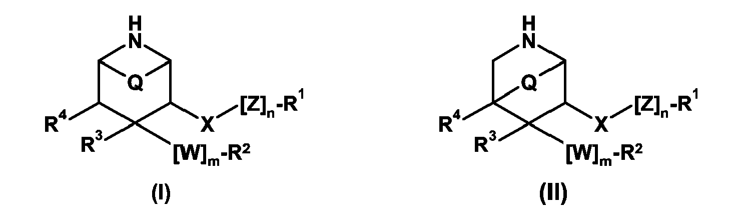 worin
(A) R¹ in Formel (I) substituiertes oder unsubstituiertes Oxazolyl, Indolyl, Pyrrolyl, Pyrazolyl, Triazinyl, 2-Oxo-dihydro-benzo-[d][1,3]oxazinyl, 4-Oxo-dihydro-imidazolyl, 5-Oxo-4H-[1,2,4]triazinyl, 3-Oxo-4H-benzo[1,4]thiazinyl, Tetrahydro-chinoxalinyl, 1,1,3-Trioxodihydro-2H-1lambda*6*-benzo[1,4]thiazinyl, 1-Oxo-pyridyl, Dihydro-2H-benzo[1,4]oxazinyl, 2-Oxo-tetrahydro-benzo[e][1,4]diazepinyl, 2-Oxo-dihydrobenzo[e][1,4]diazepinyl, 1H-Pyrrolizinyl, Phthalazinyl, 1-Oxo-3H-isobenzofuranyl, 4-Oxo-3H-thieno[2,3-d]pyrimidinyl, 3-Oxo-4H-benzo[1,4]oxazinyl, [1,5]Naphthyridyl, Dihydro-2H-benzo[1,4]thiazinyl, 1,1-Dioxo-dihydro-2H-benzo[1,4]thiazinyl, 2-Oxo-1H-pyrido[2,3-b][1,4]oxazinyl, Dihydro-1 H-pyrido[2,3-b][1,4]oxazinyl, 1 H-Pyrrolo[2,3-b]pyridyl, Benzo[1,3]dioxolyl, Benzooxazolyl, 2-Oxo-benzooxazolyl, 2-Oxo-1,3-dihydroindolyl, 2,3-Dihydroindolyl, Indazolyl, Benzofuranyl, Dihydrobenzofuranyl, Tetrahydropyranyl, 2-Oxo-piperidinyl oder 2-Oxo-azepanyl ist; oder
(B) R¹ in Formel (I) Aryl oder Heterocyclyl ist, welches durch mindestens einen Substituenten ausgewählt aus C₁₋₆-Alkoxy-C₁₋₆-alkoxy-C₁₋₆-alkyl, C₃₋₈-Cycloalkyl-C₁₋₆-alkyl, C₁₋₆₋Alkoxy-carbonyl, C₀₋₆-Alkylcarbonylamino, C₀₋₆-Alkylcarbonylamino-C₁₋₆-alkyl, C₀₋₆₋Alkylcarbonylamino-C₁₋₆-alkoxy, (N-C₁₋₆-Alkyl)-C₀₋₆-alkylcarbonylamino-C₁₋₆-alkyl, (N-C₁₋₆-Alkyl)-C₀₋₆-alkylcarbonylamino-C₁₋₆-alkoxy, C₃₋₈-Cycloalkylcarbonylamino-C₁₋₆₋alkyl, C₃₋₈-Cycloalkylcarbonylamino-C₁₋₆-alkoxy, C₁₋₆-Alkoxy-C₁₋₆-alkyl, Hydroxy-C₁₋₆₋alkyl, Hydroxy-C₁₋₆-alkoxy-C₁₋₆-alkyl, Hydroxy-C₁₋₆-alkoxy-C₁₋₆-alkoxy, C₁₋₆-Alkoxycarbonylamino-C₁₋₆-alkyl, C₁₋₆-Alkoxycarbonylamino-C₁₋₆-alkoxy, C₁₋₆-Alkylaminocarbonylamino-C₁₋₆-alkyl, C₁₋₆-Alkylaminocarbonylamino-C₁₋₆-alkoxy, C₁₋₆-Alkylaminocarbonyl-C₁₋₆-alkyl, C₁₋₆-Alkylaminocarbonyl-C₁₋₆-alkoxy, C₁₋₆-Alkylaminocarbonyl-C₁₋₆₋alkoxy-C₁₋₆-alkyl, Di-C₁₋₆-alkylaminocarbonyl-C₁₋₆-alkyl, Di-C₁₆-alkylaminocarbonyl-C₁₋₆₋alkoxy, C₁₋₆-Alkylcarbonyloxy-C₁₋₆-alkyl, C₁₋₆-Alkoxycarbonyloxy-C₁₋₆-alkyl,C₁₋₆₋Alkylcarbonyloxy-C₁₋₆-alkoxy, Cyano-C₁₋₆-alkyl, Cyano-C₁₋₆-alkoxy, C₁₋₆-Alkoxycarbonyl-C₁₋₆-alkyl, C₁₋₆-Alkoxycarbonyl-C₁₋₆-alkoxy, C₁₋₆-Alkylsulfonylamino-C₁₋₆-alkyl, C₁₋₆-Alkylsulfonylamino-C₁₋₆-alkoxy, (N-C₁₋₆-Alkyl)-C₁₋₆-alkylsulfonylamino-C₁₋₆-alkyl, (N-C₁₋₆-Alkyl)-C₁₋₆-alkylsulfonylamino-C₁₋₆-alkoxy, Amino-C₁₋₆-alkyl, Amino-C₁₋₆-alkoxy, C₁₋₆-Alkylamino-C₁₋₆-alkyl, C₁₋₆-Alkylamino-C₁₋₆-alkoxy, Di-C₁₋₆-alkylamino-C₁₋₆-alkyl, Di-C₁₋₆-alkylamino-C₁₋₆-alkoxy, C₁₋₆-Alkylsulfonyl-C₁₋₆-alkyl, C₁₋₆-Alkylsulfonyl-C₁₋₆₋alkoxy, Carboxy-C₁₋₆-alkyl, Carboxy-C₁₋₆-alkoxy, Carboxy-C₁₋₆-alkoxy-C₁₋₆-alkyl, C₁₋₆₋Alkoxy-C₁₋₆-alkyl-carbonyl, Acyl-C₁₋₆-alkoxy-C₁₋₆-alkyl, (N-C₁₋₆-Alkyl)-C₁₋₆-alkoxycarbonylamino, (N-Hydroxy)-C₁₋₆-alkylaminocarbonyl-C₁₋₆-alkyl, (N-Hydroxy)-C₁₋₆₋alkylaminocarbonyl-C₁₋₆-alkoxy, (N-Hydroxy)-aminocarbonyl-C₁₋₆-alkyl, (N-Hydroxy)-aminocarbonyl-C₁₋₆-alkoxy, C₁₋₆-Alkoxy-aminocarbonyl-C₁₋₆-alkyl, C₁₋₆-Alkoxyaminocarbonyl-C₁₋₆-alkoxy, (N-C₁₋₆-Alkoxy)-C₁₋₆-alkylaminocarbonyl-C₁₋₆-alkyl, (N-C₁₋₆₋Alkoxy)-C₁₋₆-alkylaminocarbonyl-C₁₋₆-alkoxy, (N-Acyl)-C₁₋₆-alkoxy-C₁₋₆-alkylamino, C₁₋₆-Alkoxy-C₁₋₆-alkyl-carbamoyl, (N-C₁₋₆-Alkyl)-C₁₋₆-alkoxy-C₁₋₆-alkyl-carbamoyl, C₁₋₆₋Alkoxy-C₁₋₆-alkyl-carbonyl, C₁₋₆-Alkoxy-C₁₋₆-alkyl-carbonylamino, (N-C₁₋₆-Alkyl)-C₁₋₆₋alkoxy-C₁₋₆-alkyl-carbonylamino, Carbamoyl-C₁₋₆-alkyl, Carbamoyl-C₁₋₆-alkoxy, C₁₋₆₋Alkyl-carbamoyl, Di-C₁₋₆-alkyl-carbamoyl, C₁₋₆-Alkyl-sulfonyl, C₁₋₆-Alkyl-amidinyl, Acetamidinyl-C₁₋₆-alkyl, O-Methyloximyl-C₁₋₆-alkyl und O,N-Dimethylhydroxylamino-C₁₋₆-alkyl substituiert ist; oder
(C) R¹ in Formel (I) Aryl oder Heterocyclyl ist, welches durch mindestens einen Substituenten ausgewählt aus [1,2,4]-Triazol-1-yl-alkyl, [1,2,4]-Triazol-1-yl-alkoxy, [1,2,4]-Triazol-4-yl-alkyl, [1,2,4]-Triazol-4-yl-alkoxy, [1,2,4]-Oxadiazol-5-yl-alkyl, [1,2,4]-Oxadiazol-5-yl-alkoxy, 3-Methyl-[1,2,4]-oxadiazol-5-yl-alkyl, 3-Methyl-[1,2,4]-oxadiazol-5-yl-alkoxy, 5-Methyl-[1,2,4]-oxadiazol-3-yl-alkyl, 5-Methyl-[1,2,4]-oxadiazol-3-yl-alkoxy, Tetrazol-1-yl-alkyl, Tetrazol-1-yl-alkoxy, Tetrazol-2-yl-alkyl, Tetrazol-2-yl-alkoxy, Tetrazol-5-yl-alkyl, Tetrazol-5-yl-alkoxy, 5-Methyl-tetrazol-1-yl-alkyl, 5-Methyl-tetrazol-1-yl-alkoxy, Thiazol-4-yl-alkyl, Thiazol-4-yl-alkoxy, Oxazol-4-yl-alkyl, Oxazol-4-yl-alkoxy, 2-Oxo-pyrrolidinyl-alkyl, 2-Oxo-pyrrolidinyl-alkoxy, Imidazolyl-alkyl, Imidazolyl-alkoxy, 2-Methyl-imidazolyl-alkyl, 2-Methyl-imidazolyl-alkoxy, Dioxolanyl, Dioxanyl, Dithiolanyl, Dithianyl, Pyrrolidinyl, Piperidinyl, Piperazinyl, Pyrrolyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 3-Acetamidomethylpyrrolidinyl, 3-C₁₋₆-Alkoxy-C₁₋₆-alkylpyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl, 2-Oxo-tetrahydro-pyrimidinyl, 2-Oxo-oxazolidinyl-C₁₋₆-alkyl, 2-Oxo-oxazolidinyl-C₁₋₆-alkoxy, 1-C₁₋₆-Alkoxy-C₁₋₆-alkyl-imidazol-2-yl, 1-C₁₋₆-Alkoxy-C₁₋₆-alkyl-tetrazol-5-yl, 5-C₁₋₆- Alkoxy-C₁₋₆-alkyl-tetrazol-1-yl und 2-C₁₋₆-Alkoxy-C₁₋₆-alkyl-4-oxo-imidazol-1-yl substituiert ist; oder
(D) R¹ in Formel (I) Aryl oder Heterocyclyl ist, wenn n die Zahl 0 ist und X -O-CH-R¹¹-CO- NR⁹- ist, oder wenn n und m die Zahl 0 ist und X -O-CH-R¹¹- und R² durch C₁₋₆- Alkoxybenzyloxy-C₁₋₆-alkoxy substituiertes Phenyl ist; oder
(E) R¹ in Formel (I) Aryl oder Heterocyclyl ist, wenn n die Zahl 1 und Z Alk-NR⁹- ist, wobei Alk C₁₋₆-Alkylen bezeichnet; oder
(F) R¹ in Formel (I) Aryl oder Heterocyclyl ist, wenn R² Tetrazolyl oder Imidazolyl, welche durch 1-3 Halogen, Hydroxy, Cyano, Trifluormethyl, C₁₋₆-Alkyl, Halo-C₁₋₆-alkyl, Hydroxy-C₁₋₆-alkyl, C₁₋₆-Alkoxy-C₁₋₆-alkyl, Cyano-C₁₋₆-alkyl, Carboxy-C₁₋₆-alkyl, C₁₋₆- Alkanoyloxy-C₁₋₆-alkyl, C₁₋₆-Alkoxycarbonyloxy-C₁₋₆-alkyl, C₁₋₆-Alkoxycarbonyl, oder C₁₋₆-Alkoxygruppen, oder eine C₁₋₆-Alkylendioxygruppe, und/oder durch einen Rest L1-T1-L2-T2-L3-T3-L4-T4-L5-U substituiert sein können, darstellt; oder
(G) R¹ in Formel (II) Aryl oder Heterocyclyl ist;
R² Phenyl, Naphthyl, Acenaphthyl, Cyclohexyl, Pyridyl, Pyrimidinyl, Pyrazinyl, Oxo-pyridinyl, Diazinyl, Triazolyl, Thienyl, Oxazolyl, Oxadiazolyl, Thiazolyl, Pyrrolyl, Furyl, Tetrazolyl oder Imidazolyl, welche Reste durch 1-3 Halogen, Hydroxy, Cyano, Trifluormethyl, C₁₋₆-Alkyl, Halo-C₁₋₆-alkyl, Hydroxy-C₁₋₆-alkyl, C₁₋₆-Alkoxy-C₁₋₆-alkyl, Cyano-C₁₋₆-alkyl, Carboxy-C₁₋₆- alkyl, C₁₋₆-Alkanoyloxy-C₁₋₆-alkyl, C₁₋₆-Alkoxycarbonyloxy-C₁₋₆-alkyl, C₁₋₆-Alkoxycarbonyl, oder C₁₋₆-Alkoxygruppen, oder eine C₁₋₆-Alkylendioxygruppe, und/oder durch einen Rest L1-T1-L2-T2-L3-T3-L4-T4-L5-U substituiert sein können, darstellen;
L1, L2, L3, L4 und L5 unabhängig voneinander eine Bindung, C₁₋₈-Alkylen, C₂₋₈-Alkenylen oder C₂₋₈-Alkinylen darstellen, oder abwesend sind;
T1, T2, T3 und T4 unabhängig voneinander
(a) eine Bindung darstellen, oder abwesend sind, oder eine der Gruppen
(b) -CH(OH)-
(c) -CH(OR⁶)-
(d) -CH(NR⁵R⁶)-
(e) -CO-
(f) -CR⁷R⁸-
(g) -O- oder -NR⁶-
(h) -S(O)₀₋₂-
(I) -SO₂NR⁶-
(j) -NR⁶SO₂-
(k) -CONR⁶-
(I) -NR⁶CO-
(m) ―O-CO-
(n) ―CO-O-
(o) -O-CO-O-
(p) -O-CO-NR⁶-
(q) -N(R⁶)-CO-N(R⁶)-
(r) -N(R⁶)-CO-O-
(s) Pyrrolidinylen, Piperidinylen oder Piperazinylen
(t) -C(R¹¹)(R¹²)-
darstellen,
wobei die von (b)-(t) ausgehenden Bindungen zu einem gesättigten oder aromatischen C-Atom der benachbarten Gruppe führen, falls die Bindung von einem Heteroatom ausgeht, und wobei nicht mehr als zwei Gruppen (b)-(f), drei Gruppen (g)-(h) und eine Gruppe (i)-(t) anwesend sind;
R³ Wasserstoff, Hydroxy, C₁₋₆-Alkoxy oder C₂₋₆-Alkenyloxy;
R⁴ Wasserstoff, C₁₋₆-Alkyl, C₂₋₆-Alkenyl, C₁₋₆-Alkoxy, Hydroxy-C₁₋₆-alkyl, C₁₋₆-Alkoxy-C₁₋₆-alkyl, Benzyl, Oxo, oder eine Gruppe
R^{4a}-Z1-X1- darstellen, wobei R^{4a}
(a) H-
(b) C₁₋₆-Alkyl-
(c) C₂₋₆-Alkenyl-
(d) Hydroxy-C₁₋₆-alkyl-
(e) Polyhydroxy-C₁₋₆-alkyl-
(f) C₁₋₆-Alkyl-O-C₁₋₆-alkyl-
(g) Aryl-
(h) Heterocyclyl-
(i) Arylalkyl-
(j) Heterocyclylalkyl-
(k) Aryloxyalkyl-
(I) Heterocyclyloxyalkyl-
(m) (R⁵,R⁶)N-(CH₂)₁₋₃-
(n) (R⁵, R⁶)N-
(o) C₁₋₆-Alkyl-S(O)₀₋₂-
(p) Aryl-S(O)₀₋₂-
(q) Heterocyclyl-S(O)₀₋₂-
(r) HO-SO₃- bzw. deren Salze
(s) H₂N-C(NH)-NH-
(t) NC-
darstellt und die von (n)-(t) ausgehenden Bindungen zu einem C-Atom der benachbarten Gruppe führen und dieses C-Atom gesättigt ist, falls die Bindung von einem Heteroatom ausgeht;
Z1
(a) eine Bindung darstellt, abwesend ist, oder eine der Gruppen
(b) C₁₋₆-Alkylen-
(c) C₂₋₆-Alkenylen-
(d) -O-, -N(R¹¹)-, -S(O)₀₋₂-
(e) -CO-
(f) -O-CO-
(g) -O-CO-O-
(h) -O-CO-N(R¹¹)-
(i) -N(R¹¹)-CO-O-
(j) -CO-N(R¹¹)-
(k) -N(R¹¹)-CO-
(l) -N(R¹¹)-CO-N(R¹¹)-
(m) -CH(OR⁹)-
darstellt und die von (d) und (f)-(m) ausgehenden Bindungen zu einem C-Atom der benachbarten Gruppe führen und dieses C-Atom gesättigt ist, falls die Bindung von einem Heteroatom ausgeht;
X1
(a) eine Bindung darstellt, abwesend ist, oder eine der Gruppen
(b) -O-
(c) -N(R¹¹)-
(d) -S(O)₀₋₂-
(e) -(CH₂)₁₋₃-
darstellt;
oder R³ und R⁴ zusammen eine Bindung darstellen;
R⁵ und R⁶ Wasserstoff, C₁₋₆-Alkyl, C₂₋₆-Alkenyl, Aryl-C₁₋₆-alkyl oder Acyl bedeuten, oder gemeinsam mit dem N-Atom, an das sie gebunden sind, einen 5- oder 6-gliedrigen heterocyclischen Ring bedeuten, der ein zusätzliches N-, O- oder S-Atom oder eine -SO- oder -SO₂- Gruppe enthalten kann, wobei das zusätzliche N-Atom gegebenenfalls durch C₁₆-Alkyl-Reste substituiert sein kann;
R⁷ und R⁸ gemeinsam mit dem C-Atom, an das sie gebunden sind, einen 3-7-gliedrigen Ring darstellen, der ein oder zwei -O- oder -S- Atome oder -SO- oder -SO₂- Gruppen enthalten kann;
R⁹ Wasserstoff, C₁₋₆-Alkyl, C₁₋₆-Alkoxy-C₁₋₆-alkyl, Acyl oder Arylalkyl;
R¹⁰ Carboxyalkyl, Alkoxycarbonylalkyl, Alkyl oder Wasserstoff;
R¹¹ Wasserstoff oder C₁₋₆-Alkyl ist;
R¹² Wasserstoff oder C₁₋₆-Alkyl ist;
U Wasserstoff, C₁₋₆-alkyl, C₃₋₈-Cycloalkyl, Cyano, gegebenenfalls substituiertes C₃₋₈₋Cycloalkyl, Aryl, oder Heterocyclyl darstellt;
Q Ethylen darstellt oder abwesend ist (Formel I) oder Ethylen oder Methylen darstellt (Formel II);
X eine Bindung, Sauerstoff oder Schwefel, wobei die von einem Sauerstoff- oder Schwefelatom ausgehende Bindung zu einem gesättigten C-Atom der Gruppe Z oder zu R¹ führt, oder eine Gruppe >CH-R¹¹, >CHOR⁹, -O-CO-, >CO, >C=NOR¹⁰, -O-CHR¹¹- oder -O-CHR¹¹-CO-NR⁹- darstellt;
W Sauerstoff oder Schwefel darstellt;
Z C₁₋₆-Alkylen, C₂₋₆-Alkenylen, Hydroxy-C₁₋₆-alkyliden, -O-, -S-, -O-Alk-, -S-Alk-, -Alk-O-, -Alk-S- oder -Alk-NR⁹- ist, wobei Alk C₁₋₆-Alkylen bezeichnet; und wobei
(a) falls Z -O- oder -S- darstellt, X -CH-R¹¹- ist und entweder R² einen Substituenten L1-T1-L2-T2-L3-T3-L4-T4-L5-U enthält oder R⁴ ein wie oben definierter, von Wasserstoff verschiedener Substituent ist;
(b) falls Z -O-Alk- oder -S-Alk- darstellt, X -CH-R¹¹- ist; und
(c) falls X eine Bindung darstellt, Z C₂₋₆-Alkenylen, -Alk-O- oder -Alk-S- darstellt;
n die Zahl 0 oder 1 ist;
m die Zahl 0 oder 1 ist;
und ihr Salz, Prodrug oder Verbindung, bei welcher ein oder mehrere Atome durch ihre stabilen, nicht radioaktiven Isotope ersetzt sind, insbesondere pharmazeutisch anwendbares Salz;
zur Herstellung eines Humanheilmittels zur Beta-Sekretase-, Cathepsin D-, Plasmepsin II-und/oder HIV-Protease-Hemmung.

2. Verwendung gemäss Anspruch 1, **dadurch gekennzeichnet, dass** die Verbindung der Formel (IA) oder (IIA) 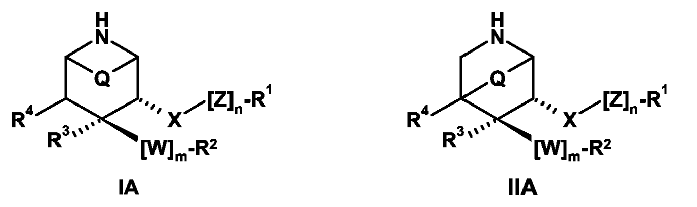 entspricht, worin R¹, R², R³, R⁴, Q, W, X, Z, n und m die für die Verbindungen der Formeln (I) bzw. (II) angegebenen Bedeutungen gemäss Anspruch 1 haben.

3. Verfahren zur Beta-Sekretase-, Cathepsin D-, Plasmepsin II- und/oder HIV-Protease-Hemmung, wobei eine therapeutisch wirksame Menge einer Verbindung der allgemeinen Formel (I), (II), (IA) oder (IIA), gemäss den Ansprüchen 1 und 2, verwendet wird.

4. Pharmazeutisches Präparat zur Beta-Sekretase-, Cathepsin D-, Plasmepsin II- und/oder HIV-Protease-Hemmung enthaltend eine Verbindung der allgemeinen Formel (I), (II), (IA) oder (IIA), gemäss den Ansprüchen 1 und 2, sowie übliche Hilfsstoffe.

5. Verwendung einer Verbindung der allgemeinen Formel (I), (II), (IA) oder (IIA), gemäss den Ansprüchen 1 und 2, zur Herstellung eines Humanheilmittels zur Prevention, zur Progressionsverzögerung oder zur Behandlung von Alzheimer, Malaria oder HIV.

6. Verfahren zur Prevention, zur Progressionsverzögerung oder zur Behandlung von Alzheimer, Malaria oder HIV, wobei eine therapeutisch wirksame Menge einer Verbindung der allgemeinen Formel (1), (II), (IA) oder (IIA), gemäss den Ansprüchen 1 und 2, verwendet wird.

7. Pharmazeutisches Präparat zur Progressionsverzögerung oder zur Behandlung von Alzheimer, Malaria oder HIV enthaltend eine Verbindung der allgemeinen Formel (I), (II), (IA) oder (IIA), gemäss den Ansprüchen 1 und 2, sowie übliche Hilfsstoffe.
